# EUROPEAN PATENT SPECIFICATION

(11) **EP 3 814 527 B1**
(45) Date of publication and mention of the grant of the patent: **22.02.2023**
(21) Application number: 19739825.8
(22) Date of filing: 26.06.2019
(51) Int. Cl.: C12Q 1/6844

(54) **CRISPR EFFECTOR SYSTEM BASED AMPLIFICATION METHODS, SYSTEMS, AND DIAGNOSTICS**
VERFAHREN, SYSTEME UND DIAGNOSTIKA AUF DER BASIS VON CRISPR-EFFEKTORSYSTEMEN
PROCÉDÉS ET SYSTÈMES D'AMPLIFICATION FONDÉS SUR UN SYSTÈME EFFECTEUR CRISPR ET DIAGNOSTICS ASSOCIÉS

(30) Priority: 26.06.2018 US 201862690257 P; 14.11.2018 US 201862767052 P; 14.03.2019 US 201962818650 P
(43) Date of publication of application: 05.05.2021
(73) Proprietor: Massachusetts Institute of Technology, Cambridge, MA 02139 (US); The Broad Institute, Inc., Cambridge, MA 02142 (US); President and Fellows of Harvard College, Cambridge, MA 02138 (US)
(72) Inventor: ZHANG, Feng, Cambridge, MA 02142 (US); KELLNER, Max, Cambridge, MA 02142 (US); GOOTENBERG, Jonathan, Cambridge, MA 02138 (US); ABUDAYYEH, Omar, Cambridge, MA 02139 (US)
(74) Representative: De Clercq & Partners
(86) International application number: PCT/US2019/039167
(87) International publication number: WO 2020/006036

(56) References cited:
- WO-A1-2016/077350
- WO-A1-2016/205749
- WO-A1-2017/189308
- WO-A1-2018/107129
- US-A1- 2004 058 378
- US-A1- 2013 224 799
- YONG-JOO JEONG ET AL: "Isothermal DNA amplification in vitro: the helicase-dependent amplification system", CELLULAR AND MOLECULAR LIFE SCIENCES, BIRKHÄUSER-VERLAG, BA, vol. 66, no. 20, 24 July 2009 (2009-07-24) , pages 3325-3336, XP019755988, ISSN: 1420-9071, DOI: 10.1007/S00018-009-0094-3
- JONATHAN S. GOOTENBERG ET AL: "Nucleic acid detection with CRISPR-Cas13a/C2c2", SCIENCE, vol. 356, no. 6336, 28 April 2017 (2017-04-28), pages 438-442, XP055481345, US ISSN: 0036-8075, DOI: 10.1126/science.aam9321
- MURUGAN KARTHIK ET AL: "The Revolution Continues: Newly Discovered Systems Expand the CRISPR-Cas Toolkit", MOLECULAR CELL, vol. 68, no. 1, 5 October 2017 (2017-10-05), pages 15-25, XP085207633, ISSN: 1097-2765, DOI: 10.1016/J.MOLCEL.2017.09.007

## Description

### CROSS-REFERENCE TO RELATED APPLICATIONS

This application claims the benefit of U.S. Provisional Applications No. 62/690,257, filed June 26, 2018, U.S. Provisional Application No. 62/767,052, filed November 14, 2018, and U.S. Provisional Application No. 62/818,650, filed March 14, 2019.

### STATEMENT REGARDING FEDERALLY SPONSORED RESEARCH

This invention was made with government support under Grant Nos. MH100706, MH110049, and HL141201 awarded by the National Institutes of Health. The government has certain rights in the invention.

### TECHNICAL FIELD

The subject matter disclosed herein is generally directed to nucleic acid amplification methods, systems, and rapid diagnostics related to the use of CRISPR effector systems.

### BACKGROUND

Nucleic acids are a universal signature of biological information. The ability to rapidly detect nucleic acids with high sensitivity and single-base specificity on a portable platform has the potential to revolutionize diagnosis and monitoring for many diseases, provide valuable epidemiological information, and serve as a generalizable scientific tool. Although many methods have been developed for detecting nucleic acids (Du et al., 2017; Green et al., 2014; Kumar et al., 2014; Pardee et al., 2014; Pardee et al., 2016; Urdea et al., 2006), they inevitably suffer from trade-offs among sensitivity, specificity, simplicity, and speed. For example, qPCR approaches are sensitive but are expensive and rely on complex instrumentation, limiting usability to highly trained operators in laboratory settings. As nucleic acid diagnostics become increasingly relevant for a variety of healthcare applications, detection technologies that provide high specificity and sensitivity at low cost would be of great utility in both clinical and basic research settings.

Many nucleic acid amplification approaches are available with various detection platforms. Among them, isothermal nucleic acid amplification methods have been developed for amplification without drastic temperature cycling and complex instrumentations. These methods include nucleic-acid sequenced-based amplification (NASBA), recombinase polymerase amplification (RPA), loop-mediated isothermal amplification (LAMP), strand displacement amplification (SDA), helicase-dependent amplification (HDA), or nicking enzyme amplification reaction (NEAR). These isothermal amplification approaches, however, may still require an initial denaturation step and multiple sets of primers. Furthermore, novel approaches combining isothermal nucleic acid amplification with portable platforms (Du et al., 2017; Pardee et al., 2016), offer high detection specificity in a point-of-care (POC) setting, but have somewhat limited applications due to low sensitivity.

US 2004/058378 discloses an isothermal HDA.

### SUMMARY

The invention is defined by the claims.

The first and second CRISPR/Cas enzymes may be active or dead. The first primer may comprise a portion that is complementary to the first strand of the target nucleic acid and the second primer may comprise a portion that is complementary to the second strand of the target nucleic acid. The target nucleic acid may be amplified by opening R-loops of the target nucleic acid using the first and second CRISPR/Cas complexes, unwinding the first and the second strand of the target nucleic acid using the helicase, and annealing and extending the strands using the primer pair and the polymerase.

In some embodiments, the dead CRISPR/Cas enzyme may be selected from the group consisting of dead Cas9, dead Cas12a, dead Cas12b, and dead Cas12c. In some embodiments, the dead CRISPR/Cas enzyme may be a dead Cas9 protein which comprises a mutation in the HNH and RuvC domain.

The dead CRISPR/Cas enzyme may be a dead Cas9 protein which comprises a mutation corresponding to D10A and N863A in SpCas9, or D10A and H840A in SpCas9. The dead CRISPR/Cas enzyme may be a dead Cas9 protein derived from a bacterial species selected from the group consisting of *Streptococcus pyogenes, Staphylococcus aureus, Streptococcus thermophilus, S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii, Francisella tularensis 1, Prevotella albensis, Lachnospiraceae bacterium MC2017* 1, *Butyrivibrio proteoclasticus, Peregrinibacteria bacterium GW2011 GWA2 33 10, Parcubacteria bacterium GW2011 GWC2 44 17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens* and *Porphyromonas macacae.*

In some embodiments, the dead CRISPR/Cas enzyme may be a dead Cast2 protein. The dead Cast2 protein may comprise a mutation in the RuvC domain. The dead Cast2 enzyme may be a dead Cas12a, Cas12b, or Cas12c. The dead Cas12a protein may comprise a mutation corresponding to D908A or E993A in AsCpfl.

The dead Cast2 protein may be derived from a bacterial species selected from the group consisting of *Francisella tularensis, Prevotella albensis, Lachnospiraceae bacterium, Butyrivibrio proteoclasticus, Peregrinibacteria bacterium, Parcubacteria bacterium, Smithella sp., Acidaminococcus sp., Lachnospiraceae bacterium, Candidatus Methanoplasma termitum, Eubacterium eligens, Moraxella bovoculi, Leptospira inadai, Porphyromonas crevioricanis, Prevotella disiens and Porphyromonas macacae, Succinivibrio dextrinosolvens, Prevotella disiens, Flavobacterium branchiophilum, Helcococcus kunzii, Eubacterium sp., Microgenomates (Roizmanbacteria) bacterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis* and *Proteocatella sphenisci.*

The dead Cas12 enzyme may be a dead Cas12b protein which comprises a mutation in the Nuc domain. The dead Cas12b may comprise a mutation corresponding to D570A, E848A, or D977A in AacC2c1.

The dead Cas12b protein may be derived from a bacterial species selected from the group consisting of *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia bacterium REFOXYA12, Omnitrophica WOR_2 bacterium RLFCSPHIGHO2, Opitutaceae bacterium TAV5, Phycisphaerae bacterium ST-NAGAB-D1, Planctomycetes bacterium RBG_13_46_10, Spirochaetes bacterium GWB1 27 13, Verrucomicrobiaceae bacterium UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri (e.g., BAB-2500),* and *Methylobacterium nodulans.*

In some embodiments, the first dead CRISPR/Cas enzyme and the second dead CRISPR/Cas enzyme may be the same. In alternative embodiments, the first dead CRISPR/Cas enzyme and the second dead CRISPR/Cas enzyme may be different.

In some embodiments, the first and second CRISPR/Cas enzyme are catalytically active enzyme. In some instances, the CRISPR/Case enzyme is a Cas9, Cas12, Cas 12b or Cas 12c enzyme.

In some embodiments, the polymerase may be selected from the group consisting of Bst 2.0 DNA polymerase, Bst 2.0 WarmStart DNA polymerase, Bst 3.0 DNA polymerase, full length Bst DNA polymerase, large fragment Bst DNA polymerase, large fragment Bsu DNA polymerase, phi29 DNA polymerase, T7 DNA polymerase, Gst polymerase, Taq polymerase, Klenow fragment of E. coli DNA polymerase I, KlenTaq NDA polymerase, Pol III DNA polymerase, T5 DNA polymerase, and Sequenase DNA polymerase.

In some embodiments, the helicase may be selected from the group consisting of UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase. In some instances, the helicase is substituted to increase helicase processivity and or activity.

The amplification of the target nucleic acid may be performed at about 37°C-65°C. The amplification of the target nucleic acid may be performed at about 50°C-59°C. The amplification of the target nucleic acid may be performed at about 60°C-72°C. The amplification of the target nucleic acid may be performed at about 37°C. The amplification of the target nucleic acid may also be performed at room temperature. Room temperature may vary from 20°C-25°C. Amplification of the target nucleic acid may be performed at a constant temperature. Amplification may be performed under mesophilic or thermophilic isothermal conditions.

In some instances, the methods disclosed herein are performed under mesophilic isothermal conditions, and requires use of a mesophilic polymerase. In some instances, the mesophilic polymerase is Sau LF polymerase. Similarly, when amplification is performed under mesophilic isothermal conditions, the helicase utilized performs with sufficient activity under mesophilic conditions. In some instances, the helicase is a UvrD-like helicase, wild-type, or a mutant with super helicase mutations. Super helicase mutations are typically DD to AA substitutions that increase activity of the helicase. In some instances, the helicase is an E. coli UvrD helicase with. Substitutions at D403A and D404A. In some embodiments, the helicase is a pcrA (UvrD)-type helicase, for example, Tte-UvrD helicase with mutations at D409/D410A. In some instances, the helicase is an ATP dependent helicase, and an ATP co-factor can be used in conjunction with the helicase. In some cases, the helicase is PcrA helicase from Thermoanaerobacter ethanolicus with mutations D403A/D404A, PcrA helicase from Bacillus sp. FJAT-27231 with mutations D407A/D408A, PcrA helicase from Bacillus megaterium with mutations D415A/D416A, PcrA helicase from Bacillus simplex with mutations D407A/D408A, or PcrA helicase from Paeniclostridium sordellii with mutations D402A/D403A.

The target nucleic acid sequence may be about 20-30, about 30-40, about 40-50, or about 50-100 nucleotides in length. The target nucleic acid sequence may be about 100-200, about 100-500, or about 100-1000 nucleotides in length. The target nucleic acid sequence may be about 1000-2000, about 2000-3000, about 3000-4000, or about 4000-5000 nucleotides in length. In some instances, when the target nucleic acid is comprised within genomic DNA, a CRISPR/Cas enzyme can be used that is catalytically active and cuts smaller target nucleic acid sequences for further amplification.

Detection of the amplified nucleic acid may be performed by a method selected from the group consisting of gel electrophoresis, intercalating dye detection, PCR, real-time PCR, fluorescence, Fluorescence Resonance Energy Transfer (FRET), mass spectrometry, lateral flow assay, colorimetric assays, and CRISPR-based detection system. The first or the second primer may comprise an RNA polymerase promoter. The amplified nucleic acid may be detected by a Cas13-based system, a CRISPR Cas12-based system, or a combination thereof. In some embodiments, the LwCas13 is used for detection.

The target nucleic acid may be detected at attomolar sensitivity. The target nucleic acid may be detected at femtomolar sensitivity.

The target nucleic acid may be selected from the group consisting of genomic DNA, mitochondrial DNA, viral DNA, plasmid DNA, circulating cell free DNA, environmental DNA, synthetic double-stranded DNA, and RNA.

In embodiments where the target nucleic acid is RNA, the RNA may be reverse transcribed into cDNA prior to amplification. Exemplary amplification methods include recombinase polymerase amplification (RPA). In some embodiments, T7 RNA polymerase is used for in vitro transcription.

In some embodiments, the method of amplification and detecting can comprise one or more additives, including crowding agents, co-factors, single-strand binding proteins, and/or assisting proteins. Additives can be selected, for example, based on measurement of signal to background ratio. In some instances, ATP can be used as a co-factor. Crowding agents can include polymers such as polyethylene glycol (PEG). In some embodiments, the PEG has a molecular weight of 20K, however, the molecular weight and level of branching can be selected on the complexity of the system in which the methods are applied.

Single-strand binding proteins are also contemplated for use in some methods and systems presently disclosed. For example, T4 gp32 or thermophilic SSB (ET-SSB). Can be used. Assisting proteins for complex templates can also be utilized, particularly for complex templates. In some embodiments, dCpf1 and/or UvsX Recombinase can be used.

The sample may be a biological sample or an environmental sample. The biological sample may be a blood, plasma, serum, urine, stool, sputum, mucous, lymph fluid, synovial fluid, bile, ascites, pleural effusion, seroma, saliva, cerebrospinal fluid, aqueous or vitreous humor, or any bodily secretion, a transudate, an exudate, or fluid obtained from a joint, or a swab of skin or mucosal membrane surface. The sample may be blood, plasma or serum obtained from a human patient. The sample may be a plant sample. The sample may be a crude sample. The sample may be a purified sample.

In another aspect, the invention provides a method for amplifying and/or detecting a target single-stranded nucleic acid. The method comprises converting the single-stranded nucleic acid in a sample to a target double-stranded nucleic acid prior to performing the steps described so far. The target single-stranded nucleic acid may be an RNA molecule. The RNA molecule may be converted to the double-stranded nucleic acid by a reverse-transcription and amplification step.

The target single-stranded nucleic acid may be selected from the group consisting of single-stranded viral DNA, viral RNA, messenger RNA, ribosomal RNA, transfer RNA, microRNA, short interfering RNA, small nuclear RNA, synthetic RNA, and synthetic single-stranded DNA, long non-coding RNA, pre-microRNA, viral dsRNA, and non-viral dsRNA.

In instances where the target nucleic acid is RNA, the RNA may be reverse transcribed into cDNA prior to amplification. Amplification may then be done by any suitable amplification method known in the art, such as any described herein. One exemplary amplification method may include recombinase polymerase amplification (RPA).

In another aspect, the invention provides a system for amplifying and/or detecting a target double-stranded nucleic acid in a sample. The system may comprise an amplification CRISPR system, a helicase, a primer pair comprising a first and second primer, a polymerase, and optionally a detection system for detecting amplification of the target nucleic acid.

In some embodiments, the method further comprises mediating a first step of amplifying with the primer pair, and subsequently mediating the steps of amplifying with an amplifier primer that comprises sequence identity to a portion of the first primer of the primer pair. In some instances, the amplifier primer and the first primer of the primer each comprise a T7 promoter sequence.

The amplification CRISPR system may comprise a first and second CRISPR/Cas complex. The first CRISPR/Cas complex may comprise a first CRISPR/Cas enzyme and a first guide molecule that guides the first CRISPR/Cas complex to a first strand of the target nucleic acid. The second CRISPR/Cas complex may comprise a second CRISPR/Cas enzyme and a second guide molecule that guides the second CRISPR/Cas complex to a second strand of the target nucleic acid. The CRISPR/Cas enzyme may be active or dead. The first primer may comprise a portion that is complementary to the first strand of the target nucleic acid and the second primer may comprise a portion that is complementary to the second strand of the target nucleic acid.

The dead CRISPR/Cas enzyme may be a dead Cas9 enzyme or a dead Cas12 enzyme. The dead Cas12 enzyme may be a dead Cas12a, Cas12b, or Cas12c enzyme.

The polymerase may be selected from the group consisting of Bst 2.0 DNA polymerase, Bst 2.0 WarmStart DNA polymerase, Bst 3.0 DNA polymerase, full length Bst DNA polymerase, large fragment Bst DNA polymerase, large fragment Bsu DNA polymerase, phi29 DNA polymerase, T7 DNA polymerase, Gst polymerase, Taq polymerase, Klenow fragment of E. coli DNA polymerase I, KlenTaq DNA polymerase, Pol III DNA polymerase, T5 DNA polymerase, and Sequenase DNA polymerase.

The helicase may be selected from the group consisting of UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase. The dead CRISPR/CAS enzyme, the helicase, and the polymerase may perform under the same temperature.

In another aspect, the invention provides a system for amplifying and/or detecting a target single-stranded nucleic acid in a sample. In addition to the components described in the previous embodiment, the system may comprise reagents for converting the target single-stranded nucleic acid to a double-stranded nucleic acid.

These and other aspects, objects, features, and advantages of the example embodiments will become apparent to those having ordinary skill in the art upon consideration of the following detailed description of illustrated example embodiments.

### BRIEF DESCRIPTION OF THE DRAWINGS

An understanding of the features and advantages of the present invention will be obtained by reference to the following detailed description that sets forth illustrative embodiments, in which the principles of the invention may be utilized, and the accompanying drawings of which:

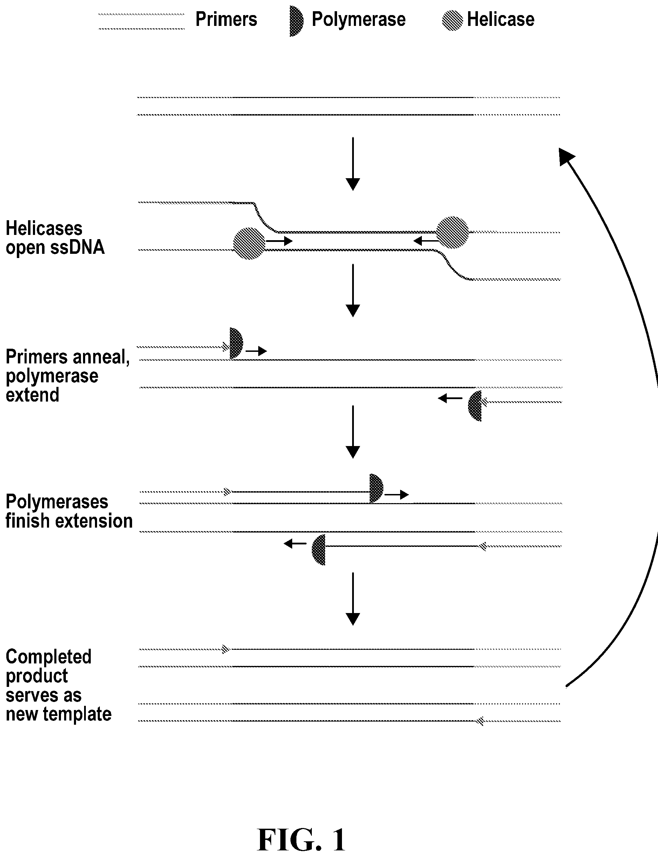

**FIG. 1** is a schematic describing the HDA workflow.
**FIG. 2** is a schematic describing the CRISPR-HDA workflow.
**FIGs. 3A-3G** are graphs illustrating the sensitivity of a 2-step thermophilic CRISPR HDA SHERLOCK isothermal amplification method for single molecule detection. The individual graphs show detection at (FIG. 3A) 2 pM, (FIG. 3B) 200fM, (FIG. 3C) 20 fM, (FIG. 3D) 2 fM, (FIG. 3E) 200 aM, (FIG. 3F) 2 aM, and (FIG. 3G) 0 aM concentrations.
**FIG. 4** is a graph showing that dAsCpf1 improves 2-step thermophilic HDA (tHDA) SHERLOCK sensitivity.
**FIG. 5** is a graph showing the effect is caused by the dAsCpf1:crRNA complex.

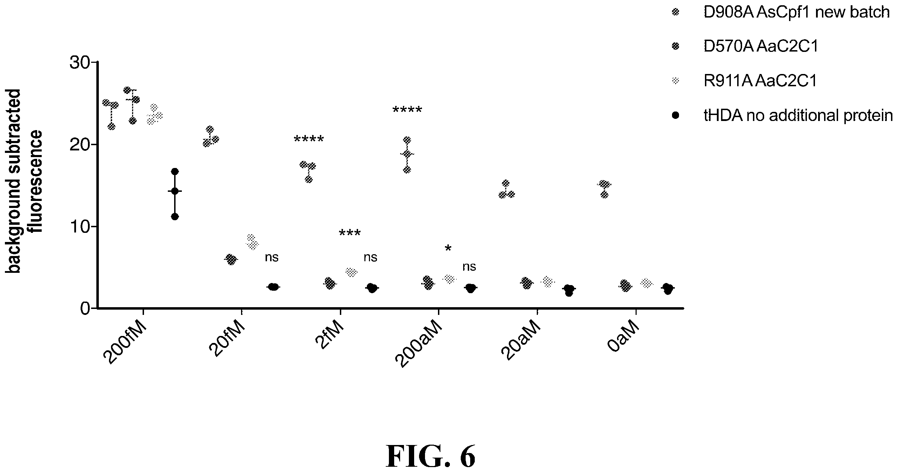

**FIG. 6** is a graph showing that both Cas12a and Cas12b improve 2-step tHDA-SHERLOCK.
**FIG. 7** is charts selection of mesophilic helicases, including range of temperature, left, and size (aa), right.
**FIG. 8** depicts an exemplary isothermal helicase reporter assay
**FIG. 9** shows Alanine substitutions for D403 and D404 in E.Coli UvrD increase Helicase processivity and activity. Consensus identity is provided, with sites conserved among selected UvrD helicases, boxed (SEQ ID NO: 1-14).
**FIG. 10** shows selected pcrA (UvrD)-type helicase have a broad activity spectrum, `Super helicase' DD-> AA substitutions increase activity.
**FIG. 11** includes graphs showing helicase reporter assay can be used to infer co-factor preference and kinetics of helicase substrate unwinding. Numbers 1 to 8, numerically represent 2 fold helicase concentration dilutions, 1 being the highest and 8 is no helicase.
**FIG. 12** charts studies of protocol optimization where 500 ng of T4 gp32 SSB and 125 ng of helicase per reaction performed best.
**FIG. 13** graphs optimization of signal to background ratio, showing the addition of crowding agent PEG 20K and ATP as a co-factor to increase signal to background ratio.
**FIG. 14** is a schematic showing conventional primer strategy and the anchored priming strategy.

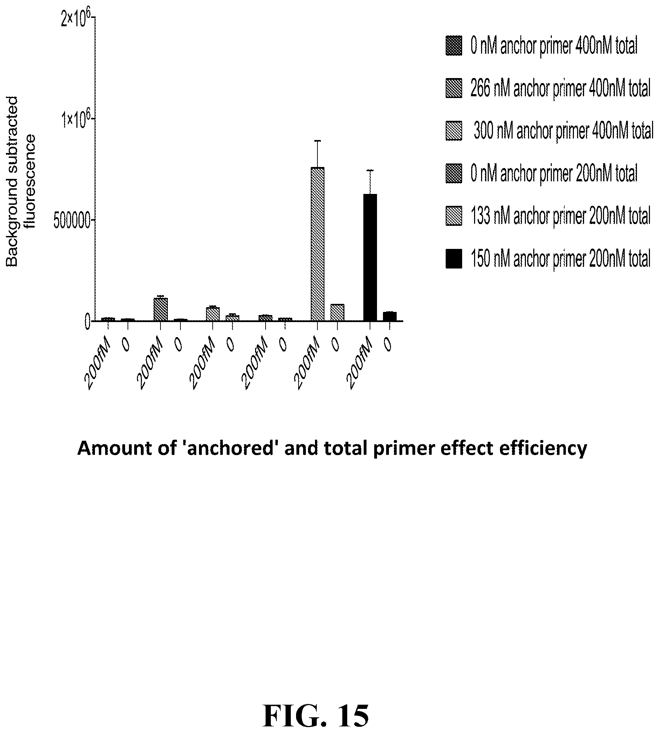

**FIG. 15** graphs the amount of 'anchored' and total primer effect efficiency, showing 'anchored' priming increases amplification efficiency.
**FIG. 16** includes graphs showing optimized one-pot mHDA-SHERLOCK achieves 2fM (1000 copies), right in less than 2 hours, left.
**FIG. 17** graphs one-pot mHDA-SHERLOCK on plasmid DNA.

The figures herein are for illustrative purposes only and are not necessarily drawn to scale.

### DETAILED DESCRIPTION OF THE EXAMPLE EMBODIMENTS

### General Definitions

Unless defined otherwise, technical and scientific terms used herein have the same meaning as commonly understood by one of ordinary skill in the art to which this disclosure pertains. Definitions of common terms and techniques in molecular biology may be found in Molecular Cloning: A Laboratory Manual, 2nd edition (1989) (Sambrook, Fritsch, and Maniatis); Molecular Cloning: A Laboratory Manual, 4th edition (2012) (Green and Sambrook); Current Protocols in Molecular Biology (1987) (F.M. Ausubel et al. eds.); the series Methods in Enzymology (Academic Press, Inc.): PCR 2: A Practical Approach (1995) (M.J. MacPherson, B.D. Hames, and G.R. Taylor eds.): Antibodies, A Laboratory Manual (1988) (Harlow and Lane, eds.): Antibodies A Laboratory Manual, 2nd edition 2013 (E.A. Greenfield ed.); Animal Cell Culture (1987) (R.I. Freshney, ed.); Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); Robert A. Meyers (ed.), Molecular Biology and Biotechnology: a Comprehensive Desk Reference, published by VCH Publishers, Inc., 1995 (ISBN 9780471185710); Singleton et al., Dictionary of Microbiology and Molecular Biology 2nd ed., J. Wiley & Sons (New York, N.Y. 1994), March, Advanced Organic Chemistry Reactions, Mechanisms and Structure 4th ed., John Wiley & Sons (New York, N.Y. 1992); and Marten H. Hofker and Jan van Deursen, Transgenic Mouse Methods and Protocols, 2nd edition (2011)

As used herein, the singular forms "a", "an", and "the" include both singular and plural referents unless the context clearly dictates otherwise.

The term "optional" or "optionally" means that the subsequent described event, circumstance or substituent may or may not occur, and that the description includes instances where the event or circumstance occurs and instances where it does not.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0. 1% or less of and from the specified value, insofar such variations are appropriate to perform in the disclosed invention. It is to be understood that the value to which the modifier "about" or "approximately" refers is itself also specifically, and preferably, disclosed.

As used herein, a "biological sample" may contain whole cells and/or live cells and/or cell debris. The biological sample may contain (or be derived from) a "bodily fluid". The present invention encompasses embodiments wherein the bodily fluid is selected from amniotic fluid, aqueous humour, vitreous humour, bile, blood serum, breast milk, cerebrospinal fluid, cerumen (earwax), chyle, chyme, endolymph, perilymph, exudates, feces, female ejaculate, gastric acid, gastric juice, lymph, mucus (including nasal drainage and phlegm), pericardial fluid, peritoneal fluid, pleural fluid, pus, rheum, saliva, sebum (skin oil), semen, sputum, synovial fluid, sweat, tears, urine, vaginal secretion, vomit and mixtures of one or more thereof. Biological samples include cell cultures, bodily fluids, cell cultures from bodily fluids. Bodily fluids may be obtained from a mammal organism, for example by puncture, or other collecting or sampling procedures.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

"C2c2" is now referred to as "Cas13a", and the terms are used interchangeably herein unless indicated otherwise. The terms "Group 29," "Group 30," and Cas13b are used interchangeably herein. The terms "Cpfl" and "Cast2a" are used interchangeably herein. The terms "C2c1" and "Cas12b" are used interchangeably herein.

The terms "melting", "unwinding" or "denaturing" refer to separating all or part of two complementary strands of a nucleic acid duplex.

"Nuclease" and "endonuclease" are used interchangeably herein to mean an enzyme which possesses endonucleolytic catalytic activity for DNA cleavage.

The terms "orthologue" (also referred to as "ortholog" herein) and "homologue" (also referred to as "homolog" herein) are well known in the art. By means of further guidance, a "homologue" of a protein as used herein is a protein of the same species which performs the same or a similar function as the protein it is a homologue of. Homologous proteins may but need not be structurally related, or are only partially structurally related. An "orthologue" of a protein as used herein is a protein of a different species which performs the same or a similar function as the protein it is an orthologue of. Orthologous proteins may but need not be structurally related, or are only partially structurally related. Homologs and orthologs may be identified by homology modelling (see, e.g., Greer, Science vol. 228 (1985) 1055, and Blundell et al. Eur J Biochem vol 172 (1988), 513) or "structural BLAST" (Dey F, Cliff Zhang Q, Petrey D, Honig B. Toward a "structural BLAST": using structural relationships to infer function. Protein Sci. 2013 Apr;22(4):359-66. doi: 10.1002/pro.2225.). See also Shmakov et al. (2015) for application in the field of CRISPR-Cas loci. Homologous proteins may but need not be structurally related, or are only partially structurally related.

### OVERVIEW

In helicase-dependent amplification (HDA), a helicase enzyme is used to unwind a double stranded nucleic acid to generate templates for primer hybridization and subsequent primer-extension. This process utilizes two oligonucleotide primers, each hybridizing to the 3'-end of either the sense strand containing the target sequence or the anti-sense strand containing the reverse-complementary target sequence. The HDA reaction is a general method for helicase-dependent nucleic acid amplification.

In combining this method with a CRISPR-Cas system, the target nucleic acid may be amplified by opening R-loops of the target nucleic acid using first and second CRISPR/Cas complexes. The first and second strand of the target nucleic acid may thus be unwound using a helicase, allowing primers and polymerase to bind and extend the DNA under isothermal conditions. Detection of target nucleic acids can be performed subsequent to HDA techniques, in some preferred embodiments, SHERLOCK (Specific High-sensitivity Enzymatic Reporter unLOCKing), can be used for detection of target nucleic acids.

In one aspect, the embodiments disclosed herein are directed to a system for amplification and/or detecting a target nucleic acid in a sample comprising an amplification CRISPR system, a helicase, a primer pair, a polymerase, and optionally a detection system for detecting amplification of the target nucleic acid. In some embodiments, the system is detecting a double stranded nucleic acid, however, in instances where the target nucleic acid is single stranded, reagents for converting the target single-stranded nucleic acid to a double-stranded nucleic acid can also be provided with the system. In certain example embodiments, the amplification CRISPR system comprises a first and second CRISPR/Cas complex, the first complex comprising a first CRISPR/Cas enzyme and first guide molecule that guides the first CRISPR/Cas complex to a first strand of the target nucleic acid, and the second complex comprising a second CRISPR/Cas enzyme and second guide molecule that guides the second CRISPR/Cas complex to a second strand of the target nucleic acid. In certain example embodiments, the system further comprises an amplifier primer comprising a promoter sequence also contained in the first primer of the primer pair. The system may further comprise one or more additives selected from a crowding agent, a co-factor, single-strand binding protein, and/or assisting proteins. In particular embodiments, the system further comprises a detection system, which may be a SHERLOCK detection system. In some instances, subsequent to HDA, a detection system comprising an effector protein and one or more guide molecules corresponding to the one or more amplified nucleic acids, and an RNA masking construct are provided.

### METHODS OF AMPLIFYING AND/OR DETECTING TARGET DOUBLE-STRANDED NUCLEIC ACIDS

In certain embodiments, the invention comprises a method of amplifying and/or detecting a target double-stranded nucleic acid, comprising: combining a sample comprising the target double-stranded nucleic acid with an amplification reaction mixture, the amplification reaction mixture comprising: (i) an amplification CRISPR system, the amplification CRISPR system comprising a first and second CRISPR/Cas complex, the first CRISPR/Cas complex comprising a first CRISPR/Cas enzyme and a first guide molecule that guides the first CRISPR/Cas complex to a first strand of the target nucleic acid, and the second CRISPR/Cas complex comprising a second CRISPR/Cas enzyme and a second guide molecule that guides the second CRISPR/Cas complex to a second strand of the target nucleic acid; (ii) a helicase; (iii) a primer pair comprising a first and second primer, wherein the first primer comprises a portion that is complementary to the first strand of the target nucleic acid and the second primer comprises a portion that is complementary to the second strand of the target nucleic acid; and (iv) a polymerase. The method may further comprise amplifying the target nucleic acid by opening R-loops of the target nucleic acid using the first and second CRISPR/Cas complexes, unwinding the first and the second strand of the target nucleic acid using the helicase, annealing and extending the strands using the primer pair and the polymerase. The method may further comprise further amplifying the target nucleic acid by repeated opening, unwinding, annealing and extension under isothermal conditions; and optionally detecting the amplified target nucleic acid.

In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise a target double-stranded or target single-stranded nucleic acid. A target sequence may comprise DNA or RNA polynucleotides. The term "target DNA or RNA" or "target nucleic acid" refers to a DNA or RNA polynucleotide being or comprising the target sequence. In other words, the target DNA or RNA may be a DNA or RNA polynucleotide or a part of a DNA or RNA polynucleotide to which a part of the gRNA, i.e. the guide sequence, is designed to have complementarity and to which the effector function mediated by the complex comprising CRISPR effector protein and a gRNA is to be directed. In some embodiments, a target sequence is located in the nucleus or cytoplasm of a cell. In some embodiments, the target nucleic acid may be single-stranded or double-stranded. In some embodiments, the target nucleic acid is double-stranded.

### Amplification CRISPR Systems

In specific embodiments, amplification reaction mixtures envisioned herein include an amplification CRISPR system. The amplification CRISPR system may comprise a first and second CRISPR/Cas complex. CRISPR/Cas complexes may include an active or dead CRISPR/Cas enzyme and a guide molecule that guides the CRISPR/Cas complex to a strand of the target nucleic acid. In cases where the target nucleic acid is really long, such as genomic DNA for example, active Cas enzymes may be useful. The active Cas enzymes may cut the genomic DNA allowing a helicase enzyme to enter via blunt ends generated by double-stranded DNA breaks.

In general, a CRISPR-Cas or CRISPR system as used herein and in documents, such as WO 2014/093622 (PCT/US2013/074667), refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). See, e.g, Shmakov et al. (2015) "Discovery and Functional Characterization of Diverse Class 2 CRISPR-Cas Systems", Molecular Cell, DOI: dx.doi.org/10.1016/j.molcel.2015.10.008.

In certain embodiments, a protospacer adjacent motif (PAM) or PAM-like motif directs binding of the effector protein complex as disclosed herein to the target locus of interest. In some embodiments, the PAM may be a 5' PAM (i.e., located upstream of the 5' end of the protospacer). In other embodiments, the PAM may be a 3' PAM (i.e., located downstream of the 5' end of the protospacer). The term "PAM" may be used interchangeably with the term "PFS" or "protospacer flanking site" or "protospacer flanking sequence".

Additional effectors for use according to the invention can be identified by their proximity to cas1 genes, for example, though not limited to, within the region 20 kb from the start of the cas1 gene and 20 kb from the end of the cas1 gene. In certain embodiments, the effector protein comprises at least one HEPN domain and at least 500 amino acids, and wherein the C2c2 effector protein is naturally present in a prokaryotic genome within 20 kb upstream or downstream of a Cas gene or a CRISPR array. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In certain example embodiments, the C2c2 effector protein is naturally present in a prokaryotic genome within 20kb upstream or downstream of a Cas 1 gene. The terms "orthologue" (also referred to as "ortholog" herein) and "homologue" (also referred to as "homolog" herein) are well known in the art. By means of further guidance, a "homologue" of a protein as used herein is a protein of the same species which performs the same or a similar function as the protein it is a homologue of. Homologous proteins may but need not be structurally related, or are only partially structurally related. An "orthologue" of a protein as used herein is a protein of a different species which performs the same or a similar function as the protein it is an orthologue of. Orthologous proteins may but need not be structurally related, or are only partially structurally related.

In certain example embodiments, the compositions, systems, and assays may comprise multiple Cas12 orthologs or one or more orthologs in combination with one or more Cas9 orthologs. In certain example embodiments, the Cas12 orthologs are Cpfl orthologs, C2c1orthologs, or C2c3 orthologs.

In particular embodiments, Cas9 proteins can be derived from an organism from a genus comprising *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium,* or *Corynebacte.*

In particular embodiments, the Cas9 protein is from an organism from a genus comprising *Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Leptospira, Desulfovibrio, Desulfonatronum, Opitutaceae, Tuberibacillus, Bacillus, Brevibacilus, Methylobacterium or Acidaminococcus.*

In further particular embodiments, the Cas9 protein is from an organism selected from *S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii.* In particular embodiments, the nickase is a Cas9 nickase from an organism from *Streptococcus pyogenes, Staphylococcus aureus,* or *Streptococcus thermophilus* Cas9.

In a more preferred embodiment, the Cas9 protein is derived from a bacterial species selected from the group consisting of *Streptococcus pyogenes, Staphylococcus aureus, Streptococcus thermophilus, S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii, Francisella tularensis 1, Prevotella albensis, Lachnospiraceae bacterium MC2017 1, Butyrivibrio proteoclasticus, Peregrinibacteria bacterium GW2011 GWA2 33 10, Parcubacteria bacterium GW2011 GWC2 44 17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium* ND2006, *Porphyromonas crevioricanis 3, Prevotella disiens* and *Porphyromonas macacae.*

The Cpfl gene is found in several diverse bacterial genomes, typically in the same locus with cas1, cas2, and cas4 genes and a CRISPR cassette (for example, FNFX1_1431-FNFX1_1428 of Francisella cf. novicida Fx1). Thus, the layout of this putative novel CRISPR-Cas system appears to be similar to that of type II-B. Furthermore, similar to Cas9, the Cpfl protein contains a readily identifiable C-terminal region that is homologous to the transposon ORF-B and includes an active RuvC-like nuclease, an arginine-rich region, and a Zn finger (absent in Cas9). However, unlike Cas9, Cpfl is also present in several genomes without a CRISPR-Cas context and its relatively high similarity with ORF-B suggests that it might be a transposon component. It was suggested that if this was a genuine CRISPR-Cas system and Cpfl is a functional analog of Cas9 it would be a novel CRISPR-Cas type, namely type V (See Annotation and Classification of CRISPR-Cas Systems. Makarova KS, Koonin EV. Methods Mol Biol. 2015;1311:47-75). However, as described herein, Cpfl is denoted to be in subtype V-A to distinguish it from C2c1p which does not have an identical domain structure and is hence denoted to be in subtype V-B.

In particular embodiments, Cpfl effector proteins may be derived from an organism from a genus comprising *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Leptospira, Desulfovibrio, Desulfonatronum, Opitutaceae, Tuberibacillus, Bacillus, Brevibacilus, Methylobacterium* or *Acidaminococcus.*

In further particular embodiments, the Cpfl effector protein is from an organism selected from *S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii.*

In a more preferred embodiment, the Cpfl protein may be derived from a bacterial species selected from *Francisella tularensis, Prevotella albensis, Lachnospiraceae bacterium, Butyrivibrio proteoclasticus, Peregrinibacteria bacterium, Parcubacteria bacterium, Smithella sp., Acidaminococcus sp., Lachnospiraceae bacterium, Candidatus Methanoplasma termitum, Eubacterium eligens, Moraxella bovoculi, Leptospira inadai, Porphyromonas crevioricanis, Prevotella disiens and Porphyromonas macacae, Succinivibrio dextrinosolvens, Prevotella disiens, Flavobacterium branchiophilum, Helcococcus kunzii, Eubacterium sp., Microgenomates (Roizmanbacteria) bacterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis* and *Proteocatella sphenisci.*

The present invention encompasses the use of C2c1 based nucleases, derived from a C2c1 locus denoted as subtype V-B. Herein such effector proteins are also referred to as "C2c1p", e.g., a C2c1 protein (and such effector protein or C2c1 protein or protein derived from a C2c1 locus is also called "CRISPR enzyme"). Presently, the subtype V-B loci encompasses cas1-Cas4 fusion, cas2, a distinct gene denoted C2c1 and a CRISPR array. C2c1 (CRISPR-associated protein C2c1) is a large protein (about 1100 - 1300 amino acids) that contains a RuvC-like nuclease domain homologous to the corresponding domain of Cas9 along with a counterpart to the characteristic arginine-rich cluster of Cas9. However, C2c1 lacks the HNH nuclease domain that is present in all Cas9 proteins, and the RuvC-like domain is contiguous in the C2c1 sequence, in contrast to Cas9 where it contains long inserts including the HNH domain. Accordingly, in particular embodiments, the CRISPR-Cas enzyme comprises only a RuvC-like nuclease domain.

C2c1 (also known as Cas12b) proteins are RNA guided nucleases. Its cleavage relies on a tracr RNA to recruit a guide RNA comprising a guide sequence and a direct repeat, where the guide sequence hybridizes with the target nucleotide sequence to form a DNA/RNA heteroduplex. Based on current studies, C2c1 nuclease activity also requires relies on recognition of PAM sequence. C2c1 PAM sequences are T-rich sequences. In some embodiments, the PAM sequence is 5' TTN 3' or 5' ATTN 3', wherein N is any nucleotide. In a particular embodiment, the PAM sequence is 5' TTC 3'. In a particular embodiment, the PAM is in the sequence of Plasmodium falciparum.

C2c1 creates a staggered cut at the target locus, with a 5' overhang, or a "sticky end" at the PAM distal side of the target sequence. In some embodiments, the 5' overhang is 7 nt. See Lewis and Ke, Mol Cell. 2017 Feb 2;65(3):377-379.

The C2c1 gene is found in several diverse bacterial genomes, typically in the same locus with cas1, cas2, and cas4 genes and a CRISPR cassette. Thus, the layout of this putative novel CRISPR-Cas system appears to be similar to that of type II-B. Furthermore, similar to Cas9, the C2c1 protein contains an active RuvC-like nuclease, an arginine-rich region, and a Zn finger (absent in Cas9).

In particular embodiments, the C2c1 protein may be derived from an organism from the genus comprising *Alicyclobacillus, Desulfovibrio, Desulfonatronum, Opitutaceae, Tuberibacillus, Bacillus, Brevibacillus, Candidatus, Desulfatirhabdium, Citrobacter, Elusimicrobia, Methylobacterium, Omnitrophica, Phycisphaerae, Planctomycetes, Spirochaetes,* and *Verrucomicrobiaceae.*

In further particular embodiments, C2c1 proteins can be derived from a bacterial species selected from the group consisting of *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia bacterium RIFOXYA12, Omnitrophica WOR 2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWBI 27 13, Verrucomicrobiaceae bacterium UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri (e.g., BAB-2500),* and *Methylobacterium nodulans.*

The methods and tools provided herein may be designed for use with or without Cas13, a type II nuclease that does not make use of tracrRNA. Orthologs of Cas13 have been identified in different bacterial species as described herein. Further type II nucleases with similar properties can be identified using methods described in the art (Shmakov et al. 2015, 60:385-397; Abudayeh et al. 2016, Science, 5;353(6299)) . In particular embodiments, such methods for identifying novel CRISPR effector proteins may comprise the steps of selecting sequences from the database encoding a seed which identifies the presence of a CRISPR Cas locus, identifying loci located within 10 kb of the seed comprising Open Reading Frames (ORFs) in the selected sequences, selecting therefrom loci comprising ORFs of which only a single ORF encodes a novel CRISPR effector having greater than 700 amino acids and no more than 90% homology to a known CRISPR effector. In particular embodiments, the seed is a protein that is common to the CRISPR-Cas system, such as Cas1 In further embodiments, the CRISPR array is used as a seed to identify new effector proteins.

With respect to general information on CRISPR/Cas Systems, components thereof, and delivery of such components, including methods, materials, delivery vehicles, vectors, particles, and making and using thereof, including as to amounts and formulations, as well as CRISPR-Cas-expressing eukaryotic cells, CRISPR-Cas expressing eukaryotes, such as a mouse, reference is made to: US Patents Nos. 8,999,641, 8,993,233, 8,697,359, 8,771,945, 8,795,965, 8,865,406, 8,871,445, 8,889,356, 8,889,418, 8,895,308, 8,906,616, 8,932,814, and 8,945,839; US Patent Publications US 2014-0310830 (US App. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); US 2015-0184139 (U.S. App. Ser. No. 14/324,960); 14/054,414 European Patent Applications EP 2 771 468 (EP13818570.7), EP 2 764 103 (EP13824232.6), and EP 2 784 162 (EP14170383.5); and PCT Patent Publications WO2014/093661 (PCT/US2013/074743), WO2014/093694 (PCT/US2013/074790), WO2014/093595 (PCT/US2013/074611), WO2014/093718 (PCT/US2013/074825), WO2014/093709 (PCT/US2013/074812), WO2014/093622 (PCT/US2013/074667), WO2014/093635 (PCT/US2013/074691), WO2014/093655 (PCT/US2013/074736), WO2014/093712 (PCT/US2013/074819), WO2014/093701 (PCT/US2013/074800), WO2014/018423 (PCT/US2013/051418), WO2014/204723 (PCT/US2014/041790), WO2014/204724 (PCT/US2014/041800), WO2014/204725 (PCT/US2014/041803), WO2014/204726 (PCT/US2014/041804), WO2014/204727 (PCT/US2014/041806), WO2014/204728 (PCT/US2014/041808), WO2014/204729 (PCT/US2014/041809), WO2015/089351 (PCT/US2014/069897), WO2015/089354 (PCT/US2014/069902), WO2015/089364 (PCT/US2014/069925), WO2015/089427 (PCT/US2014/070068), WO2015/089462 (PCT/US2014/070127), WO2015/089419 (PCT/US2014/070057), WO2015/089465 (PCT/US2014/070135), WO2015/089486 (PCT/US2014/070175), WO2015/058052 (PCT/US2014/061077), WO2015/070083 (PCT/US2014/064663), WO2015/089354 (PCT/US2014/069902), WO2015/089351 (PCT/US2014/069897), WO2015/089364 (PCT/US2014/069925), WO2015/089427 (PCT/US2014/070068), WO2015/089473 (PCT/US2014/070152), WO2015/089486 (PCT/US2014/070175), WO2016/049258 (PCT/US2015/051830), WO2016/094867 (PCT/US2015/065385), WO2016/094872 (PCT/US2015/065393), WO2016/094874 (PCT/US2015/065396), WO2016/106244 (PCT/US2015/067177).

Mention is also made of US application 62/180,709, 17-Jun-15, PROTECTED GUIDE RNAS (PGRNAS); US application 62/091,455, filed, 12-Dec-14, PROTECTED GUIDE RNAS (PGRNAS); US application 62/096,708, 24-Dec-14, PROTECTED GUIDE RNAS (PGRNAS); US applications 62/091,462, 12-Dec-14, 62/096,324, 23-Dec-14, 62/180,681, 17-Jun-2015, and 62/237,496, 5-Oct-2015, DEAD GUIDES FOR CRISPR TRANSCRIPTION FACTORS; US application 62/091,456, 12-Dec-14 and 62/180,692, 17-Jun-2015, ESCORTED AND FUNCTIONALIZED GUIDES FOR CRISPR-CAS SYSTEMS; US application 62/091,461, 12-Dec-14, DELIVERY, USE AND THERAPEUTIC APPLICATIONS OF THE CRISPR-CAS SYSTEMS AND COMPOSITIONS FOR GENOME EDITING AS TO HEMATOPOETIC STEM CELLS (HSCs); US application 62/094,903, 19-Dec-14, UNBIASED IDENTIFICATION OF DOUBLE-STRAND BREAKS AND GENOMIC REARRANGEMENT BY GENOME-WISE INSERT CAPTURE SEQUENCING; US application 62/096,761, 24-Dec-14, ENGINEERING OF SYSTEMS, METHODS AND OPTIMIZED ENZYME AND GUIDE SCAFFOLDS FOR SEQUENCE MANIPULATION; US application 62/098,059, 30-Dec-14, 62/181,641, 18-Jun-2015, and 62/181,667, 18-Jun-2015, RNA-TARGETING SYSTEM; US application 62/096,656, 24-Dec-14 and 62/181,151, 17-Jun-2015, CRISPR HAVING OR ASSOCIATED WITH DESTABILIZATION DOMAINS; US application 62/096,697, 24-Dec-14, CRISPR HAVING OR ASSOCIATED WITH AAV; US application 62/098,158, 30-Dec-14, ENGINEERED CRISPR COMPLEX INSERTIONAL TARGETING SYSTEMS; US application 62/151,052, 22-Apr-15, CELLULAR TARGETING FOR EXTRACELLULAR EXOSOMAL REPORTING; US application 62/054,490, 24-Sep-14, DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS; US application 61/939,154, 12-F EB-14, SYSTEMS, METHODS AND COMPOSITIONS FOR SEQUENCE MANIPULATION WITH OPTIMIZED FUNCTIONAL CRISPR-CAS SYSTEMS; US application 62/055,484, 25-Sep-14, SYSTEMS, METHODS AND COMPOSITIONS FOR SEQUENCE MANIPULATION WITH OPTIMIZED FUNCTIONAL CRISPR-CAS SYSTEMS; US application 62/087,537, 4-Dec-14, SYSTEMS, METHODS AND COMPOSITIONS FOR SEQUENCE MANIPULATION WITH OPTIMIZED FUNCTIONAL CRISPR-CAS SYSTEMS; US application 62/054,651, 24-Sep-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/067,886, 23-Oct-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US applications 62/054,675, 24-Sep-14 and 62/181,002, 17-Jun-2015, DELIVERY, USE AND THERAPEUTIC APPLICATIONS OF THE CRISPR-CAS SYSTEMS AND COMPOSITIONS IN NEURONAL CELLS/TISSUES; US application 62/054,528, 24-Sep-14, DELIVERY, USE AND THERAPEUTIC APPLICATIONS OF THE CRISPR-CAS SYSTEMS AND COMPOSITIONS IN IMMUNE DISEASES OR DISORDERS; US application 62/055,454, 25-Sep-14, DELIVERY, USE AND THERAPEUTIC APPLICATIONS OF THE CRISPR-CAS SYSTEMS AND COMPOSITIONS FOR TARGETING DISORDERS AND DISEASES USING CELL PENETRATION PEPTIDES (CPP); US application 62/055,460, 25-Sep-14, MULTIFUNCTIONAL-CRISPR COMPLEXES AND/OR OPTIMIZED ENZYME LINKED FUNCTIONAL-CRISPR COMPLEXES; US application 62/087,475, 4-Dec-14 and 62/181,690, 18-Jun-2015, FUNCTIONAL SCREENING WITH OPTIMIZED FUNCTIONAL CRISPR-CAS SYSTEMS; US application 62/055,487, 25-Sep-14, FUNCTIONAL SCREENING WITH OPTIMIZED FUNCTIONAL CRISPR-CAS SYSTEMS; US application 62/087,546, 4-Dec-14 and 62/181,687, 18-Jun-2015, MULTIFUNCTIONAL CRISPR COMPLEXES AND/OR OPTIMIZED ENZYME LINKED FUNCTIONAL-CRISPR COMPLEXES; and US application 62/098,285, 30-Dec-14, CRISPR MEDIATED IN VIVO MODELING AND GENETIC SCREENING OF TUMOR GROWTH AND METASTASIS.

Mention is made of US applications 62/181,659, 18-Jun-2015 and 62/207,318, 19-Aug-2015, ENGINEERING AND OPTIMIZATION OF SYSTEMS, METHODS, ENZYME AND GUIDE SCAFFOLDS OF CAS9 ORTHOLOGS AND VARIANTS FOR SEQUENCE MANIPULATION. Mention is made of US applications 62/181,663, 18-Jun-2015 and 62/245,264, 22-Oct-2015, NOVEL CRISPR ENZYMES AND SYSTEMS, US applications 62/181,675, 18-Jun-2015, 62/285,349, 22-Oct-2015, 62/296,522, 17-Feb-2016, and 62/320,231, 8-Apr-2016, NOVEL CRISPR ENZYMES AND SYSTEMS, US application 62/232,067, 24-Sep-2015, US Application 14/975,085, 18-Dec-2015, European application No. 16150428.7, US application 62/205,733, 16-Aug-2015, US application 62/201,542, 5-Aug-2015, US application 62/193,507, 16-Jul-2015, and US application 62/181,739, 18-Jun-2015, each entitled NOVEL CRISPR ENZYMES AND SYSTEMS and of US application 62/245,270, 22-Oct-2015, NOVEL CRISPR ENZYMES AND SYSTEMS. Mention is also made of US application 61/939,256, 12-Feb-2014, and WO 2015/089473 (PCT/US2014/070152), 12-Dec-2014, each entitled ENGINEERING OF SYSTEMS, METHODS AND OPTIMIZED GUIDE COMPOSITIONS WITH NEW ARCHITECTURES FOR SEQUENCE MANIPULATION. Mention is also made of PCT/US2015/045504, 15-Aug-2015, US application 62/180,699, 17-Jun-2015, and US application 62/038,358, 17-Aug-2014, each entitled GENOME EDITING USING CAS9 NICKASES. Mention is made to detection methods disclosed in US applications 62/610,066 filed December 22, 2017; 62/623,546 filed January 29, 2018, and 62/630,814 filed February 14, 2018, each entitled CRISPR EFFECTOR SYSTEM BASED MULTIPLEX DIAGNOSTICS.

### Dead CRISPR/Cas Enzymes

In particular embodiments, it is of interest to make use of an engineered Cas protein as defined herein, such as Cas9, wherein the protein complexes with a nucleic acid molecule comprising RNA to form a CRISPR complex, wherein when in the CRISPR complex, the nucleic acid molecule targets one or more target polynucleotide loci, the protein comprises at least one modification compared to unmodified Cas protein, and wherein the CRISPR complex comprising the modified protein has altered activity as compared to the complex comprising the unmodified Cas protein. It is to be understood that when referring herein to CRISPR "protein", the Cas protein preferably is a modified CRISPR-Cas protein (e.g. having increased or decreased (or no) enzymatic activity, such as without limitation including Cas9. The term "CRISPR protein" may be used interchangeably with "CRISPR-Cas protein", irrespective of whether the CRISPR protein has altered, such as increased or decreased (or no) enzymatic activity, compared to the wild type CRISPR protein. As used herein, the term "modified" with regard to a CRISPR-Cas protein generally refers to a CRISPR-Cas protein having one or more modifications or mutations (including point mutations, truncations, insertions, deletions, chimeras, fusion proteins, etc.) compared to the wild type Cas protein from which it is derived. By derived is meant that the derived enzyme is largely based, in the sense of having a high degree of sequence homology with, a wildtype enzyme, but that it has been mutated (modified) in some way as known in the art or as described herein. Alternatively, such a modified protein with no enzymatic activity may be referred to as a dead protein, or dead CRISPR/Cas enzyme.

The additional modifications of the CRISPR-Cas protein may or may not cause an altered functionality. By means of example, and in particular with reference to CRISPR-Cas protein, modifications which do not result in an altered functionality include for instance codon optimization for expression into a particular host, or providing the nuclease with a particular marker (e.g. for visualization). Modifications with may result in altered functionality may also include mutations, including point mutations, insertions, deletions, truncations (including split nucleases), etc., as well as chimeric nucleases (e.g. comprising domains from different orthologues or homologues) or fusion proteins. Fusion proteins may without limitation include for instance fusions with heterologous domains or functional domains (e.g. localization signals, catalytic domains, etc.). In certain embodiments, various different modifications may be combined (e.g. a mutated nuclease which is catalytically inactive and which further is fused to a functional domain, such as for instance to induce DNA methylation or another nucleic acid modification, such as including without limitation a break (e.g. by a different nuclease (domain)), a mutation, a deletion, an insertion, a replacement, a ligation, a digestion, a break or a recombination). As used herein, "altered functionality" includes without limitation an altered specificity (e.g. altered target recognition, increased (e.g. "enhanced" Cas proteins) or decreased specificity, or altered PAM recognition), altered activity (e.g. increased or decreased catalytic activity, including catalytically inactive nucleases or nickases), and/or altered stability (e.g. fusions with destabilization domains). Suitable heterologous domains include without limitation a nuclease, a ligase, a repair protein, a methyltransferase, (viral) integrase, a recombinase, a transposase, an argonaute, a cytidine deaminase, a retron, a group II intron, a phosphatase, a phosphorylase, a sulpfurylase, a kinase, a polymerase, an exonuclease, etc. Examples of all these modifications are known in the art. It will be understood that a "modified" nuclease as referred to herein, and in particular a "modified" Cas or "modified" CRISPR-Cas system or complex preferably still has the capacity to interact with or bind to the polynucleic acid (e.g. in complex with the guide molecule). Such modified Cas protein can be combined with the deaminase protein or active domain thereof as described herein.

In certain embodiments, CRISPR-Cas protein may comprise one or more modifications resulting in enhanced activity and/or specificity, such as including mutating residues that stabilize the targeted or non-targeted strand (e.g. eCas9; "Rationally engineered Cas9 nucleases with improved specificity", Slaymaker et al. (2016), Science, 351(6268):84-88). In certain embodiments, the altered or modified activity of the engineered CRISPR protein comprises increased targeting efficiency or decreased off-target binding. In certain embodiments, the altered activity of the engineered CRISPR protein comprises modified cleavage activity. In certain embodiments, the altered activity comprises increased cleavage activity as to the target polynucleotide loci. In certain embodiments, the altered activity comprises decreased cleavage activity as to the target polynucleotide loci. In certain embodiments, the altered activity comprises decreased cleavage activity as to off-target polynucleotide loci. In certain embodiments, the altered or modified activity of the modified nuclease comprises altered helicase kinetics. In certain embodiments, the modified nuclease comprises a modification that alters association of the protein with the nucleic acid molecule comprising RNA (in the case of a Cas protein), or a strand of the target polynucleotide loci, or a strand of off-target polynucleotide loci. In an aspect of the invention, the engineered CRISPR protein comprises a modification that alters formation of the CRISPR complex. In certain embodiments, the altered activity comprises increased cleavage activity as to off-target polynucleotide loci. Accordingly, in certain embodiments, there is increased specificity for target polynucleotide loci as compared to off-target polynucleotide loci. In other embodiments, there is reduced specificity for target polynucleotide loci as compared to off-target polynucleotide loci. In certain embodiments, the mutations result in decreased off-target effects (e.g. cleavage or binding properties, activity, or kinetics), such as in case for Cas proteins for instance resulting in a lower tolerance for mismatches between target and guide RNA. Other mutations may lead to increased off-target effects (e.g. cleavage or binding properties, activity, or kinetics). Other mutations may lead to increased or decreased on-target effects (e.g. cleavage or binding properties, activity, or kinetics). In certain embodiments, the mutations result in altered (e.g. increased or decreased) helicase activity, association or formation of the functional nuclease complex (e.g. CRISPR-Cas complex). In certain embodiments, the mutations result in an altered PAM recognition, i.e. a different PAM may be (in addition or in the alternative) be recognized, compared to the unmodified Cas protein (see e.g. "Engineered CRISPR-Cas9 nucleases with altered PAM specificities", Kleinstiver et al. (2015), Nature, 523(7561):481-485). Particularly preferred mutations include positively charged residues and/or (evolutionary) conserved residues, such as conserved positively charged residues, in order to enhance specificity. In certain embodiments, such residues may be mutated to uncharged residues, such as alanine.

In particular embodiments, it is of interest to make use of an engineered Cas9 protein as defined herein, such as Cas9, wherein the protein complexes with a nucleic acid molecule comprising RNA to form a CRISPR complex, wherein when in the CRISPR complex, the nucleic acid molecule targets one or more target polynucleotide loci, the protein comprises at least one modification compared to unmodified Cas protein, and wherein the CRISPR complex comprising the modified protein has altered activity as compared to the complex comprising the unmodified Cas9 protein. It is to be understood that when referring herein to CRISPR "protein", the Cas protein preferably is a modified CRISPR-Cas protein (e.g. having increased or decreased (or no) enzymatic activity, such as without limitation including Cas9. The term "CRISPR protein" may be used interchangeably with "CRISPR-Cas protein", irrespective of whether the CRISPR protein has altered, such as increased or decreased (or no) enzymatic activity, compared to the wild type CRISPR protein.

In particular embodiments, the homologue or orthologue of Cas as referred to herein has a sequence homology or identity of at least 80%, more preferably at least 85%, even more preferably at least 90%, such as for instance at least 95% with Cas9. In further embodiments, the homologue or orthologue of Cas as referred to herein has a sequence identity of at least 80%, more preferably at least 85%, even more preferably at least 90%, such as for instance at least 95% with the wild type Cas. Where the Cas has one or more mutations (mutated), the homologue or orthologue of said Cas as referred to herein has a sequence identity of at least 80%, more preferably at least 85%, even more preferably at least 90%, such as for instance at least 95% with the mutated Cas.

In certain embodiments, one or more catalytic domains of a Cas9 protein (e.g. RuvC I, RuvC II, and RuvC III or the HNH domain of a Cas9 protein) are mutated to produce a mutated Cas protein which cleaves only one DNA strand of a target sequence.

By means of further guidance, and without limitation, for example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As further guidance, where the enzyme is not SpCas9, mutations may be made at any or all residues corresponding to positions 10, 762, 840, 854, 863 and/or 986 of SpCas9 (which may be ascertained for instance by standard sequence comparison tools). In particular, any or all of the following mutations are preferred in SpCas9: D10A, E762A, H840A, N854A, N863A and/or D986A; as well as conservative substitution for any of the replacement amino acids is also envisaged.

In one embodiment, the CRISPR-Cas protein is SpCas9 nickase having a catalytically inactive HNH domain (e.g., an SpCas9 nickase with N863A mutation). In another embodiment, the CRISPR-Cas protein is SaCas9 having a catalytically inactive HNH domain (e.g., an SaCas9 nickase with N580A mutation). In yet another embodiment, the CRISPR-Cas protein is SpCas9 nickase having the HNH domain partially or fully removed. In yet another embodiment, the CRISPR-Cas protein is SaCas9 having the HNH domain partially or fully removed.

In certain of the above-described Cas9 enzymes, the enzyme is modified by mutation of one or more residues including but not limited to positions D917, E1006, E1028, D1227, D1255A, N1257, according to FnCas9 protein or any corresponding ortholog. In an aspect the invention provides a herein-discussed composition wherein the Cas9 enzyme is an inactivated enzyme which comprises one or more mutations selected from the group consisting of D917A, E1006A, E1028A, D1227A, D1255A and N1257A according to FnCas9 protein or corresponding positions in a Cas9 ortholog. In an aspect the invention provides a herein-discussed composition, wherein the CRISPR-Cas protein comprises D917, or E1006 and D917, or D917 and D1255, according to FnCas9 protein or a corresponding position in a Cas9 ortholog.

In certain embodiments, the modification or mutation of Cas9 comprises a mutation in a RuvCI, RuvCIII, RuvCIII or HNH domain. In certain embodiments, the modification or mutation comprises an amino acid substitution at one or more of positions 12, 13, 63, 415, 610, 775, 779, 780, 810, 832, 848, 855, 861, 862, 866, 961, 968, 974, 976, 982, 983, 1000, 1003, 1014, 1047, 1060, 1107, 1108, 1109, 1114, 1129, 1240, 1289, 1296, 1297, 1300, 1311, and 1325; preferably 855; 810, 1003, and 1060; or 848, 1003 with reference to amino acid position numbering of SpCas9.In certain embodiments, the modification or mutation at position 63, 415, 775, 779, 780, 810, 832, 848, 855, 861, 862, 866, 961, 968, 974, 976, 982, 983, 1000, 1003, 1014, 1047, 1060, 1107, 1108, 1109, 1114, 1129, 1240, 1289, 1296, 1297, 1300, 1311, or 1325; preferably 855; 810, 1003, and 1060; 848, 1003, and 1060; or 497, 661, 695, and 926 comprises an alanine substitution. In certain embodiments, the modification comprises K855A; K810A, K1003A, and R1060A; or K848A, K1003A (with reference to SpCas9), and R1060A. in certain embodiments, in certain embodiments, the modification comprises N497A, R661A, Q695A, and Q926A (with reference to SpCas9).

As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form. Where the enzyme is not SpCas9, mutations may be made at any or all residues corresponding to positions 10, 762, 840, 854, 863 and/or 986 of SpCas9 (which may be ascertained for instance by standard sequence comparison tools. In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred.

In certain example embodiments, the compositions, systems, and assays may comprise multiple Cas12 orthologs or one or more orthologs in combination with one or more Cas9 orthologs. In certain example embodiments, the Cas12 orthologs are Cpfl orthologs, C2c1orthologs, or C2c3 orthologs.

The present invention encompasses the use of a nickases based on mutated forms of wild type Cpfl effector protein, derived from a Cpfl locus denoted as subtype V-A. Herein such effector proteins are also referred to as "Cpflp", e.g., a Cpfl protein (and such effector protein or Cpfl protein or protein derived from a Cpfl locus is also called "CRISPR enzyme"). Presently, the subtype V-A loci encompasses cas1, cas2, a distinct gene denoted cpf1 and a CRISPR array. Cpf1(CRISPR-associated protein Cpfl, subtype PREFRAN) is a large protein (about 1300 amino acids) that contains a RuvC-like nuclease domain homologous to the corresponding domain of Cas9 along with a counterpart to the characteristic arginine-rich cluster of Cas9. However, Cpfl lacks the HNH nuclease domain that is present in all Cas9 proteins, and the RuvC-like domain is contiguous in the Cpfl sequence, in contrast to Cas9 where it contains long inserts including the HNH domain. Accordingly, in particular embodiments, the CRISPR-Cas enzyme comprises only a RuvC-like nuclease domain.

In particular embodiments, the homologue or orthologue of Cpfl as referred to herein has a sequence homology or identity of at least 80%, more preferably at least 85%, even more preferably at least 90%, such as for instance at least 95% with Cpfl. In further embodiments, the homologue or orthologue of Cpfl as referred to herein has a sequence identity of at least 80%, more preferably at least 85%, even more preferably at least 90%, such as for instance at least 95% with the wild type Cpfl. Where the Cpfl has one or more mutations (mutated), the homologue or orthologue of said Cpfl as referred to herein has a sequence identity of at least 80%, more preferably at least 85%, even more preferably at least 90%, such as for instance at least 95% with the mutated Cpfl.

In an embodiment, the Cpfl protein may be an ortholog of an organism of a genus which includes, but is not limited to *Acidaminococcus sp, Lachnospiraceae bacterium* or *Moraxella bovoculi*; in particular embodiments, the type V Cas protein may be an ortholog of an organism of a species which includes, but is not limited to *Acidaminococcus sp. BV3L6; Lachnospiraceae bacterium ND2006 (LbCpf1)* or *Moraxella bovoculi 237.* In particular embodiments, the homologue or orthologue of Cpfl as referred to herein has a sequence homology or identity of at least 80%, more preferably at least 85%, even more preferably at least 90%, such as for instance at least 95% with one or more of the Cpfl sequences disclosed herein. In further embodiments, the homologue or orthologue of Cpfl as referred to herein has a sequence identity of at least 80%, more preferably at least 85%, even more preferably at least 90%, such as for instance at least 95% with the wild type FnCpf1, AsCpfl or LbCpfl.

In particular embodiments, the Cpfl protein of the invention has a sequence homology or identity of at least 60%, more particularly at least 70, such as at least 80%, more preferably at least 85%, even more preferably at least 90%, such as for instance at least 95% with FnCpf1, AsCpfl or LbCpfl. In further embodiments, the Cpfl protein as referred to herein has a sequence identity of at least 60%, such as at least 70%, more particularly at least 80%, more preferably at least 85%, even more preferably at least 90%, such as for instance at least 95% with the wild type AsCpfl or LbCpfl. In particular embodiments, the Cpfl protein of the present invention has less than 60% sequence identity with FnCpf1. The skilled person will understand that this includes truncated forms of the Cpfl protein whereby the sequence identity is determined over the length of the truncated form.

In some embodiments, the Cpfl protein comprises a mutation in the Nuc domain. In some embodiments, the Cpfl protein is capable of nicking a non-targeted DNA strand at the target locus of interest displaced by the formation of the heteroduplex between the targeted DNA strand and the guide molecule. In some embodiments, the Cpfl protein comprises a mutation corresponding to R1226A in AsCpf1.

By means of further guidance, and without limitation, an arginine-to-alanine substitution (R1226A) in the Nuc domain of Cpfl from Acidaminococcus sp. converts Cpfl from a nuclease that cleaves both strands to a nickase (cleaves a single strand). It will be understood by the skilled person that where the enzyme is not AsCpfl, a mutation may be made at a residue in a corresponding position. In particular embodiments, the Cpfl is FnCpf1 and the mutation is at the arginine at position R1218. In particular embodiments, the Cpfl is LbCpf1 and the mutation is at the arginine at position R1138. In particular embodiments, the Cpfl is MbCpf1 and the mutation is at the arginine at position R1293.

In certain example embodiments the invention comprises a catalytically inactive C2c1 which comprises a mutation in the RuvC domain. In some embodiments, the catalytically inactive C2c1 protein comprises a mutation corresponding to amino acid positions D570, E848, or D977 in *Alicyclobacillus acidoterrestris* C2c1. In some embodiments, the catalytically inactive C2c1 protein comprises a mutation corresponding to D570A, E848A, or D977A in *Alicyclobacillus acidoterrestris* C2c1.

In certain embodiments, the Cas-based nickase is a C2c1 nickase which comprises a mutation in the Nuc domain. In some embodiments, the C2c1 nickase comprises a mutation corresponding to amino acid positions R911, R1000, or R1015 in *Alicyclobacillus acidoterrestris* C2c1. In some embodiments, the C2c1 nickase comprises a mutation corresponding to R911A, R1000A, or R1015A in *Alicyclobacillus acidoterrestris* C2c1. It will be understood by the skilled person that where the enzyme is not the CRISPR-Cas enzyme listed above, a mutation may be made at a residue in a corresponding position.

Mutations can also be made at neighboring residues, e.g., at amino acids near those indicated above that participate in the nuclease activity. In some embodiments, only the RuvC domain is inactivated, and in other embodiments, another putative nuclease domain is inactivated, wherein the effector protein complex functions as a nickase and cleaves only one DNA strand. In some embodiments, two CRISPR-Cas variants (each a different nickase) are used to increase specificity, two nickase variants are used to cleave DNA at a target (where both nickases cleave a DNA strand, while minimizing or eliminating off-target modifications where only one DNA strand is cleaved and subsequently repaired).

In certain embodiments the C2c1 effector protein cleaves sequences associated with or at a target locus of interest as a homodimer comprising two C2c1 effector protein molecules. In a preferred embodiment the homodimer may comprise two C2c1 effector protein molecules comprising a different mutation in their respective RuvC domains.

In particular embodiments, the homologue or orthologue of C2c1 as referred to herein has a sequence homology or identity of at least 80%, more preferably at least 85%, even more preferably at least 90%, such as for instance at least 95% with C2c1. In further embodiments, the homologue or orthologue of C2c1 as referred to herein has a sequence identity of at least 80%, more preferably at least 85%, even more preferably at least 90%, such as for instance at least 95% with the wild type C2c1. Where the C2c1 has one or more mutations (mutated), the homologue or orthologue of said C2c1 as referred to herein has a sequence identity of at least 80%, more preferably at least 85%, even more preferably at least 90%, such as for instance at least 95% with the mutated C2c1.

In specific embodiments, the dead CRISPR/Cas enzyme may include, but is not necessarily limited to, dead Cas9, dead Cpfl, dead C2c1, or dead C2c3. In specific embodiments, the dead CRISPR/Cas enzyme may be a dead Cas9 protein which comprises a mutation in the HNH and RuvC domain. In specific embodiments, the dead CRISPR/Cas enzyme may be a dead Cas9 protein which comprises a mutation corresponding to D10A and N863A in SpCas9, or D10A and H840A in SpCas9. In specific embodiments, the dead CRISPR/Cas enzyme may be a dead Cpfl protein comprising a mutation in the RuvC domain. In specific embodiments, the dead CRISPR/Cas enzyme may be a dead Cpfl protein comprising a mutation corresponding to D908A or E993A in AsCpfl. In specific embodiments, the dead CRISPR/Cas enzyme may be a dead C2c1 protein comprising a mutation in the Nuc domain. In specific embodiments, the dead CRISPR/Cas enzyme may be a dead C2c1 protein comprising a mutation corresponding to D570A, E848A, or D977A in AacC2c1.

In specific embodiments, the dead CRISPR/Cas enzyme may be a dead Cas9 protein derived from a bacterial species selected from the group consisting of *Streptococcus pyogenes, Staphylococcus aureus, Streptococcus thermophilus, S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii, Francisella tularensis 1, Prevotella albensis, Lachnospiraceae bacterium MC2017 1, Butyrivibrio proteoclasticus, Peregrinibacteria bacterium GW2011 GWA2 33 10, Parcubacteria bacterium GW2011 GWC2 44 17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens* and *Porphyromonas macacae.*

In specific embodiments, the dead CRISPR/Cas enzyme may be a Cpfl protein derived from a bacterial species selected from the group consisting of *Francisella tularensis, Prevotella albensis, Lachnospiraceae bacterium, Butyrivibrio proteoclasticus, Peregrinibacteria bacterium, Parcubacteria bacterium, Smithella sp., Acidaminococcus sp., Lachnospiraceae bacterium, Candidatus Methanoplasma termitum, Eubacterium eligens, Moraxella bovoculi, Leptospira inadai, Porphyromonas crevioricanis, Prevotella disiens and Porphyromonas macacae, Succinivibrio dextrinosolvens, Prevotella disiens, Flavobacterium branchiophilum, Helcococcus kunzii, Eubacterium sp., Microgenomates (Roizmanbacteria) bacterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis* and *Proteocatella sphenisci.*

In specific embodiments, the dead CRISPR/Cas enzyme may be a C2c1 protein derived from a bacterial species selected from the group consisting of *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia bacterium RIFOAXYA12, Omnitrophica WOR 2 bacterium RIFCSPHIGHO2, Opitutaceae bacterium TAV5, Phycisphaerae bacterium ST-NAGAB-D1, Planctomycetes bacterium RBG 13 46 10, Spirochaetes bacterium GWB1 27 13, Verrucomicrobiaceae bacterium UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri (e.g., BAB-2500),* and *Methylobacterium nodulans.*

In certain embodiments, the dead CRISPR/Cas enzyme is a dead Cas9, dead Cas12a, dead Cas12b, or dead Cas12c.

In certain embodiments, the dead CRISPR/Cas enzyme is a dead Cas9 protein which comprises a mutation in the HNH and RuvC domain.

In certain embodiments, the dead CRISPR/Cas enzyme is a dead Cas9 protein which comprises a mutation corresponding to D10A and N863A in SpCas9, or D10A and H840A in SpCas9.

In certain embodiments, the dead CRISPR/Cas enzyme is a dead Cas12 protein. In specific embodiments, the dead Cas12 protein comprises a mutation in the RuvC domain.

In certain embodiments, the dead Cas12 enzyme is a dead Cas12a, Cas12b, Cas12c. In specific embodiments, the dead Cas12a comprises a mutation corresponding to D908A or E993A in AsCpfl.

In some embodiments, the dead Cas12 protein is derived from a bacterial species selected from the group consisting of *Francisella tularensis, Prevotella albensis, Lachnospiraceae bacterium, Butyrivibrio proteoclasticus, Peregrinibacteria bacterium, Parcubacteria bacterium, Smithella sp., Acidaminococcus sp., Lachnospiraceae bacterium, Candidatus Methanoplasma termitum, Eubacterium eligens, Moraxella bovoculi, Leptospira inadai, Porphyromonas crevioricanis, Prevotella disiens and Porphyromonas macacae, Succinivibrio dextrinosolvens, Prevotella disiens, Flavobacterium branchiophilum, Helcococcus kunzii, Eubacterium sp., Microgenomates (Roizmanbacteria) bacterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis and Proteocatella sphenisci.*

In specific embodiments, the dead Cas12 is a dead Cas12b protein which comprises a mutation in the Nuc domain. In specific embodiments, the dead Cas12b comprises a mutation corresponding to D570A, E848A, or D977A in AacC2c1.

In certain embodiments, the dead Cas12b protein is derived from a bacterial species selected from the group consisting of *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWBI 27 13, Verrucomicrobiaceae bacterium UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri (e.g., BAB-2500), andMethylobacterium nodulans.*

In certain embodiments, the first dead CRISPR/Cas enzyme and the second dead CRISPR/Cas enzyme are the same. In certain embodiments, the first dead CRISPR/Cas enzyme and the second dead CRISPR/Cas enzyme are different. In certain embodiments, the dead CRISPR/Cas enzyme is a dCas9, dCas12a, dCas12b, dCas12c, or dCas14.

In certain embodiments, the polymerase is selected from the group consisting of Bst 2.0 DNA polymerase, Bst 2.0 Warm Start DNA polymerase, Bst 3.0 DNA polymerase, full length Bst DNA polymerase, large fragment Bst DNA polymerase, large fragment Bsu DNA polymerase, phi29 DNA polymerase, T7 DNA polymerase, Gst polymerase, Taq polymerase, Klenow fragment of E. coli DNA polymerase I, KlenTaq DNA polymerase, Pol III DNA polymerase, T5 DNA polymerase, and Sequenase DNA polymerase.

In certain embodiments, the helicase is selected from the group consisting of UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase.

### Guide Sequences

As used herein, the term "guide sequence," "crRNA," "guide RNA," or "single guide RNA," or "gRNA" refers to a polynucleotide comprising any polynucleotide sequence having sufficient complementarity with a target nucleic acid sequence to hybridize with the target nucleic acid sequence and to direct sequence-specific binding of a RNA-targeting complex comprising the guide sequence and a CRISPR effector protein to the target nucleic acid sequence. In some example embodiments, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a nucleic acid-targeting complex to a target nucleic acid sequence may be assessed by any suitable assay. For example, the components of a nucleic acid-targeting CRISPR system sufficient to form a nucleic acid-targeting complex, including the guide sequence to be tested, may be provided to a host cell having the corresponding target nucleic acid sequence, such as by transfection with vectors encoding the components of the nucleic acid-targeting complex, followed by an assessment of preferential targeting (e.g., cleavage) within the target nucleic acid sequence, such as by Surveyor assay as described herein. Similarly, cleavage of a target nucleic acid sequence may be evaluated in a test tube by providing the target nucleic acid sequence, components of a nucleic acid-targeting complex, including the guide sequence to be tested and a control guide sequence different from the test guide sequence, and comparing binding or rate of cleavage at the target sequence between the test and control guide sequence reactions. Other assays are possible, and will occur to those skilled in the art. A guide sequence, and hence a nucleic acid-targeting guide may be selected to target any target nucleic acid sequence. The target sequence may be DNA. The target sequence may be any RNA sequence. In some embodiments, the target sequence may be a sequence within a RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (lncRNA), and small cytoplasmic RNA (scRNA). In some preferred embodiments, the target sequence may be a sequence within a RNA molecule selected from the group consisting of mRNA, pre-mRNA, and rRNA. In some preferred embodiments, the target sequence may be a sequence within a RNA molecule selected from the group consisting of ncRNA, and lncRNA. In some more preferred embodiments, the target sequence may be a sequence within an mRNA molecule or a pre-mRNA molecule.

In some embodiments, a nucleic acid-targeting guide is selected to reduce the degree secondary structure within the nucleic acid-targeting guide. In some embodiments, about or less than about 75%, 50%, 40%, 30%, 25%, 20%, 15%, 10%, 5%, 1%, or fewer of the nucleotides of the nucleic acid-targeting guide participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g., A.R. Gruber et al., 2008, Cell 106(1): 23-24; and PA Carr and GM Church, 2009, Nature Biotechnology 27(12): 1151-62).

In certain embodiments, a guide RNA or crRNA may comprise, consist essentially of, or consist of a direct repeat (DR) sequence and a guide sequence or spacer sequence. In certain embodiments, the guide RNA or crRNA may comprise, consist essentially of, or consist of a direct repeat sequence fused or linked to a guide sequence or spacer sequence. In certain embodiments, the direct repeat sequence may be located upstream (i.e., 5') from the guide sequence or spacer sequence. In other embodiments, the direct repeat sequence may be located downstream (i.e., 3') from the guide sequence or spacer sequence.

In certain embodiments, the crRNA comprises a stem loop, preferably a single stem loop. In certain embodiments, the direct repeat sequence forms a stem loop, preferably a single stem loop.

In certain embodiments, the spacer length of the guide RNA is from 15 to 35 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27-30 nt, e.g., 27, 28, 29, or 30 nt, from 30-35 nt, e.g., 30, 31, 32, 33, 34, or 35 nt, or 35 nt or longer.

In general, the CRISPR-Cas, CRISPR-Cas9 or CRISPR system may be as used in the foregoing documents, such as WO 2014/093622 (PCT/US2013/074667) and refers collectively to transcripts and other elements involved in the expression of or directing the activity of CRISPR-associated ("Cas") genes, including sequences encoding a Cas gene, in particular a Cas9 gene in the case of CRISPR-Cas9, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. The section of the guide sequence through which complementarity to the target sequence is important for cleavage activity is referred to herein as the seed sequence. A target sequence may comprise any polynucleotide, such as DNA or RNA polynucleotides. In some embodiments, a target sequence is located in the nucleus or cytoplasm of a cell, and may include nucleic acids in or from mitochondrial, organelles, vesicles, liposomes or particles present within the cell. In some embodiments, especially for non-nuclear uses, NLSs are not preferred. In some embodiments, a CRISPR system comprises one or more nuclear exports signals (NESs). In some embodiments, a CRISPR system comprises one or more NLSs and one or more NESs. In some embodiments, direct repeats may be identified *in silico* by searching for repetitive motifs that fulfill any or all of the following criteria: 1. found in a 2Kb window of genomic sequence flanking the type II CRISPR locus; 2. span from 20 to 50 bp; and 3. interspaced by 20 to 50 bp. In some embodiments, 2 of these criteria may be used, for instance 1 and 2, 2 and 3, or 1 and 3. In some embodiments, all 3 criteria may be used.

In embodiments of the invention the terms guide sequence and guide RNA, i.e. RNA capable of guiding Cas to a target genomic locus, are used interchangeably as in foregoing cited documents such as WO 2014/093622 (PCT/US2013/074667). In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. Preferably the guide sequence is 10 30 nucleotides long. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

In some embodiments of CRISPR-Cas systems, the degree of complementarity between a guide sequence and its corresponding target sequence can be about or more than about 50%, 60%, 75%, 80%, 85%, 90%, 95%, 97.5%, 99%, or 100%; a guide or RNA or sgRNA can be about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length; or guide or RNA or sgRNA can be less than about 75, 50, 45, 40, 35, 30, 25, 20, 15, 12, or fewer nucleotides in length; and advantageously tracr RNA is 30 or 50 nucleotides in length. However, an aspect of the invention is to reduce off-target interactions, e.g., reduce the guide interacting with a target sequence having low complementarity. Indeed, in the examples, it is shown that the invention involves mutations that result in the CRISPR-Cas system being able to distinguish between target and off-target sequences that have greater than 80% to about 95% complementarity, e.g., 83%-84% or 88-89% or 94-95% complementarity (for instance, distinguishing between a target having 18 nucleotides from an off-target of 18 nucleotides having 1, 2 or 3 mismatches). Accordingly, in the context of the present invention the degree of complementarity between a guide sequence and its corresponding target sequence is greater than 94.5% or 95% or 95.5% or 96% or 96.5% or 97% or 97.5% or 98% or 98.5% or 99% or 99.5% or 99.9%, or 100%. Off target is less than 100% or 99.9% or 99.5% or 99% or 99% or 98.5% or 98% or 97.5% or 97% or 96.5% or 96% or 95.5% or 95% or 94.5% or 94% or 93% or 92% or 91% or 90% or 89% or 88% or 87% or 86% or 85% or 84% or 83% or 82% or 81% or 80% complementarity between the sequence and the guide, with it advantageous that off target is 100% or 99.9% or 99.5% or 99% or 99% or 98.5% or 98% or 97.5% or 97% or 96.5% or 96% or 95.5% or 95% or 94.5% complementarity between the sequence and the guide.

### Guide Modifications

In certain embodiments, guides of the invention comprise non-naturally occurring nucleic acids and/or non-naturally occurring nucleotides and/or nucleotide analogs, and/or chemical modifications. Non-naturally occurring nucleic acids can include, for example, mixtures of naturally and non-naturally occurring nucleotides. Non-naturally occurring nucleotides and/or nucleotide analogs may be modified at the ribose, phosphate, and/or base moiety. In an embodiment of the invention, a guide nucleic acid comprises ribonucleotides and non-ribonucleotides. In one such embodiment, a guide comprises one or more ribonucleotides and one or more deoxyribonucleotides. In an embodiment of the invention, the guide comprises one or more non-naturally occurring nucleotide or nucleotide analog such as a nucleotide with phosphorothioate linkage, boranophosphate linkage, a locked nucleic acid (LNA) nucleotides comprising a methylene bridge between the 2' and 4' carbons of the ribose ring, or bridged nucleic acids (BNA). Other examples of modified nucleotides include 2'-O-methyl analogs, 2'-deoxy analogs, 2-thiouridine analogs, N6-methyladenosine analogs, or 2'-fluoro analogs. Further examples of modified bases include, but are not limited to, 2-aminopurine, 5-bromo-uridine, pseudouridine (Ψ), N¹-methylpseudouridine (me¹Ψ), 5-methoxyuridine(5moU), inosine, 7-methylguanosine. Examples of guide RNA chemical modifications include, without limitation, incorporation of 2'-O-methyl (M), 2'-O-methyl-3'-phosphorothioate (MS), phosphorothioate (PS), S-constrained ethyl(cEt), or 2'-O-methyl-3'-thioPACE (MSP) at one or more terminal nucleotides. Such chemically modified guides can comprise increased stability and increased activity as compared to unmodified guides, though on-target vs. off-target specificity is not predictable. (See, Hendel, 2015, Nat Biotechnol. 33(9):985-9, doi: 10.1038/nbt.3290, published online 29 June 2015; Ragdarm et al., 0215, PNAS, E7110-E7111; Allerson et al., J. Med. Chem. 2005, 48:901-904; Bramsen et al., Front. Genet., 2012, 3:154; Deng et al., PNAS, 2015, 112:11870-11875; Sharma et al., MedChemComm., 2014, 5:1454-1471; Hendel et al., Nat. Biotechnol. (2015) 33(9): 985-989; Li et al., Nature Biomedical Engineering, 2017, 1, 0066 DOI:10.1038/s41551-017-0066). In some embodiments, the 5' and/or 3' end of a guide RNA is modified by a variety of functional moieties including fluorescent dyes, polyethylene glycol, cholesterol, proteins, or detection tags. (See Kelly et al., 2016, J. Biotech. 233:74-83). In certain embodiments, a guide comprises ribonucleotides in a region that binds to a target DNA and one or more deoxyribonucleotides and/or nucleotide analogs in a region that binds to Cas9, Cpfl, or C2c1. In an embodiment of the invention, deoxyribonucleotides and/or nucleotide analogs are incorporated in engineered guide structures, such as, without limitation, 5' and/or 3' end, stem-loop regions, and the seed region. In certain embodiments, the modification is not in the 5'-handle of the stem-loop regions. Chemical modification in the 5'-handle of the stem-loop region of a guide may abolish its function (see Li, et al., Nature Biomedical Engineering, 2017, 1:0066). In certain embodiments, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or 75 nucleotides of a guide is chemically modified. In some embodiments, 3-5 nucleotides at either the 3' or the 5' end of a guide is chemically modified. In some embodiments, only minor modifications are introduced in the seed region, such as 2'-F modifications. In some embodiments, 2'-F modification is introduced at the 3' end of a guide. In certain embodiments, three to five nucleotides at the 5' and/or the 3' end of the guide are chemically modified with 2'-O-methyl (M), 2'-O-methyl-3'-phosphorothioate (MS), S-constrained ethyl(cEt), or 2'-O-methyl-3'-thioPACE (MSP). Such modification can enhance genome editing efficiency (see Hendel et al., Nat. Biotechnol. (2015) 33(9): 985-989). In certain embodiments, all of the phosphodiester bonds of a guide are substituted with phosphorothioates (PS) for enhancing levels of gene disruption. In certain embodiments, more than five nucleotides at the 5' and/or the 3' end of the guide are chemically modified with 2'-O-Me, 2'-F or S-constrained ethyl(cEt). Such chemically modified guide can mediate enhanced levels of gene disruption (see Ragdarm et al., 0215, PNAS, E7110-E7111). In an embodiment of the invention, a guide is modified to comprise a chemical moiety at its 3' and/or 5' end. Such moieties include, but are not limited to amine, azide, alkyne, thio, dibenzocyclooctyne (DBCO), or Rhodamine. In certain embodiment, the chemical moiety is conjugated to the guide by a linker, such as an alkyl chain. In certain embodiments, the chemical moiety of the modified guide can be used to attach the guide to another molecule, such as DNA, RNA, protein, or nanoparticles. Such chemically modified guide can be used to identify or enrich cells generically edited by a CRISPR system (see Lee et al., eLife, 2017, 6:e25312, DOI:10.7554).

In certain embodiments, the CRISPR system as provided herein can make use of a crRNA or analogous polynucleotide comprising a guide sequence, wherein the polynucleotide is an RNA, a DNA or a mixture of RNA and DNA, and/or wherein the polynucleotide comprises one or more nucleotide analogs. The sequence can comprise any structure, including but not limited to a structure of a native crRNA, such as a bulge, a hairpin or a stem loop structure. In certain embodiments, the polynucleotide comprising the guide sequence forms a duplex with a second polynucleotide sequence which can be an RNA or a DNA sequence.

In certain embodiments, use is made of chemically modified guide RNAs. Examples of guide RNA chemical modifications include, without limitation, incorporation of 2'-O-methyl (M), 2'-O-methyl 3'phosphorothioate (MS), or 2'-O-methyl 3'thioPACE (MSP) at one or more terminal nucleotides. Such chemically modified guide RNAs can comprise increased stability and increased activity as compared to unmodified guide RNAs, though on-target vs. off-target specificity is not predictable. (See, Hendel, 2015, Nat Biotechnol. 33(9):985-9, doi: 10.1038/nbt.3290, published online 29 June 2015). Chemically modified guide RNAs further include, without limitation, RNAs with phosphorothioate linkages and locked nucleic acid (LNA) nucleotides comprising a methylene bridge between the 2' and 4' carbons of the ribose ring.

In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. Preferably the guide sequence is 10 to 30 nucleotides long. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay. Similarly, cleavage of a target RNA may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

In some embodiments, the modification to the guide is a chemical modification, an insertion, a deletion or a split. In some embodiments, the chemical modification includes, but is not limited to, incorporation of 2'-O-methyl (M) analogs, 2'-deoxy analogs, 2-thiouridine analogs, N6-methyladenosine analogs, 2'-fluoro analogs, 2-aminopurine, 5-bromo-uridine, pseudouridine (Ψ), N¹-methylpseudouridine (me¹Ψ), 5-methoxyuridine(5moU), inosine, 7-methylguanosine, 2'-O-methyl-3'-phosphorothioate (MS), S-constrained ethyl(cEt), phosphorothioate (PS), or 2'-O-methyl-3'-thioPACE (MSP). In some embodiments, the guide comprises one or more of phosphorothioate modifications. In certain embodiments, at least 1, 2, 3, 4, 5, 6, 7, 8, 9, 10, 11, 12, 13, 14, 15, 16, 17, 18, 19, 20, or 25 nucleotides of the guide are chemically modified. In certain embodiments, one or more nucleotides in the seed region are chemically modified. In certain embodiments, one or more nucleotides in the 3'-terminus are chemically modified. In certain embodiments, none of the nucleotides in the 5'-handle is chemically modified. In some embodiments, the chemical modification in the seed region is a minor modification, such as incorporation of a 2'-fluoro analog. In a specific embodiment, one nucleotide of the seed region is replaced with a 2'-fluoro analog. In some embodiments, 5 or 10 nucleotides in the 3'-terminus are chemically modified. Such chemical modifications at the 3'-terminus of the Cpfl CrRNA improve gene cutting efficiency (see Li, et al., Nature Biomedical Engineering, 2017, 1:0066). In a specific embodiment, 5 nucleotides in the 3'-terminus are replaced with 2'-fluoro analogues. In a specific embodiment, 10 nucleotides in the 3'-terminus are replaced with 2'-fluoro analogues. In a specific embodiment, 5 nucleotides in the 3'-terminus are replaced with 2'- O-methyl (M) analogs.

In some embodiments, the loop of the 5'-handle of the guide is modified. In some embodiments, the loop of the 5'-handle of the guide is modified to have a deletion, an insertion, a split, or chemical modifications. In certain embodiments, the loop comprises 3, 4, or 5 nucleotides. In certain embodiments, the loop comprises the sequence of UCUU, UUUU, UAUU, or UGUU.

A guide sequence, and hence a nucleic acid-targeting guide RNA may be selected to target any target nucleic acid sequence. In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise RNA polynucleotides. The term "target RNA" refers to an RNA polynucleotide being or comprising the target sequence. In other words, the target RNA may be an RNA polynucleotide or a part of a RNA polynucleotide to which a part of the gRNA, i.e. the guide sequence, is designed to have complementarity and to which the effector function mediated by the complex comprising CRISPR effector protein and a gRNA is to be directed. In some embodiments, a target sequence is located in the nucleus or cytoplasm of a cell. The target sequence may be DNA. The target sequence may be any RNA sequence. In some embodiments, the target sequence may be a sequence within a RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nuclear RNA (snoRNA), double stranded RNA (dsRNA), non-coding RNA (ncRNA), long non-coding RNA (lncRNA), and small cytoplasmic RNA (scRNA). In some preferred embodiments, the target sequence may be a sequence within a RNA molecule selected from the group consisting of mRNA, pre-mRNA, and rRNA. In some preferred embodiments, the target sequence may be a sequence within a RNA molecule selected from the group consisting of ncRNA, and lncRNA. In some more preferred embodiments, the target sequence may be a sequence within an mRNA molecule or a pre-mRNA molecule.

In certain embodiments, the spacer length of the guide RNA is less than 28 nucleotides. In certain embodiments, the spacer length of the guide RNA is at least 18 nucleotides and less than 28 nucleotides. In certain embodiments, the spacer length of the guide RNA is between 19 and 28 nucleotides. In certain embodiments, the spacer length of the guide RNA is between 19 and 25 nucleotides. In certain embodiments, the spacer length of the guide RNA is 20 nucleotides. In certain embodiments, the spacer length of the guide RNA is 23 nucleotides. In certain embodiments, the spacer length of the guide RNA is 25 nucleotides.

In certain embodiments, modulations of cleavage efficiency can be exploited by introduction of mismatches, e.g. 1 or more mismatches, such as 1 or 2 mismatches between spacer sequence and target sequence, including the position of the mismatch along the spacer/target. The more central (i.e. not 3' or 5') for instance a double mismatch is, the more cleavage efficiency is affected. Accordingly, by choosing mismatch position along the spacer, cleavage efficiency can be modulated. By means of example, if less than 100 % cleavage of targets is desired (e.g. in a cell population), 1 or more, such as preferably 2 mismatches between spacer and target sequence may be introduced in the spacer sequences. The more central along the spacer of the mismatch position, the lower the cleavage percentage.

In certain example embodiments, the cleavage efficiency may be exploited to design single guides that can distinguish two or more targets that vary by a single nucleotide, such as a single nucleotide polymorphism (SNP), variation, or (point) mutation. The CRISPR effector may have reduced sensitivity to SNPs (or other single nucleotide variations) and continue to cleave SNP targets with a certain level of efficiency. Thus, for two targets, or a set of targets, a guide RNA may be designed with a nucleotide sequence that is complementary to one of the targets i.e. the on-target SNP. The guide RNA is further designed to have a synthetic mismatch. As used herein a "synthetic mismatch" refers to a non-naturally occurring mismatch that is introduced upstream or downstream of the naturally occurring SNP, such as at most 5 nucleotides upstream or downstream, for instance 4, 3, 2, or 1 nucleotide upstream or downstream, preferably at most 3 nucleotides upstream or downstream, more preferably at most 2 nucleotides upstream or downstream, most preferably 1 nucleotide upstream or downstream (i.e. adjacent the SNP). When the CRISPR effector binds to the on-target SNP, only a single mismatch will be formed with the synthetic mismatch and the CRISPR effector will continue to be activated and a detectable signal produced. When the guide RNA hybridizes to an off-target SNP, two mismatches will be formed, the mismatch from the SNP and the synthetic mismatch, and no detectable signal generated. Thus, the systems disclosed herein may be designed to distinguish SNPs within a population. For, example the systems may be used to distinguish pathogenic strains that differ by a single SNP or detect certain disease specific SNPs, such as but not limited to, disease associated SNPs, such as without limitation cancer associated SNPs.

In certain embodiments, the guide RNA is designed such that the SNP is located on position 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 of the spacer sequence (starting at the 5' end). In certain embodiments, the guide RNA is designed such that the SNP is located on position 1, 2, 3, 4, 5, 6, 7, 8, or 9 of the spacer sequence (starting at the 5' end). In certain embodiments, the guide RNA is designed such that the SNP is located on position 2, 3, 4, 5, 6, or 7of the spacer sequence (starting at the 5' end). In certain embodiments, the guide RNA is designed such that the SNP is located on position 3, 4, 5, or 6 of the spacer sequence (starting at the 5' end). In certain embodiments, the guide RNA is designed such that the SNP is located on position 3 of the spacer sequence (starting at the 5' end).

In certain embodiments, the guide RNA is designed such that the mismatch (e.g. the synthetic mismatch, i.e. an additional mutation besides a SNP) is located on position 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 of the spacer sequence (starting at the 5' end). In certain embodiments, the guide RNA is designed such that the mismatch is located on position 1, 2, 3, 4, 5, 6, 7, 8, or 9 of the spacer sequence (starting at the 5' end). In certain embodiments, the guide RNA is designed such that the mismatch is located on position 4, 5, 6, or 7of the spacer sequence (starting at the 5' end. In certain embodiments, the guide RNA is designed such that the mismatch is located on position 5 of the spacer sequence (starting at the 5' end).

In certain embodiments, the guide RNA is designed such that the mismatch is located 2 nucleotides upstream of the SNP (i.e. one intervening nucleotide).

In certain embodiments, the guide RNA is designed such that the mismatch is located 2 nucleotides downstream of the SNP (i.e. one intervening nucleotide).

In certain embodiments, the guide RNA is designed such that the mismatch is located on position 5 of the spacer sequence (starting at the 5' end) and the SNP is located on position 3 of the spacer sequence (starting at the 5' end).

The embodiments described herein comprehend inducing one or more nucleotide modifications in a eukaryotic cell (in vitro, i.e. in an isolated eukaryotic cell) as herein discussed comprising delivering to cell a vector as herein discussed. The mutation(s) can include the introduction, deletion, or substitution of one or more nucleotides at each target sequence of cell(s) via the guide(s) RNA(s). The mutations can include the introduction, deletion, or substitution of 1-75 nucleotides at each target sequence of said cell(s) via the guide(s) RNA(s). The mutations can include the introduction, deletion, or substitution of 1, 5, 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s). The mutations can include the introduction, deletion, or substitution of 5, 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s). The mutations include the introduction, deletion, or substitution of 10, 11, 12, 13, 14, 15, 16, 17, 18, 19, 20, 21, 22, 23, 24, 25, 26, 27, 28, 29, 30, 35, 40, 45, 50, or 75 nucleotides at each target sequence of said cell(s) via the guide(s) RNA(s). The mutations can include the introduction, deletion, or substitution of 20, 21, 22, 23, 24, 25, 26, 27, 28, 29, 30, 35, 40, 45, 50, or 75 nucleotides at each target sequence of said cell(s) via the guide(s) RNA(s). The mutations can include the introduction, deletion, or substitution of 40, 45, 50, 75, 100, 200, 300, 400 or 500 nucleotides at each target sequence of said cell(s) via the guide(s) RNA(s).

Typically, in the context of an endogenous CRISPR system, formation of a CRISPR complex (comprising a guide sequence hybridized to a target sequence and complexed with one or more Cas proteins) results in cleavage in or near (e.g. within 1, 2, 3, 4, 5, 6, 7, 8, 9, 10, 20, 50, or more base pairs from) the target sequence, but may depend on for instance secondary structure, in particular in the case of RNA targets.

In specific embodiments, methods of amplifying and/or detecting a target double-stranded nucleic acid are provided. Such methods may comprise combining a sample comprising the target double-stranded nucleic acid with an amplification reaction mixture. Such amplification reaction mixture may comprise an amplification CRISPR system. The amplification CRISPR system may comprise a first and second CRISPR/Cas complex, the first CRISPR/Cas complex comprising a first dead CRISPR/Cas enzyme and a first guide molecule that guides the first CRISPR/Cas complex to a first strand of the target nucleic acid, and the second CRISPR/Cas complex comprising a second dead CRISPR/Cas enzyme and a second guide molecule that guides the second CRISPR/Cas complex to a second strand of the target nucleic acid.

In specific embodiments, the first dead CRISPR/Cas enzyme and the second dead CRISPR/Cas enzyme may be the same. In alternative embodiments, the first dead CRISPR/Cas enzyme and the second dead CRISPR/Cas enzyme may be different.

In specific embodiments, the amplification reaction mixture may further comprise a helicase. Examples of helicases for use in present embodiments may also be found at the following web address: http://blocks.fhcrc.org (Get Blocks by Keyword: helicase). This site lists 49 Herpes helicases, 224 DnaB helicases, 250 UvrD-helicases and UvrD/Rep helicases, 276 DEAH_ATP-dependent helicases, 147 Papillom_E1 Papillomavirus helicase E1 protein, 608 Viral helicase1 Viral (superfamily 1) RNA helicases and 556 DEAD_ATP-dependent helicases. Examples of helicases that generally replicate in a 5' to 3' direction are T7 Gp4 helicase, DnaB helicase and Rho helicase, while examples of helicases that replicate in the 3'-5' direction include UvrD helicase, PcrA, Rep, NS3 RNA helicase of HCV.

In specific embodiments, the helicase may include, but is not necessarily limited to, UvrD helicase, CRISPR-Cas3 helicase, Rep helicase, PcrA helicase, RecD helicase, Dda helicase, papilloma virus E1 helicase, and gp4 helicase.

In specific embodiments, the amplification reaction mixture may further comprise primers, capable of hybridizing to a target nucleic acid strand. The term "hybridization" refers to binding of an oligonucleotide primer to a region of the single-stranded nucleic acid template under the conditions in which primer binds only specifically to its complementary sequence on one of the template strands, not other regions in the template. The specificity of hybridization may be influenced by the length of the oligonucleotide primer, the temperature in which the hybridization reaction is performed, the ionic strength, and the pH. The term "primer" refers to a single stranded nucleic acid capable of binding to a single stranded region on a target nucleic acid to facilitate polymerase dependent replication of the target nucleic acid strand. Nucleic acid(s) that are "complementary" or "complement(s)" are those that are capable of base-pairing according to the standard Watson-Crick, Hoogsteen or reverse Hoogsteen binding complementarity rules.

In specific embodiments, the amplification reaction mixture may further comprise a first and second primer. The first and second primer may comprise a portion that is complementary to the first strand of the target nucleic acid and a second primer comprising a portion that is complementary to the second strand of the target nucleic acid. The first and second primer may be referred to as a primer pair. In some embodiments, the first or second primer may comprise an RNA polymerase promoter.

In specific embodiments, the amplification reaction mixture may further comprise a polymerase. Polymerases are selected for HDA on the basis of processivity and strand displacement activity. Subsequent to melting and hybridization with a primer, the nucleic acid is subjected to a polymerization step. A DNA polymerase is selected if the nucleic acid to be amplified is DNA. When the initial target is RNA, a reverse transcriptase may first be used to copy the RNA target into a cDNA molecule and the cDNA is then further amplified in HDA by a selected DNA polymerase. The DNA polymerase acts on the target nucleic acid to extend the primers hybridized to the nucleic acid templates in the presence of four dNTPs to form primer extension products complementary to the nucleotide sequence on the nucleic acid template.

The DNA polymerase may be selected from a group of polymerases lacking 5' to 3' exonuclease activity and which additionally may optionally lack 3'-5' exonuclease activity. Examples of suitable DNA polymerases include an exonuclease-deficient Klenow fragment of *E. coli* DNA polymerase I (New England Biolabs, Inc. (Beverly, Mass.)), an exonuclease deficient T7 DNA polymerase (Sequenase; USB, (Cleveland, Ohio)), Klenow fragment of A. *coli* DNA polymerase I (New England Biolabs, Inc. (Beverly, Mass.)), Large fragment of Bst DNA polymerase (New England Biolabs, Inc. (Beverly, Mass.)), KlenTaq DNA polymerase (AB Peptides, (St Louis, Mo.)), T5 DNA polymerase (U.S. Pat. No. 5,716,819), and Pol III DNA polymerase (U.S. Pat. No. 6,555,349). DNA polymerases possessing strand-displacement activity, such as the exonuclease-deficient Klenow fragment of *E*. *coli* DNA polymerase I, Bst DNA polymerase Large fragment, and Sequenase, are preferred for Helicase-Dependent Amplification. T7 polymerase is a high fidelity polymerase having an error rate of 3.5×10⁵ which is significantly less than Taq polymerase (Keohavong and Thilly, Proc. Natl. Acad. Sci. USA 86, 9253-9257 (1989)). T7 polymerase is not thermostable however and therefore is not optimal for use in amplification systems that require thermocycling. In HDA, which can be conducted isothermally, T7 Sequenase is one of the preferred polymerases for amplification of DNA.

In specific embodiments, the polymerase may be selected from the group consisting of *Bst* 2.0 DNA polymerase, *Bst* 2.0 WarmStart DNA polymerase, *Bst* 3.0 DNA polymerase, full length *Bst* DNA polymerase, large fragment *Bst* DNA polymerase, large fragment *Bsu* DNA polymerase, phi29 DNA polymerase, T7 DNA polymerase, Gst polymerase, Taq polymerase, Klenow fragment of E. coli DNA polymerase I, KlenTaq NDA polymerase, Pol III DNA polymerase, T5 DNA polymerase, Sau LF DNA Polymerase, and Sequenase DNA polymerase.

In specific embodiments, the methods of amplifying and/or detecting a target double-stranded nucleic acid may further comprise amplifying the target nucleic acid by opening R-loops of the target nucleic acid using first and second CRISPR/Cas complexes as described herein. R-loops are formed when an RNA molecule is non-covalently associated with the double-stranded nucleic acid and which causes localized denaturation ("bubble" formation) within the double stranded nucleic acid (Thomas et al., 1976 Proc. Natl. Acad. Sci. USA 73, 2294). The method may further comprise unwinding the first and second strand of the target nucleic acid using the helicase. The method may further comprise annealing (binding) and extending the strands using the primer pair and the polymerase. Extension of the hybridized primer pairs occurs under conditions suitable for the polymerase. In some instances, hybridization and extension are carried out at the same temperature, while in other cases, hybridization occurs at a temperature optimal for the primers while extension occurs at a temperature optimal for the polymerase. However, as disclosed herein, primers and polymerases have been optimized for thermophilic and mesophilic applications. The length of the extension step can be varied depending on the size of the products being produced. Increasing the extension time will result in the production of longer fragments. In contrast, a shorter time of extension can be utilized to select for shorter products only. One skilled in the art will realize that the variation of the extension time can be utilized to select for different size products and that this variation can be used to improve amplification of products of the desired length.

In some specific embodiments, 500 ng of SSB and about 125 ng of helicase can be used per reaction to obtain ideal performance conditions. Additionally, between about 2.5 to about 7% PEG 20K, and about 0.5 to about 5 mM ATP can be used per reaction.

In specific embodiments, the methods of amplifying and/or detecting a target double-stranded nucleic acid may further comprise further amplifying the target nucleic acid by repeated opening, unwinding, annealing and extension under isothermal conditions. "Isothermal conditions" refers to conditions in which amplification which occurs at a single temperature, or conditions in which amplification occurs at a temperature with minimal fluctuations in temperature. In embodiments, the fluctuation in temperature is less than about 1 °C, 2 °C, 3 °C, 4 °C, 5 °C, 6 °C, 7 °C, 8 °C, 9 °C, or 10 °C. This does not include the single brief time period (less than 15 minutes) at the initiation of amplification which may be conducted at the same temperature as the amplification procedure or at a higher temperature.

### Helicases

The term "helicase" refers here to any enzyme capable of unwinding a double stranded nucleic acid enzymatically. For example, helicases are enzymes that are found in all organisms and in all processes that involve nucleic acid such as replication, recombination, repair, transcription, translation and RNA splicing. (Kornberg and Baker, DNA Replication, W. H. Freeman and Company (2nd ed. (1992)), especially chapter 11). Any helicase that translocates along DNA or RNA in a 5' to 3' direction or in the opposite 3' to 5' direction may be used in present embodiments of the invention. This includes helicases obtained from prokaryotes, viruses, archaea, and eukaryotes or recombinant forms of naturally occurring enzymes as well as analogues or derivatives having the specified activity. Examples of naturally occurring DNA helicases, described by Kornberg and Baker in chapter 11 of their book, DNA Replication, W. H. Freeman and Company (2nd ed. (1992)), include *E*. *coli* helicase I, II, III, & IV, Rep, DnaB, PriA, PcrA, T4 Gp41helicase, T4 Dda helicase, T7 Gp4 helicases, SV40 Large T antigen, yeast RAD. Additional helicases that may be useful in HDA include RecQ helicase (Harmon and Kowalczykowski, J. Biol. Chem. 276:232-243 (2001)), thermostable UvrD helicases from *T. tengcongensis* (disclosed in this invention, Example XII) and *T. thermophilus* (Collins and McCarthy, Extremophiles. 7:35-41. (2003)), thermostable DnaB helicase from *T. aquaticus* (Kaplan and Steitz, J. Biol. Chem. 274:6889-6897 (1999)), and MCM helicase from archaeal and eukaryotic organisms ((Grainge et al., Nucleic Acids Res. 31:4888-4898 (2003)). Further examples of helicases include DNA helicases PcrA from Thermoanaerobacter ethanolicus (e.g., WP_003870487.1), PcrA from Bacillus sp. FJAT-27231 (e.g., WP_034654680.1), PcrA from Bacillus megaterium (e.g., WP_034654680.1), PcrA from Bacillus simplex (e.g., WP_095390358.1), and PcrA from Paeniclostridium sordellii (e.g., WP_055343022.1).

A traditional definition of a helicase is an enzyme that catalyzes the reaction of separating/unzipping/unwinding the helical structure of nucleic acid duplexes (DNA, RNA or hybrids) into single-stranded components, using nucleoside triphosphate (NTP) hydrolysis as the energy source (such as ATP). However, it should be noted that not all helicases fit this definition anymore. A more general definition is that they are motor proteins that move along the single-stranded or double stranded nucleic acids (usually in a certain direction, 3' to 5' or 5 to 3, or both), i.e. translocases, that can or cannot unwind the duplexed nucleic acid encountered. In addition, some helicases simply bind and "melt" the duplexed nucleic acid structure without an apparent translocase activity.

Helicases exist in all living organisms and function in all aspects of nucleic acid metabolism. Helicases are classified based on the amino acid sequences, directionality, oligomerization state and nucleic-acid type and structure preferences. The most common classification method was developed based on the presence of certain amino acid sequences, called motifs. According to this classification helicases are divided into 6 super families: SF1, SF2, SF3, SF4, SF5 and SF6. SF1 and SF2 helicases do not form a ring structure around the nucleic acid, whereas SF3 to SF6 do. Superfamily classification is not dependent on the classical taxonomy.

DNA helicases are responsible for catalyzing the unwinding of double-stranded DNA (dsDNA) molecules to their respective single-stranded nucleic acid (ssDNA) forms. Although structural and biochemical studies have shown how various helicases can translocate on ssDNA directionally, consuming one ATP per nucleotide, the mechanism of nucleic acid unwinding and how the unwinding activity is regulated remains unclear and controversial (T. M. Lohman, E. J. Tomko, C. G. Wu, "Non-hexameric DNA helicases and translocases: mechanisms and regulation," Nat Rev Mol Cell Biol 9:391-401 (2008)). Since helicases can potentially unwind all nucleic acids encountered, understanding how their unwinding activities are regulated can lead to harnessing helicase functions for biotechnology applications.

The term "HDA" refers to Helicase Dependent Amplification, which is an *in vitro* method for amplifying nucleic acids by using a helicase preparation for unwinding a double stranded nucleic acid to generate templates for primer hybridization and subsequent primer-extension. This process utilizes two oligonucleotide primers, each hybridizing to the 3'-end of either the sense strand containing the target sequence or the anti-sense strand containing the reverse-complementary target sequence. The HDA reaction is a general method for helicase-dependent nucleic acid amplification.

The invention comprises use of any suitable helicase known in the art. These include, but are not necessarily limited to, UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase.

In certain embodiments, helicase selection is based on amplification conditions. For example, mesophilic isothermal amplification conditions may favor a helicase of UvrD type from mesophilic bacterial species. In some examples, the helicase comprises substitutions to increase activity. In one particularly preferred embodiment, the helicase may have two Alanine substitutions for Aspartic Acid. In helicase Tte-UvrD, substitutions D409A and D410A increase helicase processivity and activity.

In some instances, the helicase is a UvrD-like helicase, wild-type, or a mutant with super helicase mutations. Super helicase mutations are typically DD to AA substitutions that increase activity of the helicase. In some instances, the helicase is an E. coli UvrD helicase with. Substitutions at D403A and D404A. In some embodiments, the helicase is a pcrA (UvrD)-type helicase, for example, Tte-UvrD helicase with mutations D409A/D410A. In some instances, the helicase is an ATP dependent helicase, and an ATP co-factor can be used in conjunction with the helicase. In some embodiments, the helicase is a DNA helicase pcrA helicase, for example, pcrA helicase from Thermoanaerobacter ethanolicus (e.g., WP_003870487.1) with mutations D403A/D404A, pcrA helicase from Bacillus sp. FJAT-27231 (e.g., WP_034654680.1) with mutations D407A/D408A, pcrA helicase from Bacillus megaterium (e.g., WP_034654680.1) with mutations D415A/D416A, pcrA helicase from Bacillus simplex (e.g., WP_095390358.1) with mutations D407A/D408A, or pcrA helicase from Paeniclostridium sordellii (e.g., WP_055343022.1) with mutations D402A/D403A.

In some embodiments, the helicase is a E. Coli UvrD helicase, optionally with two or more substitutions of D to A, for example at D403 and D404. In some embodiments, the helicase is a pcrA (UvrD)-type helicase comprising one or more mutations corresponding to D409A and D410A. In some embodiments, the helicase is Tte-UvrD D409A/D410A.

### Amplification

In certain example embodiments, target single-stranded or double-stranded nucleic acids may be amplified using an isothermal amplification technique. In certain example embodiments, the isothermal amplification may be nucleic-acid sequenced-based amplification (NASBA), recombinase polymerase amplification (RPA), loop-mediated isothermal amplification (LAMP), strand displacement amplification (SDA), helicase-dependent amplification (HDA), or nicking enzyme amplification reaction (NEAR).

"Isothermal amplification" refers to amplification which occurs at a single temperature. This does not include the single brief time period (less than 15 minutes) at the initiation of amplification which may be conducted at the same temperature as the amplification procedure or at a higher temperature.

Mesophilic amplification and thermophilic amplification are two exemplary isothermal applications. Mesophilic amplification is typically conducted at temperatures around 20 to 40 °C, or around 35 °C +/- 5 °C, with thermophilic amplification typically conducted around 50 to 70 °C, or around 55 °C to 60°C.

HDA relies on one or more helicases to separate (melt, or unwind) two strands of a nucleic acid duplex. Any helicase known in the art may be used. HDA further utilizes a DNA or RNA polymerase to extend primers which are hybridized to single stranded nucleotide sequences to form complementary primer extension products. This process repeats itself so that exponential amplification can be achieved at a single temperature. Some advantages of the present embodiments over amplification procedures in the prior art include the ability to isothermally amplify long target sequences of DNA and RNA (longer than about 200 nucleotides more particularly, greater than about 500 nucleotides, more particularly greater than about 1000 nucleotides, more particularly, greater than 2000 nucleotides, more particularly up to about 50,000 nucleotides, more particularly as much as about 100,000 nucleotides) and the ability to amplify target sequences at one temperature from the beginning to the end.

Accordingly, in certain example embodiments the systems disclosed herein may include amplification reagents. Different components or reagents useful for amplification of nucleic acids are described herein. For example, an amplification reagent as described herein may include a buffer, such as a Tris buffer. A Tris buffer may be used at any concentration appropriate for the desired application or use, for example including, but not limited to, a concentration of 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 25 mM, 50 mM, 75 mM, 1 M, or the like. One of skill in the art will be able to determine an appropriate concentration of a buffer such as Tris for use with the present invention.

A salt, such as magnesium chloride (MgCl₂), potassium chloride (KCl), or sodium chloride (NaCl), may be included in an amplification reaction, such as PCR, in order to improve the amplification of nucleic acid fragments. Although the salt concentration will depend on the particular reaction and application, in some embodiments, nucleic acid fragments of a particular size may produce optimum results at particular salt concentrations. Larger products may require altered salt concentrations, typically lower salt, in order to produce desired results, while amplification of smaller products may produce better results at higher salt concentrations. One of skill in the art will understand that the presence and/or concentration of a salt, along with alteration of salt concentrations, may alter the stringency of a biological or chemical reaction, and therefore any salt may be used that provides the appropriate conditions for a reaction of the present invention and as described herein.

Other components of a biological or chemical reaction may include a cell lysis component in order to break open or lyse a cell for analysis of the materials therein. A cell lysis component may include, but is not limited to, a detergent, a salt as described above, such as NaCl, KCl, ammonium sulfate [(NH₄)₂SO₄₁, or others. Detergents that may be appropriate for the invention may include Triton X-100, sodium dodecyl sulfate (SDS), CHAPS (3-[(3-cholamidopropyl)dimethylammonio]-1-propanesulfonate), ethyl trimethyl ammonium bromide, nonyl phenoxypolyethoxylethanol (NP-40). Concentrations of detergents may depend on the particular application, and may be specific to the reaction in some cases. Amplification reactions may include dNTPs and nucleic acid primers used at any concentration appropriate for the invention, such as including, but not limited to, a concentration of 100 nM, 150 nM, 200 nM, 250 nM, 300 nM, 350 nM, 400 nM, 450 nM, 500 nM, 550 nM, 600 nM, 650 nM, 700 nM, 750 nM, 800 nM, 850 nM, 900 nM, 950 nM, 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM, 100 mM, 150 mM, 200 mM, 250 mM, 300 mM, 350 mM, 400 mM, 450 mM, 500 mM, or the like. Likewise, a polymerase useful in accordance with the invention may be any specific or general polymerase known in the art and useful or the invention, including Taq polymerase, Q5 polymerase, or the like.

In some embodiments, amplification reagents as described herein may be appropriate for use in hot-start amplification. Hot start amplification may be beneficial in some embodiments to reduce or eliminate dimerization of adaptor molecules or oligos, or to otherwise prevent unwanted amplification products or artifacts and obtain optimum amplification of the desired product. Many components described herein for use in amplification may also be used in hot-start amplification. In some embodiments, reagents or components appropriate for use with hot-start amplification may be used in place of one or more of the composition components as appropriate. For example, a polymerase or other reagent may be used that exhibits a desired activity at a particular temperature or other reaction condition. In some embodiments, reagents may be used that are designed or optimized for use in hot-start amplification, for example, a polymerase may be activated after transposition or after reaching a particular temperature. Such polymerases may be antibody-based or aptamerbased. Polymerases as described herein are known in the art. Examples of such reagents may include, but are not limited to, hot-start polymerases, hot-start dNTPs, and photo-caged dNTPs. Such reagents are known and available in the art. One of skill in the art will be able to determine the optimum temperatures as appropriate for individual reagents.

The amplification can be isothermal and selected for temperature. In one embodiment, the amplification proceeds rapidly at 37 degrees. In other embodiments, the temperature of the isothermal amplification may be chosen by selecting a polymerase (e.g. Bsu, Bst, Phi29, klenow fragment etc.) operable at a different temperature.

Amplification of nucleic acids may be performed using specific thermal cycle machinery or equipment, and may be performed in single reactions or in bulk, such that any desired number of reactions may be performed simultaneously. In some embodiments, amplification may be performed using microfluidic or robotic devices, or may be performed using manual alteration in temperatures to achieve the desired amplification. In some embodiments, optimization may be performed to obtain the optimum reactions conditions for the particular application or materials. One of skill in the art will understand and be able to optimize reaction conditions to obtain sufficient amplification, however, the amplification as disclosed herein has the advantage of being conducted under isothermal conditions, and thus does not require thermal cycling to achieve amplification.

In certain embodiments, the invention provides methods of amplifying the target nucleic acid by opening R-loops of the target nucleic acid using the first and second CRISPR/Cas complexes, unwinding the first and the second strand of the target nucleic acid using the helicase, annealing and extending the strands using the primer pair and the polymerase; and further amplifying the target nucleic acid by repeated opening, unwinding, annealing and extension under isothermal conditions.

In some embodiments, amplification of the target nucleic acid is performed at about 37°C-65°C. In some embodiments, amplification of the target nucleic acid is performed at about 50°C-59°C. In some embodiments, amplification of the target nucleic acid is performed at about 60°C-72°C. In some embodiments, amplification of the target nucleic acid is performed at about 37°C. In some embodiments, amplification of the target nucleic acid is performed at room temperature.

In some embodiments, the methods described herein are performed under mesophilic isothermal conditions. In some embodiments, the polymerase is a mesophilic polymerase. In some embodiments, the mesophilic polymerase is Sau LF Polymerase.

In some embodiments, the target nucleic acid sequence is about 20-30, about 30-40, about 40-50, or about 50-100 nucleotides in length. In some embodiments, the target nucleic acid sequence is about 100-200, about 100-500, or about 100-1000 nucleotides in length. In some embodiments, the target nucleic acid sequence is about 1000-2000, about 2000-3000, about 3000-4000, or about 4000-5000 nucleotides in length.

In some embodiments, the first or the second primer comprises an RNA polymerase promoter.

In some embodiments, the method described herein may comprise one or more additives selected from a crowding agent, a co-factor, single-strand binding protein, and/or assisting proteins. In specific embodiments, the crowding agent is polyethylene glycol. In some embodiments, the polyethylene glycol (PEG) is between about 500 to 30,000 Da in size. In some embodiments, the PEG is 20,000 Da in size.

In some embodiments, the co-factor is ATP.

In some embodiments, the single-strand binding protein is selected from T4 gp32 or thermophilic SSB (ET-SSB).

In some embodiments, the assisting protein is dCpf1 and/or UvsX Recombinase.

In some embodiments, the amplified target nucleic acid is detected by a CRISPR Cas-13 based system comprising LwaCas13 enzyme.

In some embodiments, the target nucleic acid is detected at attomolar sensitivity. In some embodiments, the target nucleic acid is detected at femtomolar sensitivity.

In instances where the target nucleic acid is RNA, the RNA may be reverse transcribed into cDNA prior to amplification. Amplification may then be done by any suitable amplification method known in the art, such as any described herein. One exemplary amplification method may include helicase dependent amplification (HDA). Another exemplary amplification method may include recombinase polymerase amplification (RPA).

In some embodiments, the reaction is performed in less than about 2 hours, less than about 90 minutes, less than about 60 minutes, less than about 30 minutes or less than about 15 minutes.

In some embodiments, the target nucleic acid sequence may be about 20-30, about 30-40, about 40-50, or about 50-100 nucleotides in length. In some embodiments, the target nucleic acid sequence may be about 100-200, about 100-500, or about 100-1000 nucleotides in length. In some embodiments, the target nucleic acid sequence may be about 1000-2000, about 2000-3000, about 3000-4000, or about 4000-5000 nucleotides in length.

In specific embodiments, the target nucleic acid may include, but is not necessarily limited to, genomic DNA, mitochondrial DNA, viral DNA, plasmid DNA, or synthetic double-stranded DNA.

In specific embodiments, the target nucleic acid may include single-stranded viral DNA, messenger RNA, ribosomal RNA, transfer RNA, microRNA, short interfering RNA, small nuclear RNA, synthetic RNA, synthetic single-stranded DNA, long non-coding RNA, pre-microRNA, viral dsRNA, and non-viral dsRNA.

In certain embodiments, methods described herein involve combining a sample comprising the target double-stranded nucleic acid with an amplification reaction mixture.

As described herein, a sample for use with the invention may be a biological or environmental sample, such as a food sample (fresh fruits or vegetables, meats), a beverage sample, a paper surface, a fabric surface, a metal surface, a wood surface, a plastic surface, a soil sample, a freshwater sample, a wastewater sample, a saline water sample, exposure to atmospheric air or other gas sample, or a combination thereof. For example, household/commercial/industrial surfaces made of any materials including, but not limited to, metal, wood, plastic, rubber, or the like, may be swabbed and tested for contaminants. Soil samples may be tested for the presence of pathogenic bacteria or parasites, or other microbes, both for environmental purposes and/or for human, animal, or plant disease testing. Water samples such as freshwater samples, wastewater samples, or saline water samples can be evaluated for cleanliness and safety, and/or potability, to detect the presence of, for example, *Cryptosporidium parvum, Giardia lamblia,* or other microbial contamination. In further embodiments, a biological sample may be obtained from a source including, but not limited to, a tissue sample, saliva, blood, plasma, sera, stool, urine, sputum, mucous, lymph, synovial fluid, cerebrospinal fluid, ascites, pleural effusion, seroma, pus, or swab of skin or a mucosal membrane surface. In some particular embodiments, an environmental sample or biological samples may be crude samples and/or the one or more target molecules may not be purified or amplified from the sample prior to application of the method. Identification of microbes may be useful and/or needed for any number of applications, and thus any type of sample from any source deemed appropriate by one of skill in the art may be used in accordance with the invention.

In some embodiments, the biological sample may include, but is not necessarily limited to, blood, plasma, serum, urine, stool, sputum, mucous, lymph fluid, synovial fluid, bile, ascites, pleural effusion, seroma, saliva, cerebrospinal fluid, aqueous or vitreous humor, or any bodily secretion, a transudate, an exudate, or fluid obtained from a joint, or a swab of skin or mucosal membrane surface.

In specific embodiments, the sample may be blood, plasma or serum obtained from a human patient.

In some embodiments, the sample may be a plant sample. In some embodiments, the sample may be a crude sample. In some embodiments, the sample may be a purified sample.

In some embodiments, amplification of the target nucleic acid may be performed at about 37°C-65°C. In some embodiments, amplification of the target nucleic acid may be performed at about 50°C-59°C. In more specific embodiments, amplification of the target nucleic acid may be performed at about 60°C-72°C. In more specific embodiments, amplification of the target nucleic acid may be performed at about 37°C. The amplification of the target nucleic acid may also be performed at room temperature. Room temperature may vary from 20°C-25°C. In some embodiments, amplification of the target nucleic acid may be performed at a constant temperature.

In some embodiments, the method further comprises an amplifier primer that comprises a portion contained in the first primer of the primer pair, wherein the first primer of the primer pair mediates a first step of amplifying, and the amplifier primer together with the second primer of the primer pair mediates the subsequent steps of amplifying.

The amplifier primer and the first primer of the primer pair may each comprise a T7 promoter sequence.

The amplifier primer may comprise a length of about 23 to about 35 nucleotides.

In another aspect, the invention provides a method for amplifying and/or detecting a target single-stranded nucleic acid, comprising converting the single-stranded nucleic acid in a sample to a target double-stranded nucleic acid; and performing the steps of combining a sample comprising the target double-stranded nucleic acid with an amplification reaction mixture as described herein, amplifying the target nucleic acid by opening R-loops of the target nucleic acid using the first and second CRISPR/Cas complexes, unwinding the first and the second strand of the target nucleic acid using the helicase, annealing and extending the strands using the primer pair and the polymerase; further amplifying the target nucleic acid by repeated opening, unwinding, annealing and extension under isothermal conditions; and optionally detecting the amplified target nucleic acid, as described elsewhere herein.

In some embodiments, the target single-stranded nucleic acid is an RNA molecule.

In some embodiments, the RNA molecule is converted to the double-stranded nucleic acid by a reverse-transcription and amplification step.

In some embodiments, the target single-stranded nucleic acid is selected from the group consisting of single-stranded viral DNA, viral RNA, messenger RNA, ribosomal RNA, transfer RNA, microRNA, short interfering RNA, small nuclear RNA, synthetic RNA, synthetic single-stranded DNA, long non-coding RNA, pre-microRNA, viral dsRNA, and non-viral dsRNA.

### Detection

In some embodiments, the method may further comprise detecting the amplified nucleic acid by a method including, but not necessarily limited to, gel electrophoresis, intercalating dye detection, PCR, real-time PCR, fluorescence, Fluorescence Resonance Energy Transfer (FRET), mass spectrometry, lateral flow assay, colorimetric assays, and a CRISPR-based detection system. Colorimetric assays may include any known in the art, such as, but not necessarily limited to, horseradish peroxidase (HRP) assay, alkaline phosphatase (ALP) assay, or gold nanoparticle based assays.

In specific embodiments, the amplified nucleic acid may be detected by a CRISPR Cas13-based system. In specific embodiments, the amplified nucleic acid may be detected by a CRISPR Cas12-based system (see Chen et al. Science 360:436-439 (2018) and Gootenberg et al. Science 360:439-444 (2018)). In specific embodiments, the amplified nucleic acid may be detected by a combination of a CRISPR Cas13-based and a CRISPR Cas12-based system.

Detection of nucleic acids including single nucleotide variants, detection based on rRNA sequences, screening for drug resistance, monitoring microbe outbreaks, genetic perturbations, and screening of environmental samples, can be as described, for example, in PCT/US18/054472 filed October 22, 2018 at [0183] - [0327]. Reference is made to WO 2017/219027, WO2018/107129, US20180298445, US 2018-0274017, US 2018-0305773, WO 2018/170340, U.S. Application 15/922,837, filed March 15, 2018 entitled "Devices for CRISPR Effector System Based Diagnostics", PCT/US 18/50091, filed September 7, 2018 "Multi-Effector CRISPR Based Diagnostic Systems", PCT/US 18/66940 filed December 20, 2018 entitled "CRISPR Effector System Based Multiplex Diagnostics", PCT/US 18/054472 filed October 4, 2018 entitled "CRISPR Effector System Based Diagnostic", U.S. Provisional 62/740,728 filed October 3, 2018 entitled "CRISPR Effector System Based Diagnostics for Hemorrhagic Fever Detection", U.S. Provisional 62/690,278 filed June 26, 2018 and U.S. Provisional 62/767,059 filed November 14, 2018 both entitled "CRISPR Double Nickase Based Amplification, Compositions, Systems and Methods", U.S. Provisional 62/690,160 filed June 26, 2018 and 62,767,077 filed November 14, 2018, both entitled "CRISPR/CAS and Transposase Based Amplification Compositions, Systems, And Methods", U.S. Provisional 62/690,257 filed June 26, 2018 and 62/767,052 filed November 14, 2018 both entitled "CRISPR Effector System Based Amplification Methods, Systems, And Diagnostics", US Provisional 62/767,076 filed November 14, 2018 entitled "Multiplexing Highly Evolving Viral Variants With SHERLOCK" and 62/767,070 filed November 14, 2018 entitled "Droplet SHERLOCK." Reference is further made to WO2017/127807, WO2017/184786, WO 2017/184768, WO 2017/189308, WO 2018/035388, WO 2018/170333, WO 2018/191388, WO 2018/213708, WO 2019/005866, PCT/US18/67328 filed December 21, 2018 entitled "Novel CRISPR Enzymes and Systems", PCT/US 18/67225 filed December 21, 2018 entitled "Novel CRISPR Enzymes and Systems" and PCT/LTS18/67307 filed December 21, 2018 entitled "Novel CRISPR Enzymes and Systems", US 62/712,809 filed July 31, 2018 entitled "Novel CRISPR Enzymes and Systems", U.S. 62/744,080 filed October 10, 2018 entitled "Novel Cas12b Enzymes and Systems" and U.S. 62/751,196 filed October 26 2018 entitled "Novel Cas12b Enzymes and Systems", U.S. 715,640 filed August 7, 2-18 entitled "Novel CRISPR Enzymes and Systems", WO 2016/205711, U.S. 9,790,490, WO 2016/205749, WO 2016/205764, WO 2017/070605, WO 2017/106657, and WO 2016/149661, WO2018/035387, WO2018/194963, Cox DBT, et al., RNA editing with CRISPR-Cas13, Science. 2017 Nov 24;358(6366):1019-1027; Gootenberg JS, et al., Multiplexed and portable nucleic acid detection platform with Cas13, Cas12a, and Csm6., Science. 2018 Apr 27;360(6387):439-444; Gootenberg JS, et al., Nucleic acid detection with CRISPR-Cas13a/C2c2., Science. 2017 Apr 28;356(6336):438-442; Abudayyeh OO, et al., RNA targeting with CRISPR-Cas13, Nature. 2017 Oct 12;550(7675):280-284; Smargon AA, et al., Cas13b Is a Type VI-B CRISPR-Associated RNA-Guided RNase Differentially Regulated by Accessory Proteins Csx27 and Csx28. Mol Cell. 2017 Feb 16;65(4):618-630.e7; Abudayyeh OO, et al., C2c2 is a single-component programmable RNA-guided RNA-targeting CRISPR effector, Science. 2016 Aug 5;353(6299):aaf5573; Yang L, et al., Engineering and optimising deaminase fusions for genome editing. Nat Commun. 2016 Nov 2;7:13330, Myrvhold et al., Field deployable viral diagnostics using CRISPR-Cas13, Science 2018 360, 444-448, Shmakov et al. "Diversity and evolution of class 2 CRISPR-Cas systems," Nat Rev Microbiol. 2017 15(3):169-182.

In some specific embodiments, RNA targeting effectors can be utilized to provide a robust CRISPR-based detection. Embodiments disclosed herein can detect both DNA and RNA with comparable levels of sensitivity and can be used in conjunction with the HDA methods and system disclosed. For ease of reference, the detection embodiments disclosed herein may also be referred to as SHERLOCK (Specific High-sensitivity Enzymatic Reporter unLOCKing), which, in some embodiments, is performed subsequent to the HDA methods disclosed herein, including under mesophilic and thermophilic isothermal conditions.

In some embodiments, one or more elements of a nucleic acid-targeting detection system is derived from a particular organism comprising an endogenous CRISPR RNA-targeting system. In certain example embodiments, the effector protein CRISPR RNA-targeting detection system comprises at least one HEPN domain, including but not limited to the HEPN domains described herein, HEPN domains known in the art, and domains recognized to be HEPN domains by comparison to consensus sequence motifs. Several such domains are provided herein. In one non-limiting example, a consensus sequence can be derived from the sequences of C2c2 or Cas13b orthologs provided herein. In certain example embodiments, the effector protein comprises a single HEPN domain. In certain other example embodiments, the effector protein comprises two HEPN domains.

In one example embodiment, the effector protein comprises one or more HEPN domains comprising a RxxxxH motif sequence. The RxxxxH motif sequence can be, without limitation, from a HEPN domain described herein or a HEPN domain known in the art. RxxxxH motif sequences further include motif sequences created by combining portions of two or more HEPN domains. As noted, consensus sequences can be derived from the sequences of the orthologs disclosed in PCT/US2017/038154 entitled "Novel Type VI CRISPR Orthologs and Systems," at, for example, pages 256-264 and 285-336, U.S. Provisional Patent Application 62/432,240 entitled "Novel CRISPR Enzymes and Systems," U.S. Provisional Patent Application 62/471,710 entitled "Novel Type VI CRISPR Orthologs and Systems" filed on March 15, 2017, and U.S. Provisional Patent Application 62/484,786 entitled "Novel Type VI CRISPR Orthologs and Systems," filed on April 12, 2017.

In an embodiment of the invention, a HEPN domain comprises at least one RxxxxH motif comprising the sequence of R{N/H/K}X1X2X3H (SEQ ID NO: 15). In an embodiment of the invention, a HEPN domain comprises a RxxxxH motif comprising the sequence of R{N/H}X1X2X3H (SEQ ID NO: 16). In an embodiment of the invention, a HEPN domain comprises the sequence of R{N/K}X1X2X3H (SEQ ID NO: 17). In certain embodiments, X1 is R, S, D, E, Q, N, G, Y, or H. In certain embodiments, X2 is I, S, T, V, or L. In certain embodiments, X3 is L, F, N, Y, V, I, S, D, E, or A.

Additional effectors for use according to the invention can be identified by their proximity to cas1 genes, for example, though not limited to, within the region 20 kb from the start of the cas1 gene and 20 kb from the end of the cas1 gene. In certain embodiments, the effector protein comprises at least one HEPN domain and at least 500 amino acids, and wherein the C2c2 effector protein is naturally present in a prokaryotic genome within 20 kb upstream or downstream of a Cas gene or a CRISPR array. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In certain example embodiments, the C2c2 effector protein is naturally present in a prokaryotic genome within 20kb upstream or downstream of a Cas 1 gene. The terms "orthologue" (also referred to as "ortholog" herein) and "homologue" (also referred to as "homolog" herein) are well known in the art. By means of further guidance, a "homologue" of a protein as used herein is a protein of the same species which performs the same or a similar function as the protein it is a homologue of. Homologous proteins may but need not be structurally related, or are only partially structurally related. An "orthologue" of a protein as used herein is a protein of a different species which performs the same or a similar function as the protein it is an orthologue of. Orthologous proteins may but need not be structurally related, or are only partially structurally related.

In particular embodiments, the Type VI RNA-targeting Cas enzyme is C2c2. In other example embodiments, the Type VI RNA-targeting Cas enzyme is Cas 13b. In particular embodiments, the homologue or orthologue of a Type VI protein such as C2c2 as referred to herein has a sequence homology or identity of at least 30%, or at least 40%, or at least 50%, or at least 60%, or at least 70%, or at least 80%, more preferably at least 85%, even more preferably at least 90%, such as for instance at least 95% with a Type VI protein such as C2c2 (e.g., based on the wild-type sequence of any of *Leptotrichia shahii* C2c2, *Lachnospiraceae bacterium MA2020* C2c2, *Lachnospiraceae bacterium NK4A179* C2c2, *Clostridium aminophilum (DSM 10710)* C2c2, *Carnobacterium gallinarum (DSM 4847)* C2c2, *Paludibacter propionicigenes (WB4)* C2c2, *Listeria weihenstephanensis (FSL R9-0317)* C2c2, *Listeriaceae bacterium (FSL M6-0635)* C2c2, *Listeria newyorkensis (FSL M6-0635)* C2c2, *Leptotrichia wadei (F0279)* C2c2, *Rhodobacter capsulatus (SB 1003)* C2c2, *Rhodobacter capsulatus (R121)* C2c2, *Rhodobacter capsulatus (DE442)* C2c2, *Leptotrichia wadei* (Lw2) C2c2, or *Listeria seeligeri* C2c2). In further embodiments, the homologue or orthologue of a Type VI protein such as C2c2 as referred to herein has a sequence identity of at least 30%, or at least 40%, or at least 50%, or at least 60%, or at least 70%, or at least 80%, more preferably at least 85%, even more preferably at least 90%, such as for instance at least 95% with the wild type C2c2 (e.g., based on the wild-type sequence of any of *Leptotrichia shahii* C2c2, *Lachnospiraceae bacterium MA2020* C2c2, *Lachnospiraceae bacterium NK4A179* C2c2, *Clostridium aminophilum (DSM 10710)* C2c2, *Carnobacterium gallinarum (DSM 4847)* C2c2, *Paludibacter propionicigenes (WB4)* C2c2, *Listeria weihenstephanensis (FSL R9-0317)* C2c2, *Listeriaceae bacterium (FSL M6-0635)* C2c2, *Listeria newyorkensis (FSL M6-0635)* C2c2, *Leptotrichia wadei (F0279)* C2c2, *Rhodobacter capsulatus (SB 1003)* C2c2, *Rhodobacter capsulatus (R121)* C2c2, *Rhodobacter capsulatus (DE442)* C2c2, *Leptotrichia wadei (Lw2)* C2c2, or *Listeria seeligeri* C2c2).

In certain other example embodiments, the CRISPR system the effector protein is a C2c2 nuclease. The activity of C2c2 may depend on the presence of two HEPN domains. These have been shown to be RNase domains, *i.e.* nuclease (in particular an endonuclease) cutting RNA. C2c2 HEPN may also target DNA, or potentially DNA and/or RNA. On the basis that the HEPN domains of C2c2 are at least capable of binding to and, in their wild-type form, cutting RNA, then it is preferred that the C2c2 effector protein has RNase function. Regarding C2c2 CRISPR systems, reference is made to U.S. Provisional 62/351,662 filed on June 17, 2016 and U.S. Provisional 62/376,377 filed on August 17, 2016. Reference is also made to U.S. Provisional 62/351,803 filed on June 17, 2016. Reference is also made to U.S. Provisional entitled "Novel Crispr Enzymes and Systems" filed December 8, 2016 bearing Broad Institute No. 10035.PA4 and Attorney Docket No. 47627.03.2133. Reference is further made to East-Seletsky et al. "Two distinct RNase activities of CRISPR-C2c2 enable guide-RNA processing and RNA detection" Nature doi:10/1038/nature19802 and Abudayyeh et al. "C2c2 is a single-component programmable RNA-guided RNA targeting CRISPR effector" bioRxiv doi:10.1101/054742.

RNase function in CRISPR systems is known, for example mRNA targeting has been reported for certain type III CRISPR-Cas systems (Hale et al., 2014, Genes Dev, vol. 28, 2432-2443; Hale et al., 2009, Cell, vol. 139, 945-956; Peng et al., 2015, Nucleic acids research, vol. 43, 406-417) and provides significant advantages. In the *Staphylococcus epidermis* type III-A system, transcription across targets results in cleavage of the target DNA and its transcripts, mediated by independent active sites within the Cas10-Csm ribonucleoprotein effector protein complex (see, Samai et al., 2015, Cell, vol. 151, 1164-1174). A CRISPR-Cas system, composition or method targeting RNA via the present effector proteins is thus provided.

In an embodiment, the Cas protein may be a C2c2 ortholog of an organism of a genus which includes but is not limited to Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Campylobacter, and Lachnospira. Species of organism of such a genus can be as otherwise herein discussed.

In certain example embodiments, the C2c2 effector proteins of the invention include, without limitation, the following 21 ortholog species (including multiple CRISPR loci: *Leptotrichia shahii; Leptotrichia wadei (Lw2); Listeria seeligeri; Lachnospiraceae bacterium MA2020; Lachnospiraceae bacterium NK4A179; [Clostridium] aminophilum DSM 10710; Carnobacterium gallinarum DSM 4847; Carnobacterium gallinarum DSM 4847 (second CRISPR Loci); Paludibacter propionicigenes WB4; Listeria weihenstephanensis FSL R9-0317; Listeriaceae bacterium FSL M6-0635; Leptotrichia wadei F0279; Rhodobacter capsulatus SB 1003; Rhodobacter capsulatus R121; Rhodobacter capsulatus DE442; Leptotrichia buccalis C-1013-b; Herbinix hemicellulosilytica; [Eubacterium] rectale; Eubacteriaceae bacterium CHKCI004; Blautia sp. Marseille-P2398;* and *Leptotrichia sp. oral taxon 879 str. F0557.* Twelve (12) further non-limiting examples are: *Lachnospiraceae bacterium NK4A144; Chloroflexus aggregans; Demequina aurantiaca; Thalassospira sp. TSL5-1; Pseudobutyrivibrio sp. OR37; Butyrivibrio sp. YAB3001; Blautia sp. Marseille-P2398; Leptotrichia sp. Marseille-P3007; Bacteroides ihuae; Porphyromonadaceae bacterium K113CP3RA; Listeria riparia;* and *Insolitispirillum peregrinum.*

Some methods of identifying orthologues of CRISPR-Cas system enzymes may involve identifying tracr sequences in genomes of interest. Identification of tracr sequences may relate to the following steps: Search for the direct repeats or tracr mate sequences in a database to identify a CRISPR region comprising a CRISPR enzyme. Search for homologous sequences in the CRISPR region flanking the CRISPR enzyme in both the sense and antisense directions. Look for transcriptional terminators and secondary structures. Identify any sequence that is not a direct repeat or a tracr mate sequence but has more than 50% identity to the direct repeat or tracr mate sequence as a potential tracr sequence. Take the potential tracr sequence and analyze for transcriptional terminator sequences associated therewith.

It will be appreciated that any of the functionalities described herein may be engineered into CRISPR enzymes from other orthologs, including chimeric enzymes comprising fragments from multiple orthologs. Examples of such orthologs are described elsewhere herein. Thus, chimeric enzymes may comprise fragments of CRISPR enzyme orthologs of an organism which includes but is not limited to Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma and Campylobacter. A chimeric enzyme can comprise a first fragment and a second fragment, and the fragments can be of CRISPR enzyme orthologs of organisms of genera herein mentioned or of species herein mentioned; advantageously the fragments are from CRISPR enzyme orthologs of different species.

In embodiments, the C2c2 protein as referred to herein also encompasses a functional variant of C2c2 or a homologue or an orthologue thereof. A "functional variant" of a protein as used herein refers to a variant of such protein which retains at least partially the activity of that protein. Functional variants may include mutants (which may be insertion, deletion, or replacement mutants), including polymorphs, etc. Also included within functional variants are fusion products of such protein with another, usually unrelated, nucleic acid, protein, polypeptide or peptide. Functional variants may be naturally occurring or may be manmade. Advantageous embodiments can involve engineered or non-naturally occurring Type VI RNA-targeting effector protein.

In an embodiment, nucleic acid molecule(s) encoding the C2c2 or an ortholog or homolog thereof, may be codon-optimized for expression in a eukaryotic cell. A eukaryote can be as herein discussed. Nucleic acid molecule(s) can be engineered or non-naturally occurring.

In an embodiment, the C2c2 or an ortholog or homolog thereof, may comprise one or more mutations (and hence nucleic acid molecule(s) coding for same may have mutation(s). The mutations may be artificially introduced mutations and may include but are not limited to one or more mutations in a catalytic domain. Examples of catalytic domains with reference to a Cas9 enzyme may include but are not limited to RuvC I, RuvC II, RuvC III and HNH domains.

In an embodiment, the C2c2 or an ortholog or homolog thereof, may comprise one or more mutations. The mutations may be artificially introduced mutations and may include but are not limited to one or more mutations in a catalytic domain. Examples of catalytic domains with reference to a Cas enzyme may include but are not limited to HEPN domains.

In an embodiment, the C2c2 or an ortholog or homolog thereof, may be used as a generic nucleic acid binding protein with fusion to or being operably linked to a functional domain. Exemplary functional domains may include but are not limited to translational initiator, translational activator, translational repressor, nucleases, in particular ribonucleases, a spliceosome, beads, a light inducible/controllable domain or a chemically inducible/controllable domain.

In certain example embodiments, the C2c2 effector protein may be from an organism selected from the group consisting of; Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, and Campylobacter.

In certain embodiments, the effector protein may be a Listeria sp. C2c2p, preferably Listeria seeligeria C2c2p, more preferably Listeria seeligeria serovar 1/2b str. SLCC3954 C2c2p and the crRNA sequence may be 44 to 47 nucleotides in length, with a 5' 29-nt direct repeat (DR) and a 15-nt to 18-nt spacer.

In certain embodiments, the effector protein may be a Leptotrichia sp. C2c2p, preferably Leptotrichia shahii C2c2p, more preferably Leptotrichia shahii DSM 19757 C2c2p and the crRNA sequence may be 42 to 58 nucleotides in length, with a 5' direct repeat of at least 24 nt, such as a 5' 24-28-nt direct repeat (DR) and a spacer of at least 14 nt, such as a 14-nt to 28-nt spacer, or a spacer of at least 18 nt, such as 19, 20, 21, 22, or more nt, such as 18-28, 19-28, 20-28, 21-28, or 22-28 nt.

In certain example embodiments, the effector protein may be a Leptotrichia sp., Leptotrichia wadei F0279, or a Listeria sp., preferably Listeria newyorkensis FSL M6-0635.

In certain example embodiments, the C2c2 effector proteins of the invention include, without limitation, the following 21 ortholog species (including multiple CRISPR loci: Leptotrichia shahii; Leptotrichia wadei (Lw2); Listeria seeligeri; Lachnospiraceae bacterium MA2020; Lachnospiraceae bacterium NK4A179; [Clostridium] aminophilum DSM 10710; Carnobacterium gallinarum DSM 4847; Carnobacterium gallinarum DSM 4847 (second CRISPR Loci); Paludibacter propionicigenes WB4; Listeria weihenstephanensis FSL R9-0317; Listeriaceae bacterium FSL M6-0635; Leptotrichia wadei F0279; Rhodobacter capsulatus SB 1003; Rhodobacter capsulatus R121; Rhodobacter capsulatus DE442; Leptotrichia buccalis C-1013-b; Herbinix hemicellulosilytica; [Eubacterium] rectale; Eubacteriaceae bacterium CHKCI004; Blautia sp. Marseille-P2398; and Leptotrichia sp. oral taxon 879 str. F0557. Twelve (12) further non-limiting examples are: Lachnospiraceae bacterium NK4A144; Chloroflexus aggregans; Demequina aurantiaca; Thalassospira sp. TSL5-1; Pseudobutyrivibrio sp. OR37; Butyrivibrio sp. YAB3001; Blautia sp. Marseille-P2398; Leptotrichia sp. Marseille-P3007; Bacteroides ihuae; Porphyromonadaceae bacterium KH3CP3RA; Listeria riparia; and Insolitispirillum peregrinum.

In certain embodiments, the C2c2 protein according to the invention is or is derived from one of the orthologues or is a chimeric protein of two or more of the orthologues as described in this application, or is a mutant or variant of one of the orthologues (or a chimeric mutant or variant), including dead C2c2, split C2c2, destabilized C2c2, etc. as defined herein elsewhere, with or without fusion with a heterologous/functional domain.

In certain example embodiments, the RNA-targeting effector protein is a Type VI-B effector protein, such as Cas13b and Group 29 or Group 30 proteins. In certain example embodiments, the RNA-targeting effector protein comprises one or more HEPN domains. In certain example embodiments, the RNA-targeting effector protein comprises a C-terminal HEPN domain, a N-terminal HEPN domain, or both. Regarding example Type VI-B effector proteins that may be used in the context of this invention, reference is made to US Application No. 15/331,792 entitled "Novel CRISPR Enzymes and Systems" and filed October 21, 2016, International Patent Application No. PCT/US2016/058302 entitled "Novel CRISPR Enzymes and Systems", and filed October 21, 2016, and Smargon et al. "Cas13b is a Type VI-B CRISPR-associated RNA-Guided RNase differentially regulated by accessory proteins Csx27 and Csx28" Molecular Cell, 65, 1-13 (2017); dx.doi.org/10.1016/j.molcel.2016.12.023, and U.S. Provisional Application No. to be assigned, entitled "Novel Cas13b Orthologues CRISPR Enzymes and System" filed March 15, 2017. In one preferred embodiment, the Cas 13 protein is LwaCas13.

### Masking Constructs

As used herein, a "masking construct" refers to a molecule that can be cleaved or otherwise deactivated by an activated CRISPR system effector protein described herein. The term "masking construct" may also be referred to in the alternative as a "detection construct." In certain example embodiments, the masking construct is a RNA-based masking construct. The RNA-based masking construct comprises a RNA element that is cleavable by a CRISPR effector protein. Cleavage of the RNA element releases agents or produces conformational changes that allow a detectable signal to be produced. Example constructs demonstrating how the RNA element may be used to prevent or mask generation of detectable signal are described below and embodiments of the invention comprise variants of the same. Prior to cleavage, or when the masking construct is in an 'active' state, the masking construct blocks the generation or detection of a positive detectable signal. It will be understood that in certain example embodiments a minimal background signal may be produced in the presence of an active RNA masking construct. A positive detectable signal may be any signal that can be detected using optical, fluorescent, chemiluminescent, electrochemical or other detection methods known in the art. The term "positive detectable signal" is used to differentiate from other detectable signals that may be detectable in the presence of the masking construct. For example, in certain embodiments a first signal may be detected when the masking agent is present (i.e. a negative detectable signal), which then converts to a second signal (e.g. the positive detectable signal) upon detection of the target molecules and cleavage or deactivation of the masking agent by the activated CRISPR effector protein.

In certain example embodiments, the masking construct may suppress generation of a gene product. The gene product may be encoded by a reporter construct that is added to the sample. The masking construct may be an interfering RNA involved in a RNA interference pathway, such as a short hairpin RNA (shRNA) or small interfering RNA (siRNA). The masking construct may also comprise microRNA (miRNA). While present, the masking construct suppresses expression of the gene product. The gene product may be a fluorescent protein or other RNA transcript or proteins that would otherwise be detectable by a labeled probe, aptamer, or antibody but for the presence of the masking construct. Upon activation of the effector protein the masking construct is cleaved or otherwise silenced allowing for expression and detection of the gene product as the positive detectable signal.

In certain example embodiments, the masking construct may sequester one or more reagents needed to generate a detectable positive signal such that release of the one or more reagents from the masking construct results in generation of the detectable positive signal. The one or more reagents may combine to produce a colorimetric signal, a chemiluminescent signal, a fluorescent signal, or any other detectable signal and may comprise any reagents known to be suitable for such purposes. In certain example embodiments, the one or more reagents are sequestered by RNA aptamers that bind the one or more reagents. The one or more reagents are released when the effector protein is activated upon detection of a target molecule and the RNA aptamers are degraded.

In other embodiments of the invention, the RNA-based masking construct suppresses generation of a detectable positive signal or the RNA-based masking construct suppresses generation of a detectable positive signal by masking the detectable positive signal, or generating a detectable negative signal instead, or the RNA-based masking construct comprises a silencing RNA that suppresses generation of a gene product encoded by a reporting construct, wherein the gene product generates the detectable positive signal when expressed.

In further embodiments, the RNA-based masking construct is a ribozyme that generates the negative detectable signal, and wherein the positive detectable signal is generated when the ribozyme is deactivated, or the ribozyme converts a substrate to a first color and wherein the substrate converts to a second color when the ribozyme is deactivated.

In other embodiments, the RNA-based masking agent is an RNA aptamer, or the aptamer sequesters an enzyme, wherein the enzyme generates a detectable signal upon release from the aptamer by acting upon a substrate, or the aptamer sequesters a pair of agents that when released from the aptamers combine to generate a detectable signal.

In another embodiment, the RNA-based masking construct comprises an RNA oligonucleotide to which a detectable ligand and a masking component are attached. In another embodiment, the detectable ligand is a fluorophore and the masking component is a quencher molecule, or the reagents to amplify target RNA molecules such as

In certain example embodiments, the masking construct may be immobilized on a solid substrate in an individual discrete volume (defined further below) and sequesters a single reagent. For example, the reagent may be a bead comprising a dye. When sequestered by the immobilized reagent, the individual beads are too diffuse to generate a detectable signal, but upon release from the masking construct are able to generate a detectable signal, for example by aggregation or simple increase in solution concentration. In certain example embodiments, the immobilized masking agent is a RNA-based aptamer that can be cleaved by the activated effector protein upon detection of a target molecule.

In certain other example embodiments, the masking construct binds to an immobilized reagent in solution thereby blocking the ability of the reagent to bind to a separate labeled binding partner that is free in solution. Thus, upon application of a washing step to a sample, the labeled binding partner can be washed out of the sample in the absence of a target molecule. However, if the effector protein is activated, the masking construct is cleaved to a degree sufficient to interfere with the ability of the masking construct to bind the reagent thereby allowing the labeled binding partner to bind to the immobilized reagent. Thus, the labeled binding partner remains after the wash step indicating the presence of the target molecule in the sample. In certain aspects, the masking construct that binds the immobilized reagent is an RNA aptamer. The immobilized reagent may be a protein and the labeled minding partner may be a labeled antibody. Alternatively, the immobilized reagent may be streptavidin and the labeled binding partner may be labeled biotin. The label on the binding partner used in the above embodiments may be any detectable label known in the art. In addition, other known binding partners may be used in accordance with the overall design described herein.

In certain example embodiments, the masking construct may comprise a ribozyme. Ribozymes are RNA molecules having catalytic properties. Ribozymes, both naturally and engineered, comprise or consist of RNA that may be targeted by the effector proteins disclosed herein. The ribozyme may be selected or engineered to catalyze a reaction that either generates a negative detectable signal or prevents generation of a positive control signal. Upon deactivation of the ribozyme by the activated effector protein the reaction generating a negative control signal, or preventing generation of a positive detectable signal, is removed thereby allowing a positive detectable signal to be generated. In one example embodiment, the ribozyme may catalyze a colorimetric reaction causing a solution to appear as a first color. When the ribozyme is deactivated the solution then turns to a second color, the second color being the detectable positive signal. An example of how ribozymes can be used to catalyze a colorimetric reaction are described in Zhao et al. "Signal amplification of glucosamine-6-phosphate based on ribozyme glmS," Biosens Bioelectron. 2014; 16:337-42, and provide an example of how such a system could be modified to work in the context of the embodiments disclosed herein. Alternatively, ribozymes, when present can generate cleavage products of, for example, RNA transcripts. Thus, detection of a positive detectable signal may comprise detection of non-cleaved RNA transcripts that are only generated in the absence of the ribozyme.

In certain example embodiments, the one or more reagents is a protein, such as an enzyme, capable of facilitating generation of a detectable signal, such as a colorimetric, chemiluminescent, or fluorescent signal, that is inhibited or sequestered such that the protein cannot generate the detectable signal by the binding of one or more RNA aptamers to the protein. Upon activation of the effector proteins disclosed herein, the RNA aptamers are cleaved or degraded to an extent that they no longer inhibit the protein's ability to generate the detectable signal. In certain example embodiments, the aptamer is a thrombin inhibitor aptamer. In certain example embodiments the thrombin inhibitor aptamer has a sequence of GGGAACAAAGCUGAAGUACUUACCC (SEQ ID NO: 18). When this aptamer is cleaved, thrombin will become active and will cleave a peptide colorimetric or fluorescent substrate. In certain example embodiments, the colorimetric substrate is para-nitroanilide (pNA) covalently linked to the peptide substrate for thrombin. Upon cleavage by thrombin, pNA is released and becomes yellow in color and easily visible to the eye. In certain example embodiments, the fluorescent substrate is 7-amino-4-methylcoumarin a blue fluorophore that can be detected using a fluorescence detector. Inhibitory aptamers may also be used for horseradish peroxidase (HRP), beta-galactosidase, or calf alkaline phosphatase (CAP) and within the general principals laid out above.

In certain embodiments, RNAse activity is detected colorimetrically via cleavage of enzyme-inhibiting aptamers. One potential mode of converting RNAse activity into a colorimetric signal is to couple the cleavage of an RNA aptamer with the re-activation of an enzyme that is capable of producing a colorimetric output. In the absence of RNA cleavage, the intact aptamer will bind to the enzyme target and inhibit its activity. The advantage of this readout system is that the enzyme provides an additional amplification step: once liberated from an aptamer via collateral activity (e.g. Cas13a collateral activity), the colorimetric enzyme will continue to produce colorimetric product, leading to a multiplication of signal.

In certain embodiments, an existing aptamer that inhibits an enzyme with a colorimetric readout is used. Several aptamer/enzyme pairs with colorimetric readouts exist, such as thrombin, protein C, neutrophil elastase, and subtilisin. These proteases have colorimetric substrates based upon pNA and are commercially available. In certain embodiments, a novel aptamer targeting a common colorimetric enzyme is used. Common and robust enzymes, such as beta-galactosidase, horseradish peroxidase, or calf intestinal alkaline phosphatase, could be targeted by engineered aptamers designed by selection strategies such as SELEX. Such strategies allow for quick selection of aptamers with nanomolar binding efficiencies and could be used for the development of additional enzyme/aptamer pairs for colorimetric readout.

In certain embodiments, RNAse activity is detected colorimetrically via cleavage of RNA-tethered inhibitors. Many common colorimetric enzymes have competitive, reversible inhibitors: for example, beta-galactosidase can be inhibited by galactose. Many of these inhibitors are weak, but their effect can be increased by increases in local concentration. By linking local concentration of inhibitors to RNAse activity, colorimetric enzyme and inhibitor pairs can be engineered into RNAse sensors. The colorimetric RNAse sensor based upon smallmolecule inhibitors involves three components: the colorimetric enzyme, the inhibitor, and a bridging RNA that is covalently linked to both the inhibitor and enzyme, tethering the inhibitor to the enzyme. In the uncleaved configuration, the enzyme is inhibited by the increased local concentration of the small molecule; when the RNA is cleaved (e.g. by Cas13a collateral cleavage), the inhibitor will be released and the colorimetric enzyme will be activated.

In certain embodiments, RNAse activity is detected colorimetrically via formation and/or activation of G-quadruplexes. G quadraplexes in DNA can complex with heme (iron (III)-protoporphyrin IX) to form a DNAzyme with peroxidase activity. When supplied with a peroxidase substrate (e.g. ABTS: (2,2'-Azinobis [3-ethylbenzothiazoline-6-sulfonic acid]-diammonium salt)), the G-quadraplex-heme complex in the presence of hydrogen peroxide causes oxidation of the substrate, which then forms a green color in solution. An example G-quadraplex forming DNA sequence is: GGGTAGGGCGGGTTGGGA (SEQ. I.D. No. 19). By hybridizing an RNA sequence to this DNA aptamer, formation of the G-quadraplex structure will be limited. Upon RNAse collateral activation (e.g. C2c2-complex collateral activation), the RNA staple will be cleaved allowing the G quadraplex to form and heme to bind. This strategy is particularly appealing because color formation is enzymatic, meaning there is additional amplification beyond RNAse activation.

In certain example embodiments, the masking construct may be immobilized on a solid substrate in an individual discrete volume (defined further below) and sequesters a single reagent. For example, the reagent may be a bead comprising a dye. When sequestered by the immobilized reagent, the individual beads are too diffuse to generate a detectable signal, but upon release from the masking construct are able to generate a detectable signal, for example by aggregation or simple increase in solution concentration. In certain example embodiments, the immobilized masking agent is a RNA-based aptamer that can be cleaved by the activated effector protein upon detection of a target molecule.

In one example embodiment, the masking construct comprises a detection agent that changes color depending on whether the detection agent is aggregated or dispersed in solution. For example, certain nanoparticles, such as colloidal gold, undergo a visible purple to red color shift as they move from aggregates to dispersed particles. Accordingly, in certain example embodiments, such detection agents may be held in aggregate by one or more bridge molecules. At least a portion of the bridge molecule comprises RNA. Upon activation of the effector proteins disclosed herein, the RNA portion of the bridge molecule is cleaved allowing the detection agent to disperse and resulting in the corresponding change in color. See e.g. FIG. 46. In certain example embodiments the, bridge molecule is a RNA molecule. In certain example embodiments, the detection agent is a colloidal metal. The colloidal metal material may include water-insoluble metal particles or metallic compounds dispersed in a liquid, a hydrosol, or a metal sol. The colloidal metal may be selected from the metals in groups IA, IB, IIB and IIIB of the periodic table, as well as the transition metals, especially those of group VIII. Preferred metals include gold, silver, aluminum, ruthenium, zinc, iron, nickel and calcium. Other suitable metals also include the following in all of their various oxidation states: lithium, sodium, magnesium, potassium, scandium, titanium, vanadium, chromium, manganese, cobalt, copper, gallium, strontium, niobium, molybdenum, palladium, indium, tin, tungsten, rhenium, platinum, and gadolinium. The metals are preferably provided in ionic form, derived from an appropriate metal compound, for example the A13+, Ru3+, Zn2+, Fe3+, Ni2+ and Ca2+ ions.

When the RNA bridge is cut by the activated CRISPR effector, the beforementioned color shift is observed. In certain example embodiments the particles are colloidal metals. In certain other example embodiments, the colloidal metal is a colloidal gold. In certain example embodiments, the colloidal nanoparticles are 15 nm gold nanoparticles (AuNPs). Due to the unique surface properties of colloidal gold nanoparticles, maximal absorbance is observed at 520 nm when fully dispersed in solution and appear red in color to the naked eye. Upon aggregation of AuNPs, they exhibit a red-shift in maximal absorbance and appear darker in color, eventually precipitating from solution as a dark purple aggregate. In certain example embodiments the nanoparticles are modified to include DNA linkers extending from the surface of the nanoparticle. Individual particles are linked together by single-stranded RNA (ssRNA) bridges that hybridize on each end of the RNA to at least a portion of the DNA linkers. Thus, the nanoparticles will form a web of linked particles and aggregate, appearing as a dark precipitate. Upon activation of the CRISPR effectors disclosed herein, the ssRNA bridge will be cleaved, releasing the AU NPS from the linked mesh and producing a visible red color. Example DNA linkers and RNA bridge sequences are listed below. Thiol linkers on the end of the DNA linkers may be used for surface conjugation to the AuNPS. Other forms of conjugation may be used. In certain example embodiments, two populations of AuNPs may be generated, one for each DNA linker. This will help facilitate proper binding of the ssRNA bridge with proper orientation. In certain example embodiments, a first DNA linker is conjugated by the 3' end while a second DNA linker is conjugated by the 5' end.

**Table 1.**

| | |
|---|---|
| C2c2 colorimetric DNA 1 | TTATAACTATTCCTAAAAAAAAAAAA/3T hioMC3-D/ (SEQ. I.D. No. 20) |
| C2c2 colorimetric DNA2 | /5ThioMC6- D/AAAAAAAAAACTCCCCTAATAACAA T (SEQ. I.D. No. 21) |
| C2c2 colorimetric bridge | GGGUAGGAAUAGUUAUAAUUUCCCUU UCCCAUUGUUAUUAGGGAG (SEQ. I.D. No. 22) |

In certain other example embodiments, the masking construct may comprise an RNA oligonucleotide to which are attached a detectable label and a masking agent of that detectable label. An example of such a detectable label/masking agent pair is a fluorophore and a quencher of the fluorophore. Quenching of the fluorophore can occur as a result of the formation of a non-fluorescent complex between the fluorophore and another fluorophore or non-fluorescent molecule. This mechanism is known as ground-state complex formation, static quenching, or contact quenching. Accordingly, the RNA oligonucleotide may be designed so that the fluorophore and quencher are in sufficient proximity for contact quenching to occur. Fluorophores and their cognate quenchers are known in the art and can be selected for this purpose by one having ordinary skill in the art. The particular fluorophore/quencher pair is not critical in the context of this invention, only that selection of the fluorophore/quencher pairs ensures masking of the fluorophore. Upon activation of the effector proteins disclosed herein, the RNA oligonucleotide is cleaved thereby severing the proximity between the fluorophore and quencher needed to maintain the contact quenching effect. Accordingly, detection of the fluorophore may be used to determine the presence of a target molecule in a sample.

In certain other example embodiments, the masking construct may comprise one or more RNA oligonucleotides to which are attached one or more metal nanoparticles, such as gold nanoparticles. In some embodiments, the masking construct comprises a plurality of metal nanoparticles crosslinked by a plurality of RNA oligonucleotides forming a closed loop. In one embodiment, the masking construct comprises three gold nanoparticles crosslinked by three RNA oligonucleotides forming a closed loop. In some embodiments, the cleavage of the RNA oligonucleotides by the CRISPR effector protein leads to a detectable signal produced by the metal nanoparticles.

In certain other example embodiments, the masking construct may comprise one or more RNA oligonucleotides to which are attached one or more quantum dots. In some embodiments, the cleavage of the RNA oligonucleotides by the CRISPR effector protein leads to a detectable signal produced by the quantum dots.

In one example embodiment, the masking construct may comprise a quantum dot. The quantum dot may have multiple linker molecules attached to the surface. At least a portion of the linker molecule comprises RNA. The linker molecule is attached to the quantum dot at one end and to one or more quenchers along the length or at terminal ends of the linker such that the quenchers are maintained in sufficient proximity for quenching of the quantum dot to occur. The linker may be branched. As above, the quantum dot/quencher pair is not critical, only that selection of the quantum dot/quencher pair ensures masking of the fluorophore. Quantum dots and their cognate quenchers are known in the art and can be selected for this purpose by one having ordinary skill in the art Upon activation of the effector proteins disclosed herein, the RNA portion of the linker molecule is cleaved thereby eliminating the proximity between the quantum dot and one or more quenchers needed to maintain the quenching effect. In certain example embodiments the quantum dot is streptavidin conjugated. RNA are attached via biotin linkers and recruit quenching molecules with the sequences /5Biosg/UCUCGUACGUUC/3IAbRQSp/ (SEQ ID NO. 23) or /5Biosg/UCUCGUACGUUCUCUCGUACGUUC/3IAbRQSp/ (SEQ ID NO. 24), where /5Biosg/ is a biotin tag and /3lAbRQSp/ is an Iowa black quencher. Upon cleavage, by the activated effectors disclosed herein the quantum dot will fluoresce visibly.

In a similar fashion, fluorescence energy transfer (FRET) may be used to generate a detectable positive signal. FRET is a non-radiative process by which a photon from an energetically excited fluorophore (i.e. "donor fluorophore") raises the energy state of an electron in another molecule (i.e. "the acceptor") to higher vibrational levels of the excited singlet state. The donor fluorophore returns to the ground state without emitting a fluoresce characteristic of that fluorophore. The acceptor can be another fluorophore or non-fluorescent molecule. If the acceptor is a fluorophore, the transferred energy is emitted as fluorescence characteristic of that fluorophore. If the acceptor is a non-fluorescent molecule the absorbed energy is loss as heat. Thus, in the context of the embodiments disclosed herein, the fluorophore/quencher pair is replaced with a donor fluorophore/acceptor pair attached to the oligonucleotide molecule. When intact, the masking construct generates a first signal (negative detectable signal) as detected by the fluorescence or heat emitted from the acceptor. Upon activation of the effector proteins disclosed herein the RNA oligonucleotide is cleaved and FRET is disrupted such that fluorescence of the donor fluorophore is now detected (positive detectable signal).

In certain example embodiments, the masking construct comprises the use of intercalating dyes which change their absorbance in response to cleavage of long RNAs to short nucleotides. Several such dyes exist. For example, pyronine-Y will complex with RNA and form a complex that has an absorbance at 572 nm. Cleavage of the RNA results in loss of absorbance and a color change. Methylene blue may be used in a similar fashion, with changes in absorbance at 688 nm upon RNA cleavage. Accordingly, in certain example embodiments the masking construct comprises a RNA and intercalating dye complex that changes absorbance upon the cleavage of RNA by the effector proteins disclosed herein.

In certain example embodiments, the masking construct may comprise an initiator for an HCR reaction. See e.g. Dirks and Pierce. PNAS 101, 15275-15728 (2004). HCR reactions utilize the potential energy in two hairpin species. When a single-stranded initiator having a portion of complementary to a corresponding region on one of the hairpins is released into the previously stable mixture, it opens a hairpin of one species. This process, in turn, exposes a single-stranded region that opens a hairpin of the other species. This process, in turn, exposes a single stranded region identical to the original initiator. The resulting chain reaction may lead to the formation of a nicked double helix that grows until the hairpin supply is exhausted. Detection of the resulting products may be done on a gel or colorimetrically. Example colorimetric detection methods include, for example, those disclosed in Lu et al. "Ultra-sensitive colorimetric assay system based on the hybridization chain reaction-triggered enzyme cascade amplification ACS Appl Mater Interfaces, 2017, 9(1): 167-175, Wang et al. "An enzyme-free colorimetric assay using hybridization chain reaction amplification and split aptamers" Analyst 2015, 150, 7657-7662, and Song et al. "Non covalent fluorescent labeling of hairpin DNA probe coupled with hybridization chain reaction for sensitive DNA detection." Applied Spectroscopy, 70(4): 686-694 (2016).

In certain example embodiments, the masking construct may comprise a HCR initiator sequence and a cleavable structural element, such as a loop or hairpin, that prevents the initiator from initiating the HCR reaction. Upon cleavage of the structure element by an activated CRISPR effector protein, the initiator is then released to trigger the HCR reaction, detection thereof indicating the presence of one or more targets in the sample. In certain example embodiments, the masking construct comprises a hairpin with a RNA loop. When an activated CRISRP effector protein cuts the RNA loop, the initiator can be released to trigger the HCR reaction.

In specific embodiments, the target nucleic acid may be detected at attomolar sensitivity. In specific embodiments, the target nucleic acid may be detected at femtomolar sensitivity. In some specific embodiments, the methods are performed in less than about 2 hours, less than about 90 minutes, less than about 60 minutes, less than about 30 minutes or less than about 15 minutes. In some preferred embodiments, amplification and detection can occur in a one-pot method with 2 fM detection in less than about 2 hours.

### SYSTEMS FOR AMPLIFICATION AND DETECTION

### Systems for Amplification and Detection

In some embodiments, the invention provides a system for amplifying and/or detecting a target double-stranded nucleic acid in a sample. The system may include an amplification CRISPR system as described herein. The amplification CRISPR system may include a first and second CRIPR/Cas complex as described herein. The first CRISPR/Cas complex may comprise a first CRISPR/Cas enzyme and a first guide molecule that guides the first CRISPR/Cas complex to a first strand of the target nucleic acid. The second CRISPR/Cas complex may comprise a second CRISPR/Cas enzyme and a second guide molecule that guides the second CRISPR/Cas complex to a second strand of the target nucleic acid. The first and second CRISPR/Cas enzymes may be active or dead. In cases where the target nucleic acid is really long, such as genomic DNA for example, active Cas enzymes may be useful, as described herein.

The dead CRISPR/Cas enzyme may include, but is not necessarily limited to, dead Cas9, dead Cas12. The dead Cas12 may be dead Cas12a, dead Cas12b, or dead Cas12c.

The system may further comprise a helicase, a primer pair, a polymerase, and optionally, a detection system for detecting amplification of the target nucleic acid, as described herein. The primer pair may comprise a first and second primer, with the first primer comprising a portion that is complementary to the first strand of the target nucleic acid and the second primer comprising a portion that is complementary to the second strand of the target nucleic acid.

The polymerase may include, but is not necessarily limited to, Bst 2.0 DNA polymerase, Bst 2.0 WarmStart DNA polymerase, Bst 3.0 DNA polymerase, full length Bst DNA polymerase, large fragment Bst DNA polymerase, large fragment Bsu DNA polymerase, phi29 DNA polymerase, T7 DNA polymerase, Gst polymerase, Taq polymerase, Klenow fragment of E. coli DNA polymerase I, KlenTaq NDA polymerase, Pol III DNA polymerase, T5 DNA polymerase, and Sequenase DNA polymerase.

The helicase may be, but is not necessarily limited to, UvrD helicase, CRISPR-Cas3 helicase, Rep helicase, PcrA helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase.

In specific embodiments, the helicase is Tte-UvrD D409A/D410A.

In specific embodiments, the dead CRISPR/Cas enzyme, the helicase, and the polymerase may perform under the same temperature. In specific embodiments, the temperature may be 37°C, but is not necessarily limited thereto.

In alternative embodiments, the invention provides a system for amplifying and/or detecting a target single-stranded nucleic acid in a sample. The system may include, but is not necessarily limited to, reagents for converting the target single-stranded nucleic acid to a double-stranded nucleic acid as described herein. They system may also comprise an amplification CRISPR system as described previously, a helicase, a primer pair, a polymerase, and optionally, a detection system as described herein.

In some embodiments, the detection system may comprise a CRISPR system comprising a Cas12 or Cas13 effector protein and a guide RNA designed to bind to a corresponding target molecule; and a masking construct.

In other embodiments, the invention comprises methods for amplifying and/or detecting a target single-stranded nucleic acid. Such single-stranded nucleic acids may include, but are not necessarily limited to single-stranded viral DNA, viral RNA, messenger RNA, ribosomal RNA, transfer RNA, microRNA, short interfering RNA, small nuclear RNA, synthetic RNA, or synthetic single-stranded DNA. In specific embodiments, the target single-stranded nucleic acid is an RNA molecule. The single-stranded nucleic acid may be converted to a target double-stranded nucleic acid prior to performing any of the steps and methods described thus far. Such conversion may be achieved by any known methods, such as by reverse-transcription and amplification.

In some embodiments, the detection system may comprise the Cas 13 enzyme LwaCas 13 .

In some embodiments, the detection system may further comprise an additional amplifier primer comprising a promoter sequence identity with the first primer of the primer pair.

In some embodiments, the helicase in the detection system is Tte-UvrD D409A/D410A. In some embodiments, the helicase comprises one or more A substitutions at a D in an enzyme.

In some embodiments, the system may further comprise one or more additives selected from a crowding agent, a co-factor, single-strand binding protein, and/or assisting proteins.

The crowding agent may be polyethylene glycol 20K molecular weight, optionally provided at about 2.5% to about 6.5%.

The co-factor may be ATP, optionally provided at 0.75 to about 5 mM.

The single-strand binding protein may include, but is not necessarily limited to T4 gp32 or thermophilic SSB (ET-SSB).

The assisting protein may be, but is not necessarily limited to dCpf1 and/or UvsX Recombinase.

The system may comprise a mesophilic DNA polymerase, particularly Sau LF Polymerase.

In any of the methods or systems described herein, the helicase may be PcrA helicase from Thermoanaerobacter ethanolicus with mutations D403A/D404A, PcrA helicase from Bacillus sp. FJAT-27231 with mutations D407A/D408A, PcrA helicase from Bacillus megaterium with mutations D415A/D416A, PcrA helicase from Bacillus simplex with mutations D407A/D408A, or PcrA helicase from Paeniclostridium sordellii with mutations D402A/D403A.

The invention is further described in the following examples, which do not limit the scope of the invention described in the claims.

### WORKING EXAMPLES

### Example 1: 2-step CRISPR tHDA-SHERLOCK

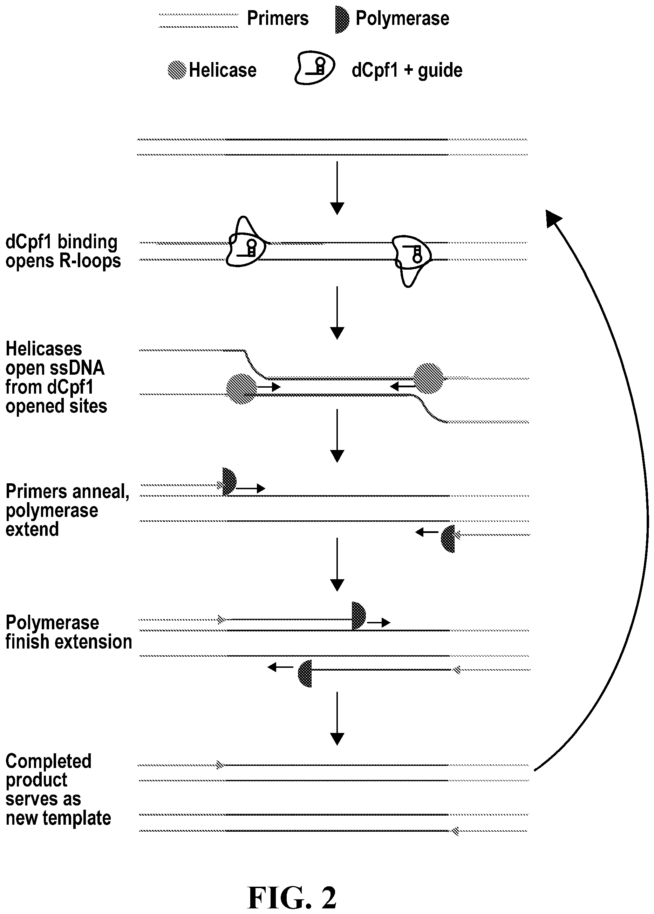

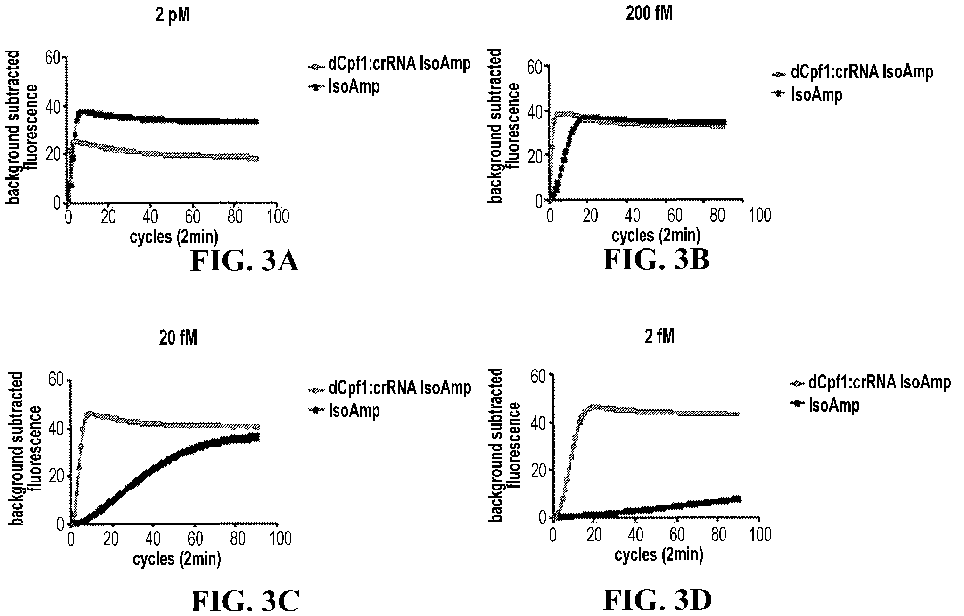

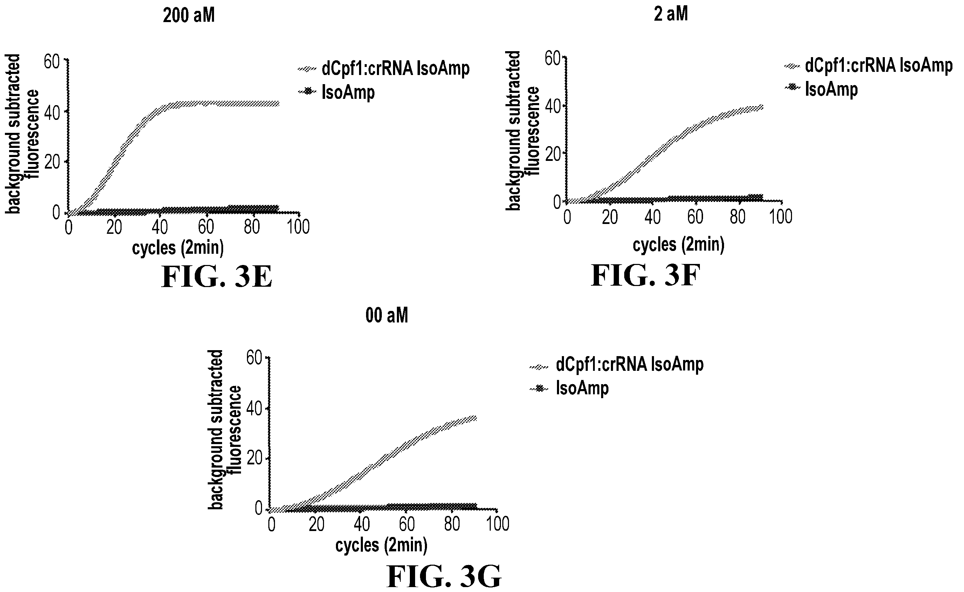

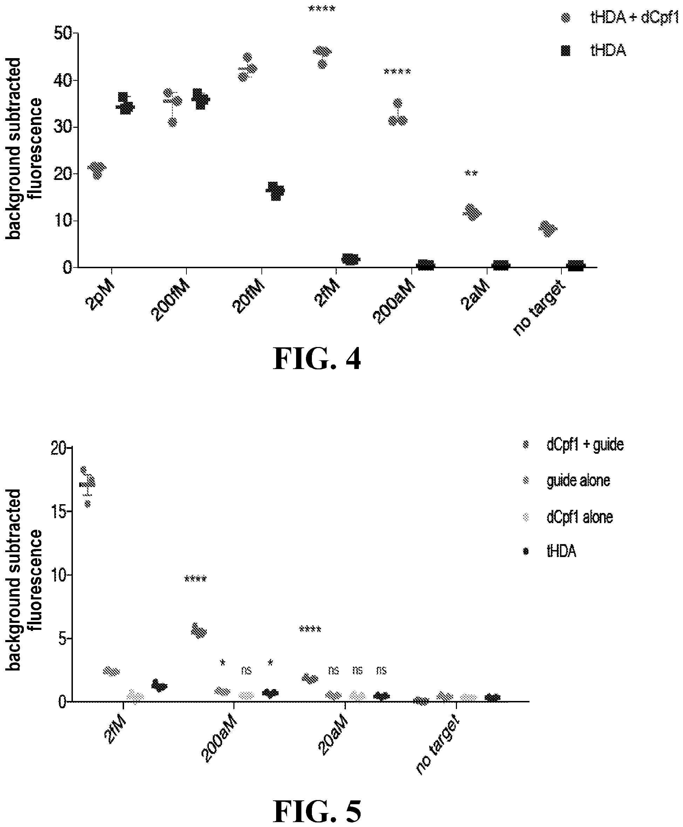

Thermophilic HDA ( tHDA) uses a reaction temperatures of 65 degree Celsius, with commercially available systems sold by NEB. An exemplary workflow of CRISPR system with tHDA is depicted in Figure 2. Utilizing the CRISPR-tDA workflow with SHERLOCK detection in a 2-step process allows for single molecule detection with improved results with the addition of CRISPR in the amplification system (Figures 3A-3G). Figure 5 shows the improvement is caused by dAsCpf1:crRNA complex, with both Cas12a and Cas12b also improving 2-step tHDA-SHERLOCK. However, accomplishing the HDA-SHERLOCK reaction at lower temperature reaction would also be an improvement.

### Example 2: 2-step CRISPR mHDA-SHERLOCK

While tHDA is a commercial system currently sold by NEB, there is no commercial system available for mesophilic HDA at 37 degree Celsius. The 2-step process takes longer than 90 minutes, and the tHDA-SHERLOCK system requires catalytically inactive programmable CRISPR enzymes such as Cas12a or Cas12b for sensitive detection and can lack sensitivity.

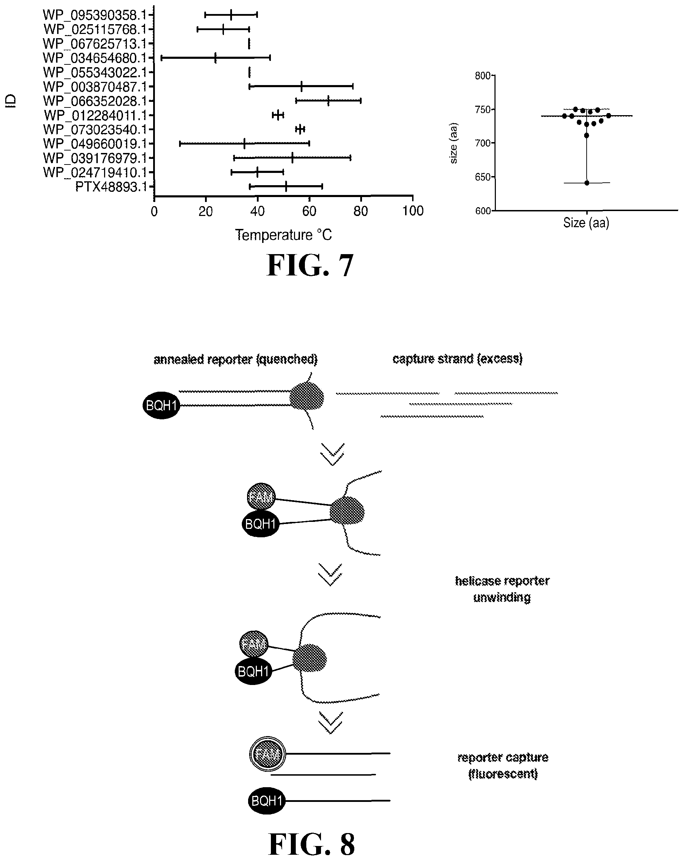

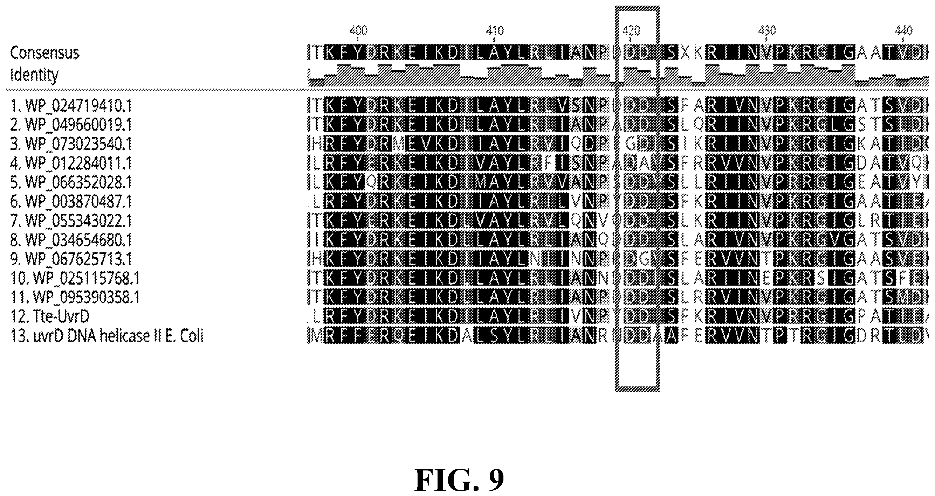

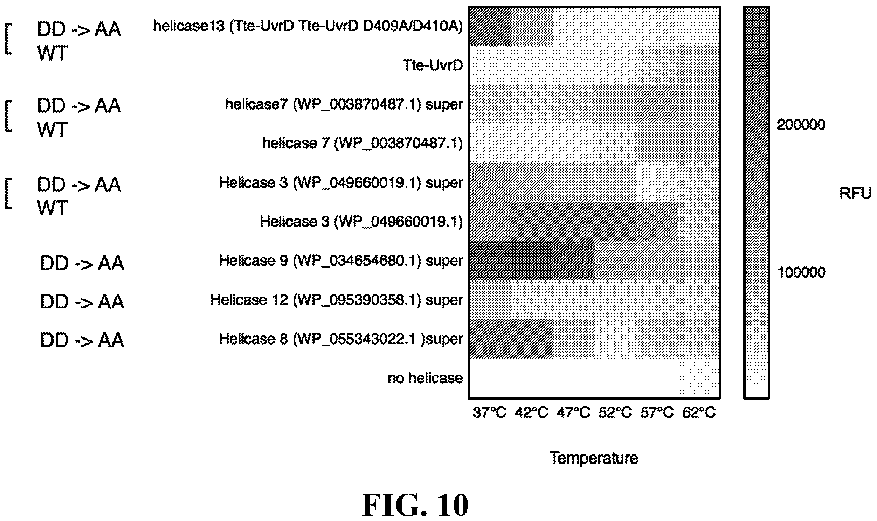

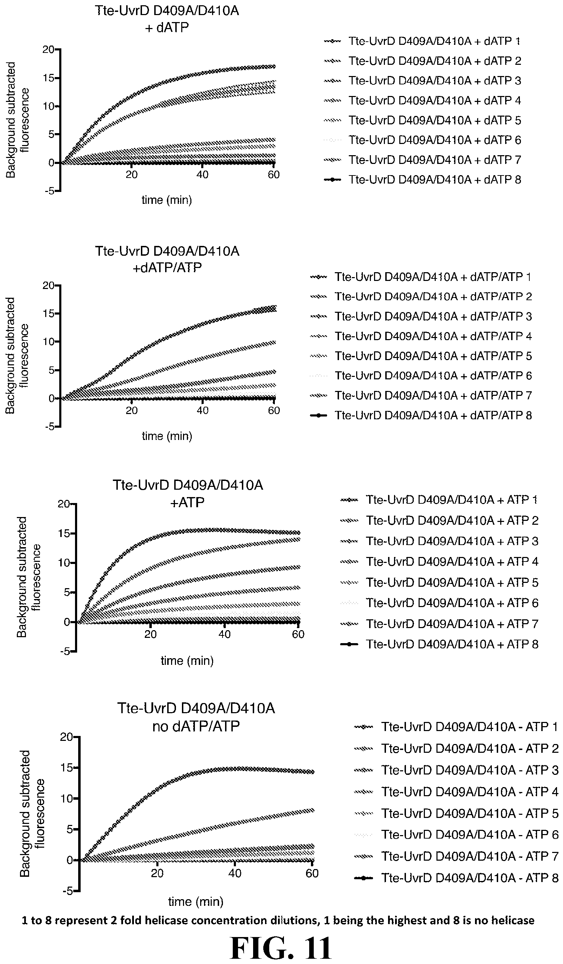

The inventors first undertook development of a workflow of combined CRISPR-HDA and SHERLOCK at mesophilic temperatures. Screening for mesophilic helicases , preferably that do not require accessory proteins for strand separation, and optimizing parameters for combined mesophilic HDA and SHERLOCK were performed. First, selection of helicases of UvrD type from mesophilic bacterial species was conducted (Figure 7). Next, a reporter assay was established to test activity (Figure 8). The helicases selected were mutagenized to increase activity (Figure 9), with selected pcrA (UvrD)-type helicase having a broad activity spectrum with substitutions increasing activity (Figure 10). Finally, Figure 11 shows the reporter assay can be used to infer co-factor preference and kinetics of helicase substrate unwinding.

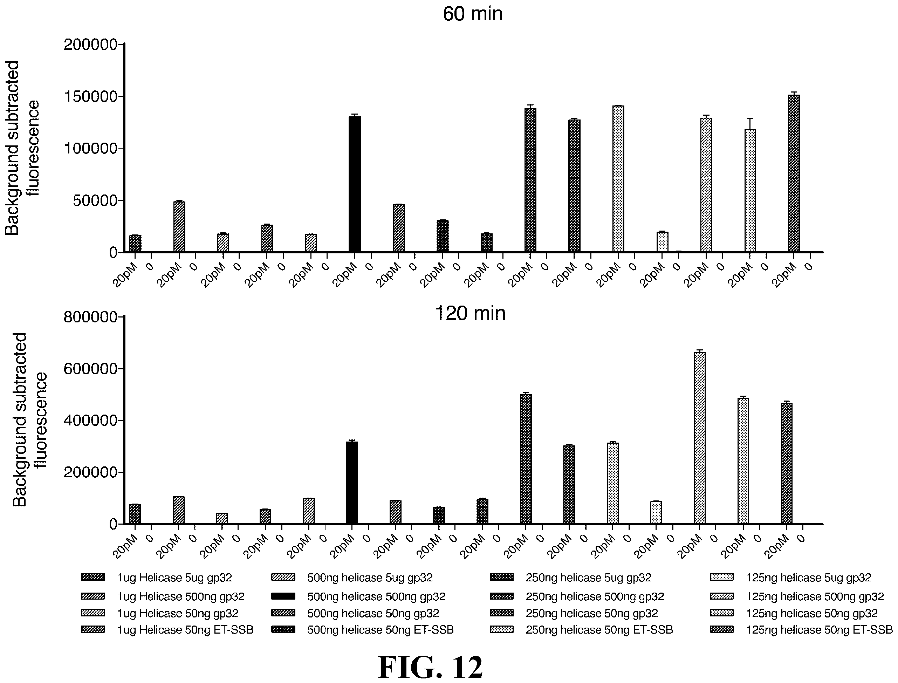

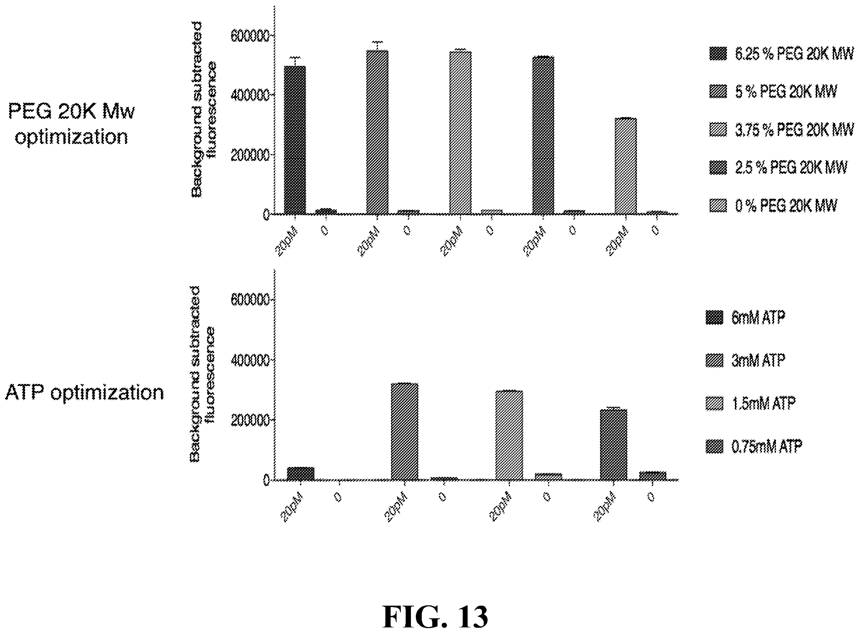

Next, the protocol was optimized for combined mesophilic HDA and SHERLOCK, including determining optimal enzymes and accessory proteins (Cas13, DNA polymerase, SSB). Regarding enzymes, six different wild-type UvrD-like helicases and mutants with super helicase mutations evaluated indicates Tte-UvrD D409A/D410A is robust. The mesophilic DNA polymerase is Sau LF Polymerase, with T7 RNA polymerase for in vitro transcription. LwaCas13 is currently the Cas 13 of choice for single-plex detection. The addition of polyethylene glycol, molecular weight 20K (PEG 20K) and ATP co-factor increase the signal to noise ratio (Fig. 13). Single-strand binding protein T4 gp32 or thermophilic SSB (ET-SSB) are currently preferred additives in optimizing the protocol. dCpf1, UvsX Recombinase can be used as assisting proteins for complex templates. Varying concentrations of T4 gp32 and helicase were tested to determine what amounts of each per reaction performed best. As shown in Figure 12, while several concentrations worked well, 500 ng of T4 gp32 and 125 ng of helicase per reaction performed best. Additives were also explored for optimizing signal to background ratios (Fig. 13). Addition of PEG 20K as a crowing agent and ATP as a co-factor increased signal to background ratio, with 2.5% to 6.25% PEG 20K Molecular weight enhancing fluorescence signal, and 0.75 mM to 3 mM ATP also enhancing fluorescence.

### Example 3:One-Pot mHDA SHERLOCK

Particularly advantageous amplification methods would include one or more of the following variables, 37C operation, fast (~10 minutes), sensitive (~2 aM), inexpensive, multiplexable, easy to perform in one-pot process, and robust to sample matrices.

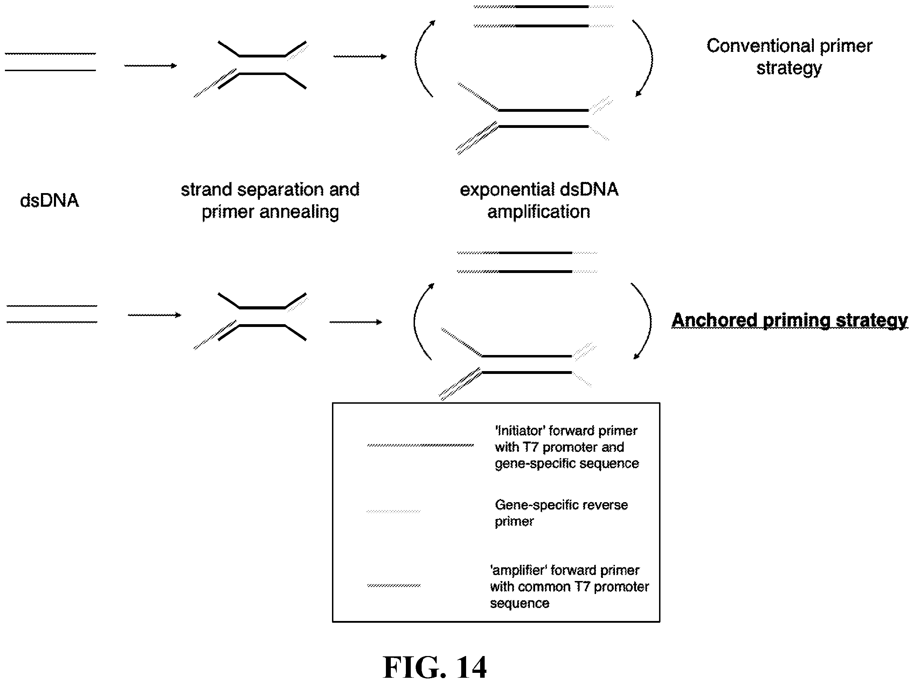

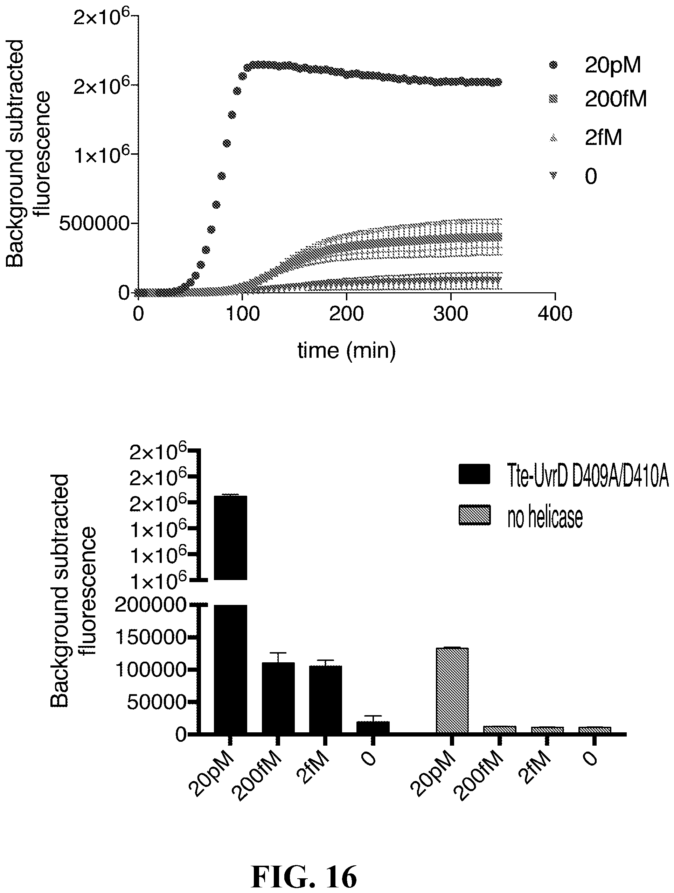

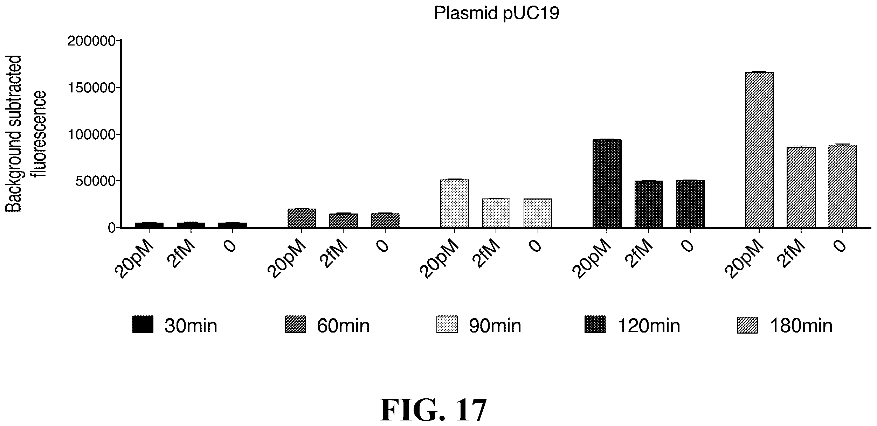

As part of optimization of mesophilic HDA (mHDA) methods, primer strategy was also optimized. It is believed a length of potentially 24 - 33 nt is optimal, but in current applications, the forward primer is much longer due to T7 promoter sequence for transcription. As presently disclosed, one way to circumvent the longer forward primer is the addition of a smaller T7 promoter priming together with longer HDA primer in a process termed "anchored priming," shown in Figure 14. By using a longer 'initiator' primer with gene specific sites that mediate the first cycle, and using smaller 'amplifier' primer for optimal amplification, primer competition may be prevented during multiplex DNA amplification when extended to the reverse priming site. Based on the approach, 'anchored priming' increased amplification efficiency, allowing one-pot mHDA-SHERLOCK to achieve 2fM (1000 copies) in less than two hours (Figure 16). As shown in Figure 17, this has also been accomplished on plasmid DNA.

## Claims

1. A method of amplifying and/or detecting a target double-stranded nucleic acid, comprising:
(a) combining a sample comprising the target double-stranded nucleic acid with an amplification reaction mixture, the amplification reaction mixture comprising:
(i) an amplification CRISPR system, the amplification CRISPR system comprising a first and second CRISPR/Cas complex, the first CRISPR/Cas complex comprising a first CRISPR/Cas enzyme and a first guide molecule that guides the first CRISPR/Cas complex to a first strand of the target nucleic acid, and the second CRISPR/Cas complex comprising a second CRISPR/Cas enzyme and a second guide molecule that guides the second CRISPR/Cas complex to a second strand of the target nucleic acid;
(ii) a helicase;
(iii) a primer pair comprising a first and second primer, wherein the first primer comprises a portion that is complementary to the first strand of the target nucleic acid and the second primer comprises a portion that is complementary to the second strand of the target nucleic acid; and
(iv) a polymerase;
(b) amplifying the target nucleic acid by opening R-loops of the target nucleic acid using the first and second CRISPR/Cas complexes, unwinding the first and the second strand of the target nucleic acid using the helicase, annealing and extending the strands using the primer pair and the polymerase; and
(c) further amplifying the target nucleic acid by repeated opening, unwinding, annealing and extension under isothermal conditions; and
(d) optionally detecting the amplified target nucleic acid.

2. The method of claim 1, wherein the CRISPR/Cas enzyme is a dead CRISPR/Cas enzyme selected from the group consisting of dead Cas9, dead Cas12a, dead Cas12b, and dead Cas12c, optionally wherein the dead CRISPR/Cas enzyme is a dead Cas9 protein which comprises a mutation in the HNH and RuvC domain, or wherein the dead CRISPR/Cas enzyme is a dead Cas9 protein which comprises a mutation corresponding to D10A and N863A in SpCas9, or D10A and H840A in SpCas9, optionally wherein the dead CRISPR/Cas enzyme is a dead Cas9 protein derived from a bacterial species selected from the group consisting of *Streptococcus pyogenes, Staphylococcus aureus, Streptococcus thermophilus, S. mutans, S. agalactiae, S. equisimilis, S. sanguinis, S. pneumonia; C. jejuni, C. coli; lV. salsuginis, lV. tergarcus; S. auricularis, S. carnosus; N. meningitides, N. gonorrhoeae; L. monocytogenes, L. ivanovii; C. botulinum, C. difficile, C. tetani, C. sordellii, Francisella tularensis 1, Prevotella albensis, Lachnospiraceae bacterium MC2017 1, Butyrivibrio proteoclasticus, Peregrinibacteria bacterium GW2011_GWA2_33_10, Parcubacteria bacterium GW2011_GWC2_44_17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens and Porphyromonas macacae,* or wherein the dead CRISPR/Cas enzyme is a dead Cas12 protein, optionally wherein the dead Cas12 protein comprises a mutation in the RuvC domain, optionally wherein the dead Cas12 enzyme is a dead Cas12a, Cas12b, Cas12c, optionally wherein the dead Cas12a comprises a mutation corresponding to D908A or E993A in AsCpf1, optionally wherein the dead Cas12 protein is derived from a bacterial species selected from the group consisting of *Francisella tularensis, Prevotella albensis, Lachnospiraceae bacterium, Butyrivibrio proteoclasticus, Peregrinibacteria bacterium, Parcubacteria bacterium, Smithella sp., Acidaminococcus sp., Lachnospiraceae bacterium, Candidatus Methanoplasma termitum, Eubacterium eligens, Moraxella bovoculi, Leptospira inadai, Porphyromonas crevioricanis, Prevotella disiens and Porphyromonas macacae, Succinivibrio dextrinosolvens, Prevotella disiens, Flavobacterium branchiophilum, Helcococcus kunzii, Eubacterium sp., Microgenomates (Roizmanbacteria) bacterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis and Proteocatella sphenisci,* or wherein the dead Cas12 is a dead Cas12b protein which comprises a mutation in the Nuc domain, optionally wherein the dead Cas12b comprises a mutation corresponding to D570A, E848A, or D977A in AacC2c1, optionally wherein the dead Cas12b protein is derived from a bacterial species selected from the group consisting of *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia bacterium RIFOXYA12, Omnitrophica WOR_2 bacterium RIFCSPHIGHO2, Opitutaceae bacterium TAV5, Phycisphaerae bacterium ST-NAGAB-D1, Planctomycetes bacterium RBG_13_46_10, Spirochaetes bacterium GWB1_27_13, Verrucomicrobiaceae bacterium UBA2429, Tuberibacillus calidus, Bacillus thermoamylovorans, Breuibacillus sp. CF112, Bacillus sp. NSP2.1, Desulfatirhabdium butyrativorans, Alicyclobacillus herbarius, Citrobacter freundii, Brevibacillus agri (e.g., BAB-2500), and Methylobacterium nodulans.*

3. The method of any of the preceding claims, wherein the first dead CRISPR/Cas enzyme and the second dead CRISPR/Cas enzyme are the same, or wherein the first dead CRISPR/Cas enzyme and the second dead CRISPR/Cas enzyme are different, optionally wherein the dead CRISPR/Cas enzyme is a dCas9, dCas12a, dCas12b, dCas12c, or dCas14, optionally wherein the polymerase is selected from the group consisting of *Bst2.0* DNA polymerase, *Bst2.0* WarmStart DNA polymerase, *Bst* 3.0 DNA polymerase, full length *Bst* DNA polymerase, large fragment *Bst* DNA polymerase, large fragment *Bsu* DNA polymerase, phi29 DNA polymerase, T7 DNA polymerase, Gst polymerase, Taq polymerase, Klenow fragment of E. coli DNA polymerase I, KlenTaq DNA polymerase, Pol III DNA polymerase, T5 DNA polymerase, and Sequenase DNA polymerase, optionally wherein the helicase is selected from the group consisting of UvrD helicase, CRISPR-Cas3 helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase.

4. The method of any of the preceding claims, wherein amplification of the target nucleic acid is performed at about 37°C-65°C, at about 50°C-59°C, at about 60°C-72°C, or at room temperature.

5. The method of claim 1, performed under mesophilic isothermal conditions, optionally wherein the polymerase is a mesophilic polymerase, optionally wherein the mesophilic polymerase is Sau LF Polymerase, optionally wherein amplification of the target nucleic acid is performed at about 37°C, optionally wherein the helicase is a E. Coli UvrD helicase, optionally with two or more substitutions of D to A, for example at D403 and D404, or wherein the helicase is a pcrA (UvrD)-type helicase comprising one or more mutations corresponding to D409A and D410A, optionally wherein the helicase is Tte-UvrD D409A/D410A.

6. The method of any of the preceding claims, wherein the first or the second primer comprises an RNA polymerase promoter.

7. The method of any of the preceding claims, comprising one or more additives selected from a crowding agent, a co-factor, single-strand binding protein, and/or assisting proteins, optionally wherein the crowding agent is polyethylene glycol, optionally wherein the co-factor is ATP, optionally wherein the single-strand binding protein is selected from T4 gp32 or thermophilic SSB (ET-SSB), optionally wherein the assisting protein is dCpf1 and/or UvsX Recombinase.

8. The method of any of the preceding claims, further comprising detecting the amplified nucleic acid by a method selected from the group consisting of gel electrophoresis, intercalating dye detection, PCR, real-time PCR, fluorescence, Fluorescence Resonance Energy Transfer (FRET), mass spectrometry, lateral flow assay, colorimetric assays, and CRISPR-based detection system, optionally wherein the amplified target nucleic acid is detected by a CRISPR Cas13-based system, a CRISPR Cas12-based system, or a combination thereof, optionally wherein the amplified target nucleic acid is detected by a CRISPR Cas-13 based system comprising LwaCas13 enzyme.

9. The method of any of the preceding claims, wherein the target nucleic acid is selected from the group consisting of genomic DNA, mitochondrial DNA, viral DNA, plasmid DNA, circulating cell free DNA, environmental DNA, synthetic double-stranded DNA, and RNA, optionally wherein the target nucleic acid is RNA, and wherein the RNA is reverse transcribed into cDNA prior to amplification, optionally wherein the amplification is recombinase polymerase amplification (RPA).

10. The method of any of the preceding claims, wherein the sample is a biological sample or an environmental sample, optionally wherein the biological sample is a blood, plasma, serum, urine, stool, sputum, mucous, lymph fluid, synovial fluid, bile, ascites, pleural effusion, seroma, saliva, cerebrospinal fluid, aqueous or vitreous humor sample, or any bodily secretion, a transudate, an exudate, or fluid obtained from a joint, or a swab of skin or mucosal membrane surface, preferably wherein the sample is blood, plasma or serum obtained from a human patient, or wherein the sample is a plant sample, optionally wherein the sample is a crude sample, or wherein the sample is a purified sample.

11. A method for amplifying and/or detecting a target single-stranded nucleic acid, comprising:
(a) converting the single-stranded nucleic acid in a sample to a target double-stranded nucleic acid; and
(b) performing the steps of claim 1, optionally wherein the target single-stranded nucleic acid is an RNA molecule, optionally wherein the RNA molecule is converted to the double-stranded nucleic acid by a reverse-transcription and amplification step, optionally wherein the target single-stranded nucleic acid is selected from the group consisting of single-stranded viral DNA, viral RNA, messenger RNA, ribosomal RNA, transfer RNA, microRNA, short interfering RNA, small nuclear RNA, synthetic RNA, synthetic single-stranded DNA, long non-coding RNA, pre-microRNA, viral dsRNA, and non-viral dsRNA.

12. The method of claim 1 further comprising an amplifier primer that comprises a portion contained in the first primer of the primer pair; and comprising the steps of the first primer of the primer pair mediates a first step of amplifying, and the amplifier primer together with the second primer of the primer pair mediating the subsequent steps of amplifying, optionally wherein the amplifier primer and the first primer of the primer pair each comprise a T7 promoter sequence, optionally wherein the amplifier primer comprises a length of about 23 to about 35 nucleotides.

13. A system for amplifying and/or detecting a target double-stranded nucleic acid in a sample, the system comprising:
a) an amplification CRISPR system, the amplification CRISPR system comprising a first and second CRISPR/Cas complex, the first CRISPR/Cas complex comprising a first CRISPR/Cas enzyme and a first guide molecule that guides the first CRISPR/Cas complex to a first strand of the target nucleic acid, and the second CRISPR/Cas complex comprising a second CRISPR/Cas enzyme and a second guide molecule that guides the second CRISPR/Cas complex to a second strand of the target nucleic acid;
b) a helicase;
c) a primer pair comprising a first and second primer, wherein the first primer comprising a portion that is complementary to the first strand of the target nucleic acid and the second primer comprising a portion that is complementary to the second strand of the target nucleic acid;
d) a polymerase; and optionally
e) a detection system for detecting amplification of the target nucleic acid;
optionally wherein the CRISPR/Cas enzyme is a dead CRISPR/Cas enzyme selected from a dead Cas9 enzyme or a dead Cas12 enzyme, optionally wherein the dead Cas12 enzyme is a dead Cas12a, Cas12b, or Cas12c enzyme, or wherein the CRISPR/Cas enzyme is a Cas9 or Cas12 enzyme, optionally wherein the Cas12 enzyme is a Cas12a, Cas12b, or Cas12c enzyme, optionally wherein the polymerase is selected from the group consisting of *Bst* 2.0 DNA polymerase, *Bst* 2.0 WarmStart DNA polymerase, *Bst3.0* DNA polymerase, full length *Bst* DNA polymerase, large fragment *Bst DNA* polymerase, large fragment *Bsu* DNA polymerase, phi29 DNA polymerase, T7 DNA polymerase, Gst polymerase, Taq polymerase, Klenow fragment of E. coli DNA polymerase I, KlenTaq NDA polymerase, Pol III DNA polymerase, T5 DNA polymerase, and Sequenase DNA polymerase, and Sau LF DNA polymerase, optionally wherein the helicase is selected from the group consisting of UvrD helicase, CRISPR-Cas3 helicase, Rep helicase, PcrA helicase, E. coli helicase I, E. coli helicase II, E. coli helicase III, E. coli helicase IV, Rep helicase, DnaB helicase, PriA helicase, PcrA helicase, T4 Gp41 helicase, T4 Dda helicase, SV40 Large T antigen, yeast RAD helicase, RecD helicase, RecQ helicase, thermostable T. tengcongensis UvrD helicase, thermostable T. thermophilus UvrD helicase, thermostable T. aquaticus DnaB helicase, Dda helicase, papilloma virus E1 helicase, archaeal MCM helicase, eukaryotic MCM helicase, and T7 Gp4 helicase, optionally wherein the helicase is Tte-UvrD D409A/D410A,
optionally further comprising one or more additives selected from a crowding agent, a co-factor, single-strand binding protein, and/or assisting proteins, optionally wherein the crowding agent is polyethylene glycol 20K molecular weight, optionally provided at about 2.5% to about 6.5%, optionally wherein the co-factor is ATP, optionally provided at 0.75 to about 5 mM, optionally wherein the single-strand binding protein is selected from T4 gp32 or thermophilic SSB (ET-SSB), optionally wherein the assisting protein is dCpf1 and/or UvsX Recombinase, optionally wherein the CRISPR/Cas enzyme, the helicase, and the polymerase perform under the same temperature, in some instances, at about 37 °C,
optionally wherein the detection system comprises a CRISPR system comprising a Cas 12 or Cas 13 effector protein and a guide RNA designed to bind to a corresponding target molecule; and a masking construct, optionally wherein the detection system comprises Cas 13 enzyme LwaCas13,
optionally further comprising an additional amplifier primer comprising a promoter sequence identity with the first primer of the primer pair.

14. A system for amplifying and/or detecting a target single-stranded nucleic acid in a sample, the system comprising:
a) reagents for converting the target single-stranded nucleic acid to a double-stranded nucleic acid;
b) components of claim 13;
optionally wherein the detection system comprises a CRISPR system comprising a Cas 12 or Cas 13 effector protein and a guide RNA designed to bind to a corresponding target molecule; and a masking construct, optionally wherein the detection system comprises Cas 13 enzyme LwaCas13, optionally further comprising reagents for purifying the single-stranded nucleic acid in the sample.

15. The method of claim 1 or the system of claim 13, wherein the helicase is PcrA helicase from Thermoanaerobacter ethanolicus with mutations D403A/D404A, PcrA helicase from Bacillus sp. FJAT-27231 with mutations D407A/D408A, PcrA helicase from Bacillus megaterium with mutations D415A/D416A, PcrA helicase from Bacillus simplex with mutations D407A/D408A, or PcrA helicase from Paeniclostridium sordellii with mutations D402A/D403A.

## Patentansprüche

1. Verfahren zum Amplifizieren und/oder Nachweisen einer doppelsträngigen Zielnukleinsäure, umfassend:
(a) Kombinieren einer die doppelsträngige Zielnukleinsäure umfassenden Probe mit einem Amplifikationsreaktionsgemisch, wobei der Amplifikationsreaktionsgemisch Folgendes umfasst:
(i) ein CRISPR-Amplifikationssystem, wobei das CRISPR-Amplifikationssystem einen ersten und zweiten CRISPR/Cas-Komplex umfasst, wobei der erste CRISPR/Cas-Komplex ein erstes CRISPR/Cas-Enzym und ein erstes Guide-Molekül, das den ersten CRISPR/Cas-Komplex an einen ersten Strang der Zielnukleinsäure führt, umfasst und der zweite CRISPR/Cas-Komplex ein zweites CRISPR/Cas-Enzym und ein zweites Guide-Molekül, das den zweiten CRISPR/Cas-Komplex an einen zweiten Strang der Zielnukleinsäure führt, umfasst;
(ii) eine Helicase;
(iii) ein Primerpaar, das einen ersten und zweiten Primer umfasst, wobei der erste Primer einen Teil umfasst, der zum ersten Strang der Zielnukleinsäure komplementär ist, und der zweite Primer einen Teil umfasst, der zum zweiten Strang der Zielnukleinsäure komplementär ist; und
(iv) eine Polymerase;
(b) Amplifizieren der Zielnukleinsäure durch Öffnen von R-Loops der Zielnukleinsäure unter Verwendung des ersten und des zweiten CRISPR/Cas-Komplexes, Auseinanderwickeln des ersten und des zweiten Strangs der Zielnukleinsäure unter Verwendung der Helicase, Annealing und Verlängern der Stränge unter Verwendung des Primerpaars und der Polymerase; und
(c) weiteres Amplifizieren der Zielnukleinsäure durch wiederholtes Öffnen, Auseinanderwickeln, Annealing und Verlängern unter isothermen Bedingungen; und
(d) gegebenenfalls Nachweisen der amplifizierten Zielnukleinsäure.

2. Verfahren nach Anspruch 1, wobei es sich bei dem CRISPR/Cas-Enzym um ein d(dead)-CRISPR/Cas-Enzym ausgewählt aus der Gruppe bestehend aus d-Cas9, d-Cas12a, d-Cas12b und d-Cas12c handelt, gegebenenfalls wobei es sich bei dem d-CRISPR/Cas-Enzym um ein d-Cas9-Protein handelt, das eine Mutation in der HNH- und RuvC-Domäne umfasst, oder wobei es sich bei dem d-CRISPR/Cas-Enzym um ein d-Cas9-Protein handelt, das eine Mutation umfasst, die D10A und N863A in SpCas9 oder D10A und H840A in SpCas9 entspricht, gegebenenfalls wobei es sich bei dem d-CRISPR/Cas-Enzym um ein d-Cas9-Protein handelt, das von einer Bakterienspezies ausgewählt aus der Gruppe bestehend aus *Streptococcus pyogenes, Staphylococcus aureus, Streptococcus thermophilus, S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii, Francisella tularensis 1, Prevotella albensis, Lachnospiraceae Bakterium MC2017 1, Butyrivibrio proteoclasticus, Peregrinibacteria-*Bakterium *GW2011_GWA2_33_10, Parcubacteria-Bakterium GW2011*_*GWC2_44*_*17, Smithella sp. SCADC, Acidaminococcus sp. BV3L6, Lachnospiraceae-Bakterium MA2020, Candidatus Methanoplasma termitum, Eubacterium eligens, Moraxella bovoculi 237, Leptospira inadai, Lachnospiraceae-*Bakterium *ND2006, Porphyromonas crevioricanis 3, Prevotella disiens* und *Porphyromonas macacae* stammt, oder wobei es sich bei dem d-CRISPR/Cas-Enzym um ein d-Cas12-Protein handelt, gegebenenfalls wobei das d-Cas12-Protein eine Mutation in der RuvC-Domäne umfasst, gegebenenfalls wobei es sich bei dem d-Cas12-Enzym um ein d-Cas12a, d-Cas12b, d-Cas12c handelt, gegebenenfalls wobei das d-Cas12a eine Mutation umfasst, die D908A oder E993A in AsCpf1 entspricht, gegebenenfalls wobei das d-Cas12-Protein von einer Bakterienspezies ausgewählt aus der Gruppe bestehend aus *Francisella tularensis, Prevotella albensis, Lachnospiraceae-*Bakterium, *Butyrivibrio proteoclasticus, Peregrinibacteria-Bakterium, Parcubacteria-Bakterium, Smithella sp., Acidaminococcus sp., Lachnospiraceae-*Bakterium, *Candidatus Methanoplasma termitum, Eubacterium eligens, Moraxella bovoculi, Leptospira inadai, Porphyromonas crevioricanis, Prevotella disiens* und *Porphyromonas macacae, Succinivibrio dextrinosolvens, Prevotella disiens, Flavobacterium branchiophilum, Helcococcus kunzii, Eubacterium sp., Microgenomates(Roizmanbacteria) -Bakterium, Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp., Pseudobutyrivibrio ruminis* und *Proteocatella sphenisci* stammt, oder wobei es sich bei dem d-Cas12 um ein d-Cas12b-Protein handelt, das eine Mutation in der Nuc-Domäne umfasst, gegebenenfalls wobei das d-Cas12b eine Mutation umfasst, die D570A, E848A oder D977A in AacC2c1 entspricht, gegebenenfalls wobei das d-Cas12b-Protein von einer Bakterienspezies ausgewählt aus der Gruppe bestehend aus *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, Elusimicrobia-*Bakterium *RIFOXYA12, Omnitrophica WOR_2*-Bakterium *RIFCSPHIGHO2, Opitutaceae*-Bakterium *TAV5, Phycisphaerae*-Bakterium *ST-NAGAB-D1, Planctomycetes*-Bakterium *RBG_13_46_10, Spirochaetes*-Bakterium *GWB1_27_13, Verrucomicrobiaceae-*Bakterium *UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri* (z. B. *BAB-2500)* und *Methylobacterium nodulans* stammt.

3. Verfahren nach einem der vorhergehenden Ansprüche, wobei das erste d-CRISPR/Cas-Enzym und das zweite d-CRISPR/Cas-Enzym identisch sind oder wobei das erste d-CRISPR/Cas-Enzym und das zweite d-CRISPR/Cas-Enzym verschieden sind, gegebenenfalls wobei es sich bei dem d-CRISPR/Cas-Enzym um ein dCas9, dCas 12 a, dCas12b, dCas12c oder dCas14 handelt, gegebenenfalls wobei die Polymerase ausgewählt ist aus der Gruppe bestehend aus *Bst* 2.0-DNA-Polymerase, *Bst* 2.0 WarmStart-DNA-Polymerase, *Bst* 3.0-DNA-Polymerase, Volllängen-Bst-DNA-Polymerase, *Bst*-DNA-Polymerase großes Fragment, *Bsu*-DNA-Polymerase großes Fragment, phi29-DNA-Polymerase, T7-DNA-Polymerase, Gst-Polymerase, Taq-Polymerase, Klenow-Fragment von E. coli-DNA-Polymerase I, KlenTaq-DNA-Polymerase, Pol III-DNA-Polymerase, T5-DNA-Polymerase und Sequenase-DNA-Polymerase, gegebenenfalls wobei die Helicase ausgewählt ist aus der Gruppe bestehend aus UvrD-Helicase, CRISPR-CasS-Helicase, E. coli-Helicase I, E. coli-Helicase II, E. coli-Helicase III, E. coli-Helicase IV, Rep-Helicase, DnaB-Helicase, PriA-Helicase, PcrA-Helicase, T4-Gp41-Helicase, T4-Dda-Helicase, SV40-Large-T-Antigen, Hefe-RAD-Helicase, RecD-Helicase, RecQ-Helicase, thermostabiler T. tengcongensis-UvrD-Helicase, thermostabiler T. thermophilus-UvrD-Helicase, thermostabiler T. aquaticus-DnaB-Helicase, Dda-Helicase, Papillomavirus-E1-Helicase, Archaeen-MCM-Helicase, eukaryontischer MCM-Helicase und T7-Gp4-Helicase.

4. Verfahren nach einem der vorhergehenden Ansprüche, wobei die Amplifikation der Zielnukleinsäure bei etwa 37°C-65°C, bei etwa 50°C-59°C, bei etwa 60°C-72°C oder bei Raumtemperatur erfolgt.

5. Verfahren nach Anspruch 1, durchgeführt unter mesophilen isothermen Bedingungen, gegebenenfalls wobei es sich bei der Polymerase um eine mesophile Polymerase handelt, gegebenenfalls wobei es sich bei der mesophilen Polymerase um Sau LF-Polymerase handelt, gegebenenfalls wobei die Amplifikation der Zielnukleinsäure bei etwa 37°C erfolgt, gegebenenfalls wobei es sich bei der Helicase um eine E. coli-UvrD-Helicase, gegebenenfalls mit zwei oder mehr Substitutionen von D zu A, beispielsweise an D403 und D404, handelt oder wobei es sich bei der Helicase um eine pcrA(UvrD)-Typ-Helicase handelt, die eine oder mehrere Mutationen, die D409A und D410A entsprechen, umfasst, handelt, gegebenenfalls wobei es sich bei der Helicase um Tte-UvrD-D409A/D410A handelt.

6. Verfahren nach einem der vorhergehenden Ansprüche, wobei der erste und der zweite Primer einen RNA-Polymerase-Promotor umfasst.

7. Verfahren nach einem der vorhergehenden Ansprüche, umfassend einen oder mehrere Zusätze ausgewählt aus einem Volumenausschlussmittel, einem Cofaktor, einem Einzelstrang-Bindungsprotein und/oder Hilfsproteinen, gegebenenfalls wobei es sich bei dem Volumenausschlussmittel um Polyethylenglykol handelt, gegebenenfalls wobei es sich bei dem Cofaktor um ATP handelt, gegebenenfalls wobei das Einzelstrang-Bindungsprotein ausgewählt ist aus T4-gp32 oder thermophilem SSB (ET-SSB), gegebenenfalls wobei es sich bei dem Hilfsprotein um dCpf1 und/oder UvsX Recombinase handelt.

8. Verfahren nach einem der vorhergehenden Ansprüche, ferner umfassend Nachweisen der amplifizierten Nukleinsäure mit einer Methode ausgewählt aus der Gruppe bestehend aus Gelelektrophorese, Nachweis mit interkalierenden Farbstoffen, PCR, Echtzeit-PCR, Fluoreszenz, FRET (Fluorescence Resonance Energy Transfer), Massenspektrometrie, Lateral-Flow-Testverfahren, kolorimetrischen Testverfahren und einem auf CRISPR beruhenden Nachweissystem, gegebenenfalls wobei die amplifizierte Zielnukleinsäure mit einem auf CRISPR-Cas13 beruhenden System, einem auf CRISPR-Cas12 beruhenden System oder einer Kombination nachgewiesen wird, gegebenenfalls wobei die amplifizierte Zielnukleinsäure mit einem auf CRISPR-Cas13 beruhenden System, das LwaCas13-Enzym umfasst, nachgewiesen wird.

9. Verfahren nach einem der vorhergehenden Ansprüche, wobei die Zielnukleinsäure ausgewählt ist aus der Gruppe bestehend aus genomischer DNA, mitochondrialer DNA, viraler DNA, Plasmid-DNA, zellfreier DNA im Kreislauf, DNA in der Umwelt, synthetischer Doppelstrang-DNA und RNA, gegebenenfalls wobei es sich bei der Zielnukleinsäure um RNA handelt und wobei die RNA vor der Amplifikation in cDNA revers-transkribiert wird, gegebenenfalls wobei es sich bei der Amplifikation um Rekombinase-Polymerase-Amplifikation (RPA) handelt.

10. Verfahren nach einem der vorhergehenden Ansprüche, wobei es sich bei der Probe um eine biologische Probe oder eine Umweltprobe handelt, gegebenenfalls wobei es sich bei der biologischen Probe um eine Blut-, Plasma-, Serum-, Urin-, Stuhl-, Sputum-, Schleim-, Lymphflüssigkeits-, Gelenkflüssigkeits-, Gallen-, Aszites-, Pleuraerguss-, Serom-, Speichel-, Liquor-, Kammerwasseroder Glaskörperprobe oder ein beliebiges Körpersekret, ein Transudat, ein Exudat oder aus einem Gelenk erhaltene Flüssigkeit oder einen Abstrich von Haut- oder Schleimhautoberfläche handelt, bevorzugt wobei es sich bei der Probe um von einem menschlichen Patienten erhaltenes Blut, Plasma oder Serum handelt, oder wobei es sich bei der Probe um eine Pflanzenprobe handelt, gegebenenfalls wobei es sich bei der Probe um eine Rohprobe handelt oder wobei es sich bei der Probe um eine aufgereinigte Probe handelt.

11. Verfahren zum Amplifizieren und/oder Nachweisen einer einzelsträngigen Zielnukleinsäure, umfassend:
(a) Überführen der einzelsträngigen Nukleinsäure in einer Probe in eine doppelsträngige Zielnukleinsäure; und
(b) Durchführen der Schritte unter Anspruch 1, gegebenenfalls wobei es sich bei der einzelsträngigen Zeilnukleinsäure um ein RNA-Molekül handelt, gegebenenfalls wobei das RNA-Molekül mit einem Schritt von reverser Transkription und Amplifikation in die doppelsträngige Nukleinsäure überführt wird, gegebenenfalls wobei die einzelsträngige Zielnukleinsäure ausgewählt ist aus der Gruppe bestehend aus viraler Einzelstrang-DNA, viraler RNA, mRNA (messenger RNA), ribosomaler RNA, tRNA (transfer RNA), microRNA, siRNA (short interfering RNA), snRNA (small nuclear RNA), synthetischer RNA, synthetischer Einzelstrang-DNA, langer nicht codierender RNA, pre-microRNA, viraler dsRNA und nichtviraler dsRNA.

12. Verfahren nach Anspruch 1, ferner umfassend einen Amplifikatorprimer, der einen im ersten Primer des Primerpaars enthaltenen Teil umfasst; und umfassend die Schritte, bei denen der erste Primer des Primerpaars einen ersten Amplifizierschritt vermittelt und der Amplifikatorprimer zusammen mit dem zweiten Primer des Primerpaars die nachfolgenden Amplifizierschritte vermittelt, gegebenenfalls wobei der Amplifikatorprimer und der erste Primer des Primerpaars jeweils eine T7-Promotorsequenz umfassen, gegebenenfalls wobei der Amplifikatorprimer eine Länge von etwa 23 bis etwa 35 Nukleotiden umfasst.

13. System zum Amplifizieren und/oder Nachweisen einer doppelsträngigen Zielnukleinsäure in einer Probe, wobei das System Folgendes umfasst:
a) ein CRISPR-Amplifikationssystem, wobei das CRISPR-Amplifikationssystem einen ersten und zweiten CRISPR/Cas-Komplex umfasst, wobei der erste CRISPR/Cas-Komplex ein erstes CRISPR/Cas-Enzym und ein erstes Guide-Molekül, das den ersten CRISPR/Cas-Komplex an einen ersten Strang der Zielnukleinsäure führt, umfasst und der zweite CRISPR/Cas-Komplex ein zweites CRISPR/Cas-Enzym und ein zweites Guide-Molekül, das den zweiten CRISPR/Cas-Komplex an einen zweiten Strang der Zielnukleinsäure führt, umfasst;
b) eine Helicase;
c) ein Primerpaar, das einen ersten und zweiten Primer umfasst, wobei der erste Primer einen Teil umfasst, der zum ersten Strang der Zielnukleinsäure komplementär ist, und der zweite Primer einen Teil umfasst, der zum zweiten Strang der Zielnukleinsäure komplementär ist;
d) eine Polymerase; und gegebenenfalls
e) ein Nachweissystem zum Nachweisen der Amplifikation der Zielnukleinsäure;
gegebenenfalls wobei es sich bei dem CRISPR/Cas-Enzym um ein d(dead)-CRISPR/Cas-Enzym ausgewählt aus einem d-Cas9-Enzym oder einem d-Cas12-Enzym handelt, gegebenenfalls wobei es sich bei dem d-Cas12-Enzym um ein d-Cas 12 a -, d-Cas12b- oder d-Cas12c-Enzym handelt, oder wobei es sich bei dem CRISPR/Cas-Enzym um ein Cas9- oder Cas12-Enzym handelt, gegebenenfalls wobei es sich bei dem Cas12-Enzym um ein Cas 12 a -, Cas12b- oder Cas 12 c- Enzym handelt, gegebenenfalls wobei die Polymerase ausgewählt ist aus der Gruppe bestehend aus *Bst 2.0-*DNA-Polymerase, *Bst* 2.0 WarmStart-DNA-Polymerase, *Bst* 3.0-DNA-Polymerase, Volllängen-*Bst*-DNA-Polymerase, *Bst*-DNA-Polymerase großes Fragment, *Bsu*-DNA-Polymerase großes Fragment, phi29-DNA-Polymerase, T7-DNA-Polymerase, Gst-Polymerase, Taq-Polymerase, Klenow-Fragment von E. coli-DNA-Polymerase I, KlenTaq-DNA-Polymerase, Pol III-DNA-Polymerase, T5-DNA-Polymerase und Sequenase-DNA-Polymerase und Sau LF-DNA-Polymerase, gegebenenfalls wobei die Helicase ausgewählt ist aus der Gruppe bestehend aus UvrD-Helicase, CRISPR-Cas3-Helicase, Rep-Helicase, PcrA-Helicase, E. coli-Helicase I, E. coli-Helicase II, E. coli-Helicase III, E. coli-Helicase IV, Rep-Helicase, DnaB-Helicase, PriA-Helicase, PcrA-Helicase, T4-Gp41-Helicase, T4-Dda-Helicase, SV40-Large-T-Antigen, Hefe-RAD-Helicase, RecD-Helicase, RecQ-Helicase, thermostabiler T. tengcongensis-UvrD-Helicase, thermostabiler T. thermophilus-UvrD-Helicase, thermostabiler T. aquaticus-DnaB-Helicase, Dda-Helicase, Papillomavirus-E1-Helicase, Archaeen-MCM-Helicase, eukaryontischer MCM-Helicase und T7-Gp4-Helicase, gegebenenfalls wobei es sich bei der Helicase um Tte-UvrD D409A/D410A handelt, gegebenenfalls ferner umfassend einen oder mehrere Zusätze ausgewählt aus einem Volumenausschlussmittel, einem Cofaktor, einem Einzelstrang-Bindungsprotein und/oder Hilfsproteinen, gegebenenfalls wobei es sich bei dem Volumenausschlussmittel um Polyethylenglykol mit Molekulargewicht 20K, gegebenenfalls bereitgestellt mit etwa 2,5% bis etwa 6,5%, handelt, gegebenenfalls wobei es sich bei dem Cofaktor um ATP, gegebenenfalls bereitgestellt mit 0,75 bis etwa 5 mM, handelt, gegebenenfalls wobei das Einzelstrang-Bindungsprotein ausgewählt ist aus T4-gp32 oder thermophilem SSB (ET-SSB), gegebenenfalls wobei es sich bei dem Hilfsprotein um dCpf1 und/oder UvsX Recombinase handelt, gegebenenfalls wobei das CRISPR/Cas-Enzym, die Helicase und die Polymerase unter der gleichen Temperatur, in einigen Fällen bei etwa 37 °C, arbeiten,
gegebenenfalls wobei das Nachweissystem ein CRISPR-System, das ein Cas 12 - oder Cas13-Effektorprotein und eine guide RNA, die zur Bindung an ein entsprechendes Zielmolekül vorgesehen ist, umfasst; und ein Maskierungskonstrukt umfasst, gegebenenfalls wobei das Nachweissystem Cas13-Enzym LwaCas13 umfasst,
gegebenenfalls ferner umfassend einen zusätzlichen Amplifikatorprimer, der eine Promotorsequenzidentität mit dem ersten Primer des Primerpaars umfasst.

14. System zum Amplifizieren und/oder Nachweisen einer einzelsträngigen Zielnukleinsäure in einer Probe, wobei das System Folgendes umfasst:
a) Reagentien zum Überführen der einzelsträngigen Zielnukleinsäure in eine doppelsträngige Nukleinsäure;
b) Komponenten unter Anspruch 13;
gegebenenfalls wobei das Nachweissystem ein CRISPR-System, das ein Cas 12 - oder Cas13-Effektorprotein und eine guide RNA, die zur Bindung an ein entsprechendes Zielmolekül vorgesehen ist, umfasst; und ein Maskierungskonstrukt umfasst, gegebenenfalls wobei das Nachweissystem Cas13-Enzym LwaCas13 umfasst, gegebenenfalls ferner umfassend Reagentien zum Aufreinigen der einzelsträngigen Nukleinsäure in der Probe.

15. Verfahren nach Anspruch 1 oder System nach Anspruch 13, wobei es sich bei der Helicase um PcrA-Helicase aus Thermoanaerobacter ethanolicus mit Mutationen D403A/D404A, PcrA-Helicase aus Bacillus sp. FJAT-27231 mit Mutationen D407A/D408A, PcrA-Helicase aus Bacillus megaterium mit Mutationen D415A/D416A, PcrA-Helicase aus Bacillus simplex mit Mutationen D407A/D408A oder PcrA-Helicase aus Paeniclostridium sordellii mit Mutationen D402A/D403A handelt.

## Revendications

1. Procédé d'amplification et/ou de détection d'un acide nucléique double brin cible, comprenant :
(a) le mélange d'un échantillon comprenant l'acide nucléique double brin cible avec un mélange réactionnel d'amplification, le mélange réactionnel d'amplification comprenant :
(i) un système CRISPR d'amplification, le système CRISPR d'amplification comprenant un premier et un deuxième complexe CRISPR/Cas, le premier complexe CRISPR/Cas comprenant une première enzyme CRISPR/Cas et une première molécule de guidage qui guide le premier complexe CRISPR/Cas vers un premier brin de l'acide nucléique cible, et le deuxième complexe CRISPR/Cas comprenant une deuxième enzyme CRISPR/Cas et une deuxième molécule de guidage qui guide le deuxième complexe CRISPR/Cas vers un deuxième brin de l'acide nucléique cible ;
(ii) une hélicase ;
(iii) une paire d'amorces comprenant une première et une deuxième amorce, dans laquelle la première amorce comprend une partie qui est complémentaire du premier brin de l'acide nucléique cible et la deuxième amorce comprend une partie qui est complémentaire du deuxième brin de l'acide nucléique cible ; et
(iv) une polymérase ;
(b) l'amplification de l'acide nucléique cible en ouvrant les boucles R de l'acide nucléique cible en utilisant les premier et deuxième complexes CRISPR/Cas, en déroulant le premier et le deuxième brin de l'acide nucléique cible en utilisant l'hélicase, en annelant et en étendant les brins en utilisant la paire d'amorces et la polymérase ; et
(c) l'amplification davantage de l'acide nucléique cible par ouverture, déroulement, annelage et extension répétés dans des conditions isothermes ; et
(d) éventuellement la détection de l'acide nucléique cible amplifié.

2. Procédé selon la revendication 1, dans lequel l'enzyme CRISPR/Cas est une enzyme CRISPR/Cas morte choisie dans le groupe constitué par Cas9 morte, Cas12a morte, Cas12b morte et Cas12c morte, éventuellement dans lequel l'enzyme CRISPR/Cas morte est une protéine Cas9 morte qui comprend une mutation dans le domaine HNH et RuvC, ou dans lequel l'enzyme CRISPR/Cas morte est une protéine Cas9 morte qui comprend une mutation correspondant à D10A et N863A dans SpCas9, ou D10A et H840A dans SpCas9, éventuellement dans laquelle l'enzyme CRISPR/Cas morte est une protéine Cas9 morte dérivée d'une espèce bactérienne choisie dans le groupe constitué par *Streptococcus pyogenes, Staphylococcus aureus, Streptococcus thermophilus, S. mutans, S. agalactiae, S. equisimilis, S. sanguinis, S. pneumonia ; C. jejuni, C. coli ; N. salsuginis, N. tergarcus ; S. auricularis, S. carnosus* ; *N. meningitides, N. gonorrhoeae* ; *L. monocytogenes, L. ivanovii ; C. botulinum, C. difficile, C. tetani, C. sordellii, Francisella tularensis 1, Prevotella albensis, bactérie Lachnospiraceae MC2017 1, Butyrivibrio proteoclasticus, bactérie Peregrinibacteria GW2011_GWA2_33_10, bactérie Parcubacteria GW2011_GWC2_44_17, Smithella sp. SCADC, Acidaminococcus sp. BV3L6, bactérie Lachnospiraceae MA2020, Candidatus Methanoplasma termitum, Eubacterium eligens, Moraxella bovoculi 237, Leptospira inadai, bactérie Lachnospiraceae ND2006, Porphyromonas crevioricanis 3, Prevotella disiens et Porphyromonas macacae,* ou dans laquelle l'enzyme CRISPR/Cas morte est une protéine Cas12 morte, éventuellement dans laquelle la protéine Cas12 morte comprend une mutation dans le domaine RuvC, éventuellement dans lequel l'enzyme Cas12 morte est une Cas12a, Cas12b, Cas12c morte, éventuellement dans lequel la Cas12a morte comprend une mutation correspondant à D908A ou E993A dans AsCpf1, éventuellement dans lequel la protéine Cas12 morte est dérivée d'une espèce bactérienne choisie dans le groupe constitué par *Francisella tularensis, Prevotella albensis, bactérie Lachnospiraceae, Butyrivibrio proteoclasticus, bactérie Peregrinibacteria, bactérie Parcubacteria, Smithella sp., Acidaminococcus sp., bactérie Lachnospiraceae, Candidatus Methanoplasma termitum, Eubacterium eligens, Moraxella bovoculi, Leptospira inadai, Porphyromonas crevioricanis, Prevotella disiens et Porphyromonas macacae, Succinivibrio dextrinosolvens, Prevotella disiens, Flavobacterium branchiophilum, Helcococcus kunzii, Eubacterium sp, bactérie Microgenomates (Roizmanbacteria)*, *Flavobacterium sp., Prevotella brevis, Moraxella caprae, Bacteroidetes oral, Porphyromonas cansulci, Synergistes jonesii, Prevotella bryantii, Anaerovibrio sp., Butyrivibrio fibrisolvens, Candidatus Methanomethylophilus, Butyrivibrio sp., Oribacterium sp, Pseudobutyrivibrio ruminis et Proteocatella sphenisci,* ou dans lequel la Cas12 morte est une protéine Cas12b morte qui comprend une mutation dans le domaine Nue, éventuellement dans lequel la Cas12b morte comprend une mutation correspondant à D570A, E848A, ou D977A dans AacC2c1, éventuellement dans lequel la protéine Cas12b morte est dérivée d'une espèce bactérienne choisie dans le groupe constitué par *Alicyclobacillus acidoterrestris, Alicyclobacillus contaminans, Alicyclobacillus macrosporangiidus, Bacillus hisashii, Candidatus Lindowbacteria, Desulfovibrio inopinatus, Desulfonatronum thiodismutans, bactérie Elusimicrobia RIFOXYA12, bactérie Omnitrophica WOR_2 RIFCSPHIGHO2*, *bactérie Opitutaceae TAV5, bactérie Phycisphaerae ST-NAGAB-D1, bactérie Planctomycetes RBG_13_46_10, bactérie Spirochaetes GWB1_27_13, bactérie Verrucomicrobiaceae UBA2429, Tuberibacillus calidus, Bacillus thermoamylovorans, Brevibacillus sp. CF112, Bacillus sp. NSP2.1, Desulfatirhabdium butyrativorans, Alicyclobacillus herbarius, Citrobacter freundii, Brevibacillus agri (par ex., BAB-2500),* et *Methylobacterium nodulans.*

3. Procédé selon l'une quelconque des revendications précédentes, dans lequel la première enzyme CRISPR/Cas morte et la deuxième enzyme CRISPR/Cas morte sont identiques, ou dans lequel la première enzyme CRISPR/Cas morte et la deuxième enzyme CRISPR/Cas morte sont différentes, éventuellement dans lequel l'enzyme CRISPR/Cas morte est une dCas9, dCas12a, dCas12b, dCas12c, ou dCas14, éventuellement dans lequel la polymérase est choisie dans le groupe constitué par l'ADN polymérase *Bst* 2.0, l'ADN polymérase *Bst* 2.0 WarmStart, l'ADN polymérase *Bst* 3.0, l'ADN polymérase *Bst* pleine longueur, l'ADN polymérase *Bst* grand fragment, l'ADN polymérase *Bsu* grand fragment, l'ADN polymérase phi29, l'ADN polymérase T7, la polymérase Gst, la polymérase Taq, le fragment Klenow de l'ADN polymérase I d'E. coli, l'ADN polymérase KlenTaq, l'ADN polymérase Pol III, l'ADN polymérase T5 et l'ADN polymérase Sequénase, éventuellement dans lequel l'hélicase est choisie dans le groupe constitué par l'hélicase UvrD, l'hélicase CRISPR-Cas3, l'hélicase I d'E. coli, l'hélicase II d'E. coli, l'hélicase III d'E. coli, l'hélicase IV d'E. coli, l'hélicase Rep, l'hélicase DnaB, l'hélicase PriA, l'hélicase PcrA, l'hélicase T4 Gp41, l'hélicase T4 Dda, le grand antigène T de SV40, l'hélicase de levure RAD, l'hélicase RecD, l'hélicase RecQ, l'hélicase thermostable T. tengcongensis UvrD, l'hélicase thermostable T. thermophilus UvrD, l'hélicase thermostable T. aquaticus DnaB, l'hélicase Dda, l'hélicase de papilloma virus E1, l'hélicase d'archée MCM, l'hélicase d'eucaryote MCM, et l'hélicase T7 Gp4.

4. Procédé selon l'une quelconque des revendications précédentes, dans lequel l'amplification de l'acide nucléique cible est réalisée à une température d'environ 37 °C à 65 °C, d'environ 50 °C à 59 °C, d'environ 60 °C à 72 °C, ou à température ambiante.

5. Procédé de la revendication 1, réalisé dans des conditions isothermes mésophiles, éventuellement dans lequel la polymérase est une polymérase mésophile, éventuellement dans lequel la polymérase mésophile est la polymérase Sau LF, éventuellement dans lequel l'amplification de l'acide nucléique cible est réalisée à environ 37°C, éventuellement dans lequel l'hélicase est une hélicase UvrD de E. Coli, éventuellement avec deux ou plusieurs substitutions de D en A, par exemple à D403 et D404, ou dans lequel l'hélicase est une hélicase de type pcrA (UvrD) comprenant une ou plusieurs mutations correspondant à D409A et D410A, éventuellement dans laquelle l'hélicase est Tte-UvrD D409A/D410A.

6. Procédé selon l'une quelconque des revendications précédentes, dans lequel la première ou la deuxième amorce comprend un promoteur d'ARN polymérase.

7. Procédé selon l'une quelconque des revendications précédentes, comprenant un ou plusieurs additifs choisis parmi un agent d'encombrement, un cofacteur, une protéine de liaison de simple brin, et/ou des protéines d'assistance, éventuellement dans lequel l'agent d'encombrement est un polyéthylène glycol, éventuellement dans lequel le cofacteur est l'ATP, éventuellement dans lequel la protéine de liaison de simple brin est choisie parmi la gp32 de T4 ou la SSB thermophile (ET-SSB), éventuellement dans lequel la protéine d'assistance est la dCpf1 et/ou la recombinase UvsX.

8. Procédé selon l'une quelconque des revendications précédentes, comprenant en outre la détection de l'acide nucléique amplifié par un procédé choisi dans le groupe constitué par l'électrophorèse sur gel, la détection par colorant d'intercalation, la PCR, la PCR en temps réel, la fluorescence, le transfert d'énergie par résonance de fluorescence (FRET), la spectrométrie de masse, l'essay à flux latéral, des essais colorimétriques, et un système de détection basé sur CRISPR, éventuellement dans lequel l'acide nucléique cible amplifié est détecté par un système basé sur CRISPR Cas13, un système basé sur CRISPR Cas12, ou une combinaison de ceux-ci, éventuellement dans lequel l'acide nucléique cible amplifié est détecté par un système basé sur CRISPR Cas-13 comprenant l'enzyme LwaCas13.

9. Procédé selon l'une quelconque des revendications précédentes, dans lequel l'acide nucléique cible est choisi dans le groupe constitué par l'ADN génomique, l'ADN mitochondrial, l'ADN viral, l'ADN plasmidique, l'ADN libre de cellules circulantes, l'ADN environnemental, l'ADN double brin synthétique et l'ARN, éventuellement dans lequel l'acide nucléique cible est un ARN, et dans lequel l'ARN est transcrit de manière inverse en ADNc avant l'amplification, éventuellement dans lequel l'amplification est une amplification par recombinase polymérase (RPA).

10. Procédé selon l'une quelconque des revendications précédentes, dans lequel l'échantillon est un échantillon biologique ou un échantillon environnemental, éventuellement dans lequel l'échantillon biologique est un échantillon de sang, de plasma, de sérum, d'urine, de selles, de crachats, de mucus, de liquide lymphatique, de liquide synovial, de bile, d'ascite, d'épanchement pleural, de sérum, de salive, de liquide céphalorachidien, d'humeur aqueuse ou vitrée, ou toute sécrétion corporelle, un transsudat, un exsudat, ou un fluide obtenu à partir d'une articulation, ou un prélèvement de peau ou de surface de membrane muqueuse, de préférence dans lequel l'échantillon est du sang, du plasma ou du sérum obtenu à partir d'un patient humain, ou dans lequel l'échantillon est un échantillon de plante, éventuellement dans lequel l'échantillon est un échantillon brut, ou dans lequel l'échantillon est un échantillon purifié.

11. Méthode d'amplification et/ou de détection d'un acide nucléique simple brin cible, comprenant :
(a) la conversion de l'acide nucléique simple brin dans un échantillon en un acide nucléique double brin cible ; et
(b) la réalisation des étapes de la revendication 1, éventuellement dans laquelle l'acide nucléique simple brin cible est une molécule d'ARN, éventuellement dans laquelle la molécule d'ARN est convertie en acide nucléique double brin par une étape de transcription inverse et d'amplification, éventuellement dans laquelle l'acide nucléique simple brin cible est choisi dans le groupe constitué par l'ADN viral simple brin, l'ARN viral, l'ARN messager, l'ARN ribosomal, l'ARN de transfert, le microARN, l'ARN interférent court, le petit ARN nucléaire, l'ARN synthétique, l'ADN simple brin synthétique, l'ARN non codant long, le pré-microARN, l'ARNds viral et l'ARNds non viral.

12. Procédé de la revendication 1 comprenant en outre une amorce amplificatrice qui comprend une partie contenue dans la première amorce de la paire d'amorces ; et comprenant les étapes dans lesquelles la première amorce de la paire d'amorces sert de médiateur à une première étape d'amplification, et l'amorce amplificatrice conjointement avec la deuxième amorce de la paire d'amorces sert de médiateur aux étapes suivantes d'amplification, éventuellement dans lequel l'amorce amplificatrice et la première amorce de la paire d'amorces comprennent chacune une séquence de promoteur T7, éventuellement dans lequel l'amorce amplificatrice comprend une longueur d'environ 23 à environ 35 nucléotides.

13. Système pour l'amplification et/ou la détection d'un acide nucléique double brin cible dans un échantillon, le système comprenant :
a) un système CRISPR d'amplification, le système CRISPR d'amplification comprenant un premier et un deuxième complexe CRISPR/Cas, le premier complexe CRISPR/Cas comprenant une première enzyme CRISPR/Cas et une première molécule de guidage qui guide le premier complexe CRISPR/Cas vers un premier brin de l'acide nucléique cible, et le deuxième complexe CRISPR/Cas comprenant une deuxième enzyme CRISPR/Cas et une deuxième molécule de guidage qui guide le deuxième complexe CRISPR/Cas vers un deuxième brin de l'acide nucléique cible ;
b) une hélicase ;
c) une paire d'amorces comprenant une première et une deuxième amorce, dans laquelle la première amorce comprend une partie qui est complémentaire du premier brin de l'acide nucléique cible et la deuxième amorce comprend une partie qui est complémentaire du deuxième brin de l'acide nucléique cible ;
d) une polymérase ; et éventuellement
e) un système de détection pour détecter l'amplification de l'acide nucléique cible ;
éventuellement dans lequel l'enzyme CRISPR/Cas est une enzyme CRISPR/Cas morte choisie parmi une enzyme Cas9 morte ou une enzyme Cas12 morte, éventuellement dans laquelle l'enzyme Cas12 morte est une enzyme Cas12a, Cas12b ou Cas12c morte, ou dans laquelle l'enzyme CRISPR/Cas est une enzyme Cas9 ou Cas12, éventuellement dans laquelle l'enzyme Cas12 est une enzyme Cas12a, Cas12b ou Cas12c, éventuellement dans laquelle la polymérase est choisie dans le groupe constitué par l'ADN polymérase *Bst* 2.0, l'ADN polymérase *Bst* 2.0 WarmStart, l'ADN polymérase *Bst* 3.0, l'ADN polymérase *Bst* pleine longueur, l'ADN polymérase *Bst* grand fragment, l'ADN polymérase *Bsu* grand fragment, l'ADN polymérase phi29, l'ADN polymérase T7, la polymérase Gst, la polymérase Taq, le fragment Klenow de l'ADN polymérase I d'E. coli, l'ADN polymérase KlenTaq, l'ADN polymérase Pol III, l'ADN polymérase T5, et l'ADN polymérase Sequenase, et l'ADN polymérase Sau LF, éventuellement dans laquelle l'hélicase est choisie dans le groupe constitué par l'hélicase UvrD, l'hélicase CRISPR-Cas3, l'hélicase Rep, l'hélicase PcrA, l'hélicase E. coli I, l'hélicase E. coli II, l'hélicase E. coli III, l'hélicase IV d'E. coli, l'hélicase Rep, l'hélicase DnaB, l'hélicase PriA, l'hélicase PcrA, l'hélicase T4 Gp41, l'hélicase T4 Dda, le grand antigène T de SV40, l'hélicase de levure RAD, l'hélicase RecD, l'hélicase RecQ, l'hélicase thermostable T. tengcongensis UvrD, l'hélicase thermostable T. thermophilus UvrD, l'hélicase thermostable T. aquaticus DnaB, l'hélicase Dda, l'hélicase de papilloma virus E1, l'hélicase d'archée MCM, l'hélicase d'eucaryote MCM, et l'hélicase T7 Gp4, dans lequel l'hélicase est éventuellement Tte-UvrD D409A/D410A,
éventuellement comprenant en outre un ou plusieurs additifs choisis parmi un agent d'encombrement, un cofacteur, une protéine de liaison simple brin, et/ou des protéines d'assistance, éventuellement dans lequel l'agent d'encombrement est le polyéthylène glycol de poids moléculaire 20K, éventuellement fourni à environ 2,5 % à environ 6,5 %, éventuellement dans lequel le cofacteur est l'ATP, éventuellement fourni à 0,75 à environ 5 mM, éventuellement dans lequel la protéine de liaison simple brin est choisie parmi T4 gp32 ou SSB thermophile (ET-SSB), éventuellement dans lequel la protéine d'assistance est dCpf1 et/ou UvsX Recombinase, éventuellement dans lequel l'enzyme CRISPR/Cas, l'hélicase et la polymérase fonctionnent à la même température, dans certains cas, à environ 37° C,
éventuellement dans lequel le système de détection comprend un système CRISPR comprenant une protéine effectrice Cas 12 ou Cas 13 et un ARN guide conçu pour se lier à une molécule cible correspondante ; et une construction de masquage, éventuellement dans lequel le système de détection comprend l'enzyme Cas 13 LwaCas13,
comprenant éventuellement en outre une amorce amplificatrice supplémentaire comprenant une identité de séquence promotrice avec la première amorce de la paire d'amorces.

14. Système pour l'amplification et/ou la détection d'un acide nucléique simple brin cible dans un échantillon, le système comprenant :
a) des réactifs pour convertir l'acide nucléique simple brin cible en un acide nucléique double brin ;
b) des composants de la revendication 13 ;
éventuellement dans lequel le système de détection comprend un système CRISPR comprenant une protéine effectrice Cas 12 ou Cas 13 et un ARN guide conçu pour se lier à une molécule cible correspondante ; et une construction de masquage, éventuellement dans lequel le système de détection comprend l'enzyme Cas 13 LwaCas13, éventuellement comprenant en outre des réactifs pour purifier l'acide nucléique simple brin dans l'échantillon.

15. Procédé de la revendication 1 ou système de la revendication 13, dans lequel l'hélicase est l'hélicase PcrA de Thermoanaerobacter ethanolicus avec les mutations D403A/D404A, l'hélicase PcrA de Bacillus sp. FJAT-27231 avec les mutations D407A/D408A, l'hélicase PcrA de Bacillus megaterium avec les mutations D415A/D416A, l'hélicase PcrA de Bacillus simplex avec les mutations D407A/D408A, ou l'hélicase PcrA de Paeniclostridium sordellii avec les mutations D402A/D403A.
